# EUROPEAN PATENT SPECIFICATION

(11) **EP 1 203 007 B1**
(45) Date of publication and mention of the grant of the patent: **15.04.2009**
(21) Application number: 00948923.8
(22) Date of filing: 24.07.2000
(51) Int. Cl.: C07H 21/04, C07H 21/02, A61K 48/00, C12Q 1/68, C12N 15/85, C12N 15/86

(54) **NUCLEIC ACID LIGANDS TO HEPATOCYTE GROWTH FACTOR/SCATTER FACTOR (HGF/SF) AND ITS RECEPTOR C-MET**
NUKLEINSÄURELIGANDEN FÜR DEN HEPATOZYTISCHEN WACHSTUMSFAKTOR/DISPERSIONSFAKTOR (HGF/SF) UND SEINES C-MET REZEPTORS
LIGANDS D'ACIDE NUCLEIQUE AU FACTEUR DE CROISSANCE/FACTEUR DE DISPERSION DES HEPATOCYTES (HGF/SF) ET SON RECEPTEUR C-MET

(30) Priority: 29.07.1999 US 364543; 29.07.1999 US 364539
(43) Date of publication of application: 08.05.2002
(73) Proprietor: GILEAD SCIENCES, INC., Foster City CA 94404 (US)
(72) Inventor: RUCKMAN, Judy, Boulder, CO 80303 (US); GOLD, Larry, Boulder, CO 80302 (US); STEPHENS, Andrew, Boulder, CO 80302 (US); JANJIC, Nebojsa, Boulder, CO 80301 (US); RABIN, Ross, Lafayette, CO 80026 (US); LOCHRIE, Michael, Hayward, CA 94541-3581 (US)
(74) Representative: Clegg, Richard Ian
(86) International application number: PCT/US2000/020139
(87) International publication number: WO 2001/009159

(56) References cited:
- WO-A1-91/19813
- LOKKER N A ET AL: "GENERATION AND CHARACTERIZATION OF A COMPETITIVE ANTAGONIST OF HUMAN HEPATOCYTE GROWTH FACTOR, HGF/NK1" JOURNAL OF BIOLOGICAL CHEMISTRY, AMERICAN SOCIETY OF BIOLOGICAL CHEMISTS, BALTIMORE, MD, US, vol. 268, no. 23, 15 August 1993 (1993-08-15), pages 17145-17150, XP000942254 ISSN: 0021-9258
- BOTTARO D P ET AL: "IDENTIFICATION OF THE HEPATOCYTE GROWTH FACTOR RECEPTOR AS THE C-MET PROTO-OCOGENE PRODUCT" SCIENCE, AMERICAN ASSOCIATION FOR THE ADVANCEMENT OF SCIENCE,, US, vol. 251, 15 February 1991 (1991-02-15), pages 802-804, XP000941505 ISSN: 0036-8075
- JHAVERI S ET AL: "In vitro selection of phosphorothiolated aptamers" BIOORGANIC & MEDICINAL CHEMISTRY LETTERS, OXFORD, GB, vol. 8, no. 17, 8 September 1998 (1998-09-08), pages 2285-2290, XP004138219 ISSN: 0960-894X
- JELLINEK DEREK ET AL: "Potent 2'-amino-2'deoxypyrimidine RNA inhibitors of basic fibroblast growth factor" BIOCHEMISTRY, AMERICAN CHEMICAL SOCIETY. EASTON, PA, US, vol. 34, no. 36, 1995, pages 11363-11372, XP002205031 ISSN: 0006-2960
- RUCKMAN JUDY ET AL: "2'-Fluoropyrimidine RNA-based aptamers to the 165-amino acid form of vascular endothelial growth factor (VEGF165): Inhibition of receptor binding and VEGF-induced vascular permeability through interactions requiring the exon 7-encoded domain." JOURNAL OF BIOLOGICAL CHEMISTRY, vol. 273, no. 32, 7 August 1998 (1998-08-07), pages 20556-20567, XP002226849 ISSN: 0021-9258
- GOLD L ET AL: "DIVERSITY OF OLIGONUCLEOTIDE FUNCTIONS" ANNUAL REVIEW OF BIOCHEMISTRY, PALTO ALTO, CA, US, vol. 64, 1995, pages 763-797, XP000618016 ISSN: 0066-4154
- TUERK C ET AL: "SYSTEMATIC EVOLUTION OF LIGANDS BY EXPONENTIAL ENRICHMENT: RNA LIGANDS TO BACTERIOPHAGE T4 DNA POLYMERASE" SCIENCE, AMERICAN ASSOCIATION FOR THE ADVANCEMENT OF SCIENCE,, US, vol. 249, 3 August 1990 (1990-08-03), pages 505-510, XP000647748 ISSN: 0036-8075
- BLIND ET AL: "CYTOPLASMIC RNA MODULATORS OF AN INSIDE-OUT SIGNAL-TRANSDUCTION CASCADE" PROCEEDINGS OF THE NATIONAL ACADEMY OF SCIENCES OF USA, NATIONAL ACADEMY OF SCIENCE. WASHINGTON, US, vol. 96, March 1999 (1999-03), pages 3606-3610, XP002113353 ISSN: 0027-8424
- DAVIS ET AL.: 'Identifying concensus patterns and secondary structure in SELEX sequence sets' METHODS IN ENZYMOLOGY vol. 267, 1996, pages 302 - 314, XP002932130
- TUCKER ET AL.: 'Detection and plasma pharmacokinetics of an anti-vascular endothelial growth factor oligonucleotide-aptamer (NX1838) in rhesus monkeys' JOURNAL OF CHROMATOGRAPHY vol. 732, no. 1, 10 September 1999, pages 203 - 212, XP002932129
- GREEN ET AL.: 'Nuclease-resistant nucleic acid ligands to vascular permeability factor/vascular endothelial growth factor' CHEMISTRY AND BIOLOGY vol. 2, no. 10, October 1995, pages 683 - 695, XP002932128
- FLOEGE ET AL.: 'Novel approach to specific growth factor inhibition in vivo' AMERICAN JOURNAL OF PATHOLOGY vol. 154, no. 1, 01 January 1999, pages 169 - 179, XP002932200

## Description

### Field of the Invention

Described herein are methods for identifying and preparing high affinity nucleic acid ligands of hepatocyte growth factor/scatter factor (HGF) and its receptor c-met. The method utilized herein for identifying such nucleic acid ligands is called SELEX, an acronym for Systematic Evolution of Ligands by EXponential enrichment. The invention is also directed towards therapeutic and diagnostic reagents for diseases in which elevated HGF and c-met activity are causative factors.

Also described herein are methods for identifying nucleic acid ligands of integrins using the SELEX method. This relates to integrin proteins, and methods and compositions for treating and diagnosing diseases involving integrins.

### Background of the Invention

Hepatocyte growth factor/scatter factor (abbreviated herein as HGF) is a potent cytokine which, through interaction with its receptor c-met, stimulates proliferation, morphogenesis, and migration of a wide variety of cell types, predominantly epithelial. HGF and c-met are involved in several cellular processes involved in tumorigenesis, notably angiogenesis and motogenesis, the latter having been implicated in the migration of cells required for metastasis (reviewed in Jiang and Hiscox (1997) Histol Histopathol. 12:537-55; Tamagnone and Comoglio (1997) Cytokine Growth Factor Rev. 8:129-42; Jiang, et al. (1999) Crit Rev Oncol Hematol. 29:209-48). Interestingly, proteases that degrade the extracellular matrix also activate HGF, which in turn up-regulates urokinase type plasminogen activator (uPA) and its receptor, resulting in an activating loop feeding the invasive and migratory processes required for metastatic cancer.

HGF and the c-met receptor are expressed at abnormally high levels in a large variety of solid tumors. In addition to numerous demonstrations *in vitro* of the effects of HGF/c-met on the behavior of tumor cell lines, the levels of HGF and/or c-met have been measured in human tumor tissues (reviewed in Jiang (1999) Crit Rev Oncol Hematol. 29:209-48). High levels of HGF and/or c-met have been observed in liver, breast, pancreas, lung, kidney, bladder, ovary, brain, prostate, gallbladder and myeloma tumors in addition to many others.

For several of the cancer types listed above, the prognostic value of measuring HGF/c-met levels has been evaluated and found to be potentially useful for determining the progression and severity of disease. The correlative data are strongest in the case of breast cancer (Ghoussoub, Dillon et al. (1998) Cancer. 82:1513-20; Toi, Taniguchi et al. (1998) Clin Cancer Res. 4:659-64), and non-small cell lung cancer (Siegfried, Weissfeld et al. (1997) Cancer Res. 57:433-9; Siegfried, Weissfeld et al. (1998) Ann Thorac Surg. 66:1915-8).

Elevated levels of HGF and c-met have also been observed in non-oncological settings, such as hypertension (Morishita, Aoki et al. (1997) J Atheroscler Thromb. 4:12-9; Nakamura, Moriguchi et al. (1998), Biochem Biophys Res Commun. 242:238-43), arteriosclerosis (Nishimura, Ushiyama et al. (1997) J Hypertens. 15:1137-42; Morishita, Nakamura et al. (1998) J Atheroscler Thromb. 4:128-34), myocardial infarction (Sato, Yoshinouchi et al. (1998) J Cardiol. 32:77-82), and rheumatoid arthritis (Koch, Halloran et al. (1996) Arthritis Rheum. 39:1566-75), raising the possibility of additional therapeutic and diagnostic applications.

The role of HGF/c-met in metastasis has been elucidated in mice using cell lines transformed with HGF/c-met (reviewed in Jeffers, Rong et al. (1996) J Mol Med. 74:505-13). In another metastasis model, human breast carcinoma cells expressing HGF/c-met were injected in the mouse mammary fat pad, resulting in eventual lung metastases in addition to the primary tumor (Meiners, Brinkmann et al. (1998) Oncogene. 16:9-20). Also, transgenic mice which overexpress HGF become tumor-laden at many loci (Takayama, LaRochelle et al. (1997) Proc. Natl. Acad. Sci. U.S.A. 94:701-6).

None of the data mentioned above provide proof of a direct causative role of HGF/c-met in human cancer, although the accumulated weight of the correlative data are convincing. However, a causal connection was established between germ-line c-met mutations, which constitutively activate its tyrosine kinase domain, and the occurrence of human papillary renal carcinoma (Schmidt, Duh et al. (1997) Nat Genet. 16:68-73).

Recent work on the relationship between inhibition of angiogenesis and the suppression or reversion of tumor progression shows great promise in the treatment of cancer (Boehm, Folkman et al. (1997) Nature. 390:404-7). In this report, it was shown that the use of multiple angiogenesis inhibitors confers superior tumor suppression/regression compared to the effect of a single inhibitor. Angiogenesis is markedly stimulated by HGF, as well as vascular endothelial growth factor (VEGF) and basic fibroblast growth factor (bFGF) (Rosen, Lamszus et al. (1997) Ciba Found Symp. 212:215-26). HGF and VEGF were recently reported to have an additive or synergistic effect on mitogenesis of human umbilical vein endothelial cells (HUVECs) (Van Belle, Witzenbichler et al. (1998) Circulation. 97:381-90). Similar combined effects are likely to contribute to angiogenesis and metastasis.

Human HGF protein is expressed as a single peptide chain of 728 amino acids (reviewed in Mizuno and Nakamura (1993) Exs. 65:1-29; Rubin, Bottaro et al. (1993) Biochim Biophys Acta. 1155:357-71; Jiang (1999) Crit Rev Oncol Hematol. 29:209-48). The amino-terminal 31 residue signal sequence of HGF is cleaved upon export; followed by proteolytic cleavage by uPA and/or other proteases. The mature protein is a heterodimer consisting of a 463 residue α-subunit and a 234 residue β-subunit, linked via a single disulfide bond. HGF is homologous to plasminogen: its α-subunit contains an N-terminal plasminogen-activator-peptide (PAP) followed by four kringle domains, and the β-subunit is a serine protease-like domain, inactive because it lacks critical catalytic amino acids. The recently solved crystal structure of an HGF fragment containing PAP and the first kringle domain indicate that this region is responsible for heparin binding and dimerization (Chirgadze, Hepple et al. (1999) Nat Struct Biol. 6:72-9), in addition to receptor interaction.

Human c-met protein is exported to the cell surface via a 23 amino acid signal sequence (reviewed in Comoglio (1993) Exs. 65:131-65; Rubin (1993) Biochim Biophys Acta. 1155:357-71; Jiang (1999) Crit Rev Oncol Hematol. 29:209-48). The exported form of c-met is initially a pro-peptide which is proteolytically cleaved. The mature protein is a heterodimer consisting of an extracellular 50 kDa α-subunit bound by disulfide bonds to a 140 kDa β-subunits. In addition to its extracellular domain, the β-subunit has a presumed membrane-spanning sequence and a 435 amino acid intracellular domain containing a typical tyrosine kinase.

HGF is produced primarily by mesenchymal cells, while c-met is mainly expressed on cells of epithelial origin. HGF is very highly conserved at the amino acid level between species. This homology extends into the functional realm as observed in mitogenic stimulation of hepatocytes in culture by HGF across species, including human, rat, mouse, pig and dog. This indicates that human HGF can be used cross-specifically in a variety of assays.

Given the roles of HGF and c-met in disease, it would be desirable to have agents that bind to and inhibit the activity of these proteins. It would also be desirable to have agents that can quantitate the levels of HGF and c-met in individual in order to gather diagnostic and prognostic information.

The integrins are a class of heterodimeric integral membrane proteins, one or more of which are expressed by most cell types (Hynes (1992) Cell 69:11-25). Some 16 homologous α subunits and 8 homologous β subunits associate in various combinations to yield an extensive family of receptors. Each integrin heterodimer has a large extracellular domain that mediates binding to specific ligands. These ligands may include plasma proteins, proteins expressed on the surface of adjacent cells, or components of the extracellular matrix. Several of the integrins show affinity for more than one ligand and many have overlapping specificities (Hynes (1992) Cell 69:11-25). Both the α and β subunits contribute to a small intracellular domain that contacts components of the actin cytoskeleton, thus forming a physical link between proteins outside and inside the cell.

Integrins play an important role in cellular adhesion and migration, and these properties are controlled by the cell, partly by modulation of integrin affinity for its ligands (so-called "inside-out" signaling). Conversely, the presence or absence of integrin ligation provides specific information about the cellular microenvironment, and in many instances integrins serve as a conduit for signal transduction. Ligand binding by an integrin may promote its incorporation into focal adhesions, the assembly of cytoskeletal and intracellular signaling molecules into supra-molecular complexes, and the initiation of a cascade of downstream signaling events including protein phosphorylation, calcium release, and an increase in intracellular pH (reviewed by Schwartz et al. (1995) Ann. Rev. Cell Dev. Biol. 11:549-99). Such "outside-in" signaling ties into pathways controlling cell proliferation, migration and apoptosis (Stromblad et al. (1996) J. Clin. Invest. 98:426-33; Eliceiri et al. (1998) J. Cell. Biol. 140:1255-63). Integrins have been shown to play a role in such diverse physiological settings as embryonic development, wound healing, angiogenesis, clot formation, leukocyte extravasation, bone resorption and tumor metastasis.

The β₃-containing integrins are among the best studied of the receptor superfamily. The β₃ subunit forms heterodimers with either αᵥ, (αᵥβ₃) or α_{IIb} (α_{IIb}β₃). While these integrins show substantial overlap in ligand specificity, they play very different roles in normal physiology and in disease.

αᵥβ₃ is expressed by activated endothelial cells, smooth muscle cells, osteoclasts, and, at a very low level, by platelets. It is also expressed by a variety of tumor cell types. The integrin binds to a number of plasma proteins or proteins of the extracellular matrix, many of which are associated with sites of inflammation or wound healing (Albelda (1991) Am. J. Resp. Cell Mol. Biol. 4:195-203). These include vitronectin, fibronectin, osteopontin, von Willebrand factor, thrombospondin, fibrinogen, and denatured collagen Type I (Hynes (1992) Cell 69:11-25). Each of these proteins share a common sequence motif, arginine-glycine-aspartic acid (RGD), that forms the core of the integrin binding site.

αᵥβ₃ has been most intensely studied in the context of new blood vessel formation (angiogenesis) where it mediates the adhesion and migration of endothelial cells through the extracellular matrix. Angiogenesis in adults is normally associated with the cyclical development of the corpus luteum and endometrium and with the formation of granulation tissue during wound repair. In the latter case, microvascular endothelial cells form vascular sprouts that penetrate into the temporary matrix within a wound. These cells transiently express αᵥβ₃ and inhibition of the ligand binding function of the integrin temporarily inhibits the formation of granulation tissue (Clark et al. (1996) Am. J. Pathol. 148:1407-21). In cytokine-stimulated or unstimulated angiogenesis on the chick chorioallantoic membrane, blockade of αᵥβ₃ with a heterodimer-specific antibody prevents new vessel formation without affecting the pre-existing vasculature (Brooks et al. (1994) Science 264:569-71). Furthermore, the loss of adhesive contacts by endothelial cells activated for angiogenesis induces a phenotype characteristic of apoptotic cells (Brooks et al. (1994) Cell 79:1157-64); that is, ligand binding by αᵥβ₃ appears to transmit a survival signal to the cell. Thus, adhesion and/or signaling mediated by αᵥβ₃ is essential for the formation of new blood vessels.

Solid tumors are unable to grow to significant size without an independent blood supply. It is currently hypothesized that the acquisition of an angiogenic phenotype is one of the limiting steps in the growth of primary tumors and of tumors at secondary sites (Folkman (1995) Nat. Med. 1:27-31). In addition, while the vasculature that penetrates a tumor mass provides a source of oxygen and nutrients, it also serves as a conduit for metastatic cells to leave the primary tumor and migrate throughout the body. Thus, inhibition of angiogenesis may limit both the growth and metastasis of cancerous lesions. In experimental settings of tumor-induced angiogenesis, inhibition of ligand-binding by endothelial αᵥβ₃ prevented the formation of new blood vessels (Brooks et al. (1994) Cell 79:1157-64; Brooks et al. (1995) J. Clin. Invest. 96:1815-22), and inhibitors of αᵥβ₃ were shown to reduce the growth of experimental tumors *in vivo* (Brooks et al. (1995) J. Clin. Invest. 96:1815-22; Carron et al. (1998) Canc. Res. 58:1930-5).

αᵥβ₃ is not only expressed by the microvasculature within tumors, but in some cases, is also found on the surface of tumor cells themselves. In particular, expression of αᵥβ₃ integrin has been detected in tissue sections from tumors of melanocytic and astroglial origin (Albelda et al. (1990) Canc. Res. 50:6757-64; Gladson and Cheresh (1991) J. Clin. Invest. 88:1924-32), and the level of integrin expression has been correlated with the stage or metastatic potential of the tumor (Albelda et al. (1990) Canc. Res. 50:6757-64; Gladson (1996) Am. J. Pathol. 148:1423-34; Hieken et al. (1996) J. Surg. Res. 63:169-73). Furthermore, melanoma cells grown *in vitro* in a three-dimensional matrix of denatured collagen undergo apoptosis upon αᵥβ₃ blockade.

Data such as these have driven an interest in inhibitors of αᵥβ₃ for the treatment of cancer. At present, two such inhibitors are in or near clinical trial: Vitaxin is a chimeric Fab fragment derived from the αᵥβ₃-specific monoclonal antibody, LM609 (Wu et al. (1998) Proc. Nat. Acad. Sci. 95:6037-42). A phase I trial in late-stage cancer patients has been completed and no significant treatment-associated toxicities were observed (Gutheil et al. (1998) Am. Soc. Clin. Onc.). EMD121974 is a cyclic pentapeptide inhibitor of αᵥβ₃. A Phase I study of this compound in Kaposi's sarcoma, brain tumors and solid tumors is scheduled to begin in 1999.

Angiogenesis (and αᵥβ₃) are implicated in the pathology of several other diseases, including psoriasis (Creamer et al. (1995) Am. J. Pathol. 147:1661-7), rheumatoid arthritis (Walsh et al. (1998) Am. J. Pathol. 152:691-702; Storgard et al. (1999) J. Clin. Invest. 103:47-54), endometriosis (Healy et al. (1998) Hum. Reprod. Update 4:736-40), and several proliferative diseases of the eye (Casaroli Marano et al. (1995) Exp. Eye Res. 60:5-17; Friedlander et al. (1996) Proc. Nat. Acad. Sci. 93:9764-9; Hammes et al. (1996) Nat. Med. 2:529-33). Inhibition of integrin ligand binding in each of these contexts may provide significant therapeutic benefit.

Atheromatous plaque and restenosis following angioplasty are pathologies characterized by thickening of the intima, the innermost layer of the arterial wall. The proliferation and/or migration of smooth muscle cells into the neointima with concomitant deposition of fibrous extracellular proteins contributes to vessel wall thickening and subsequent vessel occlusion. Platelets may also contribute to the development of restenotic lesions through adhesion to endothelial cells and the release of growth factors and cytokines that stimulate the underlying smooth muscle cell layer (Le Breton et al. (1996) J. Am. Coll. Cardiol. 28:1643-51). αᵥβ₃ integrin is expressed on arterial smooth muscle cells (Hoshiga et al. (1995) Circ. Res. 77:1129-35) and mediates their migration on vitronectin and osteopontin (Brown et al. (1994) Cardiovasc. Res. 28:1815-20; Jones et al. (1996) Proc. Nat. Acad. Sci. 93:2482-7; Liaw et al. (1995) J. Clin. Invest. 95:713-24; Panda et al. (1997) Proc. Nat. Acad. Sci. 94:9308-13), two matrix proteins associated with atheroschlerotic tissues *in vivo* (Brown et al. (1994) Cardiovasc. Res. 28:1815-20; Giachelli et al. (1995) Ann. N. Y. Acad. Sci. 760:109-26; Panda et al. (1997) Proc. Nat. Acad. Sci. 94:9308-13). In addition, αᵥβ₃ expression on endothelial cells, and to a much lesser extent on platelets, is responsible for at least part of the adhesive interaction between these cell types (Le Breton et al. (1996) J. Am. Coll. Cardiol. 28:1643-51; Gawaz et al. (1997) Circulation 96:1809-18). αᵥβ3 blockade with RGD-containing peptides or a monoclonal antibody was found to limit neointimal hyperplasia in several animal models of restenosis following arterial injury (Choi et al. (1994) J. Vasc. Surg. 19:125-34; Srivatsa et al. (1997) Cardiovasc. Res. 36:408-28; Slepian et al. (1998) Circulation 97:1818-27; Coleman et al. (1999) Circ. Res. 84:1268-76). Furthermore, treatment of patients undergoing percutaneous coronary intervention with an anti-β3 antibody (Reopro/abciximab/c7E3), which blocks both the platelet fibrinogen receptor, α_{IIb}β₃, and αᵥβ₃, provided long term reduction in the rates of death or myocardial infarction and in the rate of reocclusion of the artery (Lefkovits et al. (1996) Am. J. Cardiol. 77:1045-51), an effect that may be mediated through inhibition of αᵥβ₃ ligation. The observation that αᵥβ₃ is expressed by microvascular smooth muscle cells after experimentally-induced focal cerebral ischemia (Okada et al. (1996) Am. J. Pathol. 149:37-44) suggests that this integrin may also play some role in the development of ischemia/reperfusion injury in stroke.

Finally, αᵥβ₃ mediates the attachment of osteoclasts to matrix proteins, particularly osteopontin, on the surface of bone. Osteoclasts are responsible for the resorption of bone in normal physiology as well as in pathological conditions such as osteoporosis. A monoclonal antibody specific for αᵥβ₃ inhibited the binding and resorption of bone particles by osteoclasts *in vitro* (Ross et al. (1993) J. Biol. Chem. 268:9901-7). Furthermore, an RGD-containing protein, echistatin, was shown to block parathyroid-stimulated bone resorption in an animal model, as monitored by serum calcium levels (Fisher et al. (1993) Endocrin. 132:1411-3). Inhibitors of αᵥβ₃ integrin are thus considered of potential utility in treating debilitating bone loss such as occurs in osteoporosis.

α_{IIb}β₃ (also referred to as GPIIbIIIa) is the major integrin on the surface of platelets where it mediates the adhesion of activated platelets to the plasma protein fibrinogen (Nachman and Leung (1982) J. Clin. Invest. 69:263-9; Shattil et al. (1985) J. Biol. Chem. 260:11107-14). During clot formation, fibrinogen dimers cross-link platelets to one another through the integrin receptor. α_{IIb}β₃ also binds to several other plasma and cell matrix proteins, including von Willebrand factor, vitronectin, and fibronectin (Faull and Ginsberg (1996) J. Am. Soc. Nephrol. 7:1091-7).

Clot formation is a tightly regulated process that balances the need for rapid response to vascular injury, with the risk of aberrant occlusion of critical vessels. The α_{IIb}β₃ heterodimer is constitutively expressed on the surface of resting platelets at approximately 80,000 copies per cell (Wagner et al. (1996) Blood 88:907-14); however, the affinity of the integrin for fibrinogen is very low on these cells. Activation of platelets by ADP, epinephrine, collagen or thrombin leads to a dramatic enhancement in integrin ligand binding activity (Bennett and Vilaire (1979) J. Clin. Invest. 64:1393-401; Marguerie et al. (1979) J. Biol. Chem. 254:5357-63), probably accomplished through a conformational change in the receptor (Shattil et al. (1985) J. Biol. Chem. 260:11107-14; O'Toole et al. (1990) Cell Reg. 1:883-93; Du et al. (1993) J. Biol. Chem. 268:23087-92). In this prototypic example of "inside-out" control of integrin function, cross-linking of platelets through the α_{IIb}β₃-fibrinogen interaction is confined to local sites of platelet activation.

Inhibitors of α_{IIb}β₃ ligand binding have been primarily explored in the context of cardiovascular disease (Chong (1998) Am. J. Health Syst. Pharm. 55:2363-86; Topol et al. (1999) Lancet 353:227-31), but may have application in any of a number of indications where thrombus formation is suspected or is likely. Three α_{IIb}β₃ inhibitors, Reopro, Integrilin and Aggrastat, have been approved for use in patients experiencing acute coronary syndrome and/or in patients who are undergoing percutaneous coronary intervention. Reopro (Centocor/Eli Lilly) is a humanized murine monoclonal antibody Fab fragment with specificity for the β₃ chain of α_{IIb}β₃. Integrilin (COR Therapeutics) is a cyclic heptapeptide based on the integrin binding site of barbourin, an α_{IIb}β₃ inhibitory protein derived from snake venom. Aggrastat (Merck & Co.) is a non-peptide small molecule antagonist of the integrin. Unlike the small molecule inhibitors, Reopro cross-reacts with αᵥβ₃, a fact that may account for the greater reduction in long-term rates of death and non-fatal myocardial infarction associated with its use (see above). A significant effort is underway to identify new inhibitors of the platelet integrin with characteristics not found in the cohort of approved drugs. Specifically, compounds with specificity for the active, ligand-binding conformation of α_{IIb}β₃ may reduce the risk of bleeding complications associated with the existing anti-clotting therapies. Orally available compounds would be particularly useful for longer term therapy of patients at risk for recurrent myocardial infarction or unstable angina.

Given the role of integrins in the various disease states described above, it would be desirable to have high specificity inhibitors of particular integrins. The present invention provides such agents.

The dogma for many years was that nucleic acids had primarily an informational role. Through a method known as Systematic Evolution of Ligands by EXponential enrichment, termed the SELEX process, it has become clear that nucleic acids have three dimensional structural diversity not unlike proteins. The SELEX process is a method for the *in vitro* evolution of nucleic acid molecules with highly specific binding to target molecules and is described in U.S. Patent Application Serial No. 07/536,428, filed June 11, 1990, entitled "Systematic Evolution of Ligands by Exponential Enrichment," now abandoned, U.S. Patent No. 5,475,096, entitled "Nucleic Acid Ligands," and U.S. Patent No. 5,270,163 (see also WO 91/19813), entitled "Methods for Identifying Nucleic Acid Ligands," each of which is specifically incorporated herein by reference in its entirety. Each of these applications, collectively referred to herein as the SELEX Patent Applications, describes a fundamentally novel method for making a nucleic acid ligand to any desired target molecule.

The SELEX process provides a class of products which are referred to as nucleic acid ligands or aptamers, each having a unique sequence, and which has the property of binding specifically to a desired target compound or molecule. Each SELEX-identified nucleic acid ligand is a specific ligand of a given target compound or molecule. The SELEX process is based on the unique insight that nucleic acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size or composition can serve as targets. The SELEX method applied to the application of high affinity binding involves selection from a mixture of candidate oligonucleotides and step-wise iterations of binding, partitioning and amplification, using the same general selection scheme, to achieve virtually any desired criterion of binding affinity and selectivity. Starting from a mixture of nucleic acids, preferably comprising a segment of randomized sequence, the SELEX method includes steps of contacting the mixture with the target under conditions favorable for binding, partitioning unbound nucleic acids from those nucleic acids which have bound specifically to target molecules, dissociating the nucleic acid-target complexes, amplifying the nucleic acids dissociated from the nucleic acid-target complexes to yield a ligand-enriched mixture of nucleic acids, then reiterating the steps of binding, partitioning, dissociating and amplifying through as many cycles as desired to yield highly specific high affinity nucleic acid ligands to the target molecule.

It has been recognized by the present inventors that the SELEX method demonstrates that nucleic acids as chemical compounds can form a wide array of shapes, sizes and configurations, and are capable of a far broader repertoire of binding and other functions than those displayed by nucleic acids in biological systems.

The basic SELEX method has been modified to achieve a number of specific objectives. For example, U.S. Patent Application Serial No. 07/960,093, filed October 14, 1992, now abandoned, and U.S. Patent No. 5,707,796, both entitled "Method for Selecting Nucleic Acids on the Basis of Structure," describe the use of the SELEX process in conjunction with gel electrophoresis to select nucleic acid molecules with specific structural characteristics, such as bent DNA. U.S. Patent Application Serial No. 08/123,935, filed September 17, 1993, entitled "Photoselection of Nucleic Acid Ligands," now abandoned, U.S. Patent No. 5,763,177 and U.S. Patent No. 6,001,577, both entitled "Systematic Evolution of Ligands by Exponential Enrichment: Photoselection of Nucleic Acid Ligands and Solution SELEX," describe a SELEX based method for selecting nucleic acid ligands containing photoreactive groups capable of binding and/or photocrosslinking to and/or photoinactivating a target molecule. U.S. Patent No. 5,580,737, entitled "High-Affinity Nucleic Acid Ligands That Discriminate Between Theophylline and Caffeine," describes a method for identifying highly specific nucleic acid ligands able to discriminate between closely related molecules, which can be non-peptidic, termed Counter-SELEX. U.S. Patent No. 5,567,588, entitled "Systematic Evolution of Ligands by Exponential Enrichment: Solution SELEX," describes a SELEX-based method which achieves highly efficient partitioning between oligonucleotides having high and low affinity for a target molecule.

The SELEX method encompasses the identification of high-affinity nucleic acid ligands containing modified nucleotides conferring improved characteristics on the ligand, such as improved *in vivo* stability or improved delivery characteristics. Examples of such modifications include chemical substitutions at the ribose and/or phosphate and/or base positions. SELEX process-identified nucleic acid ligands containing modified nucleotides are described in U.S. Patent No. 5,660,985, entitled "High Affinity Nucleic Acid Ligands Containing Modified Nucleotides," that describes oligonucleotides containing nucleotide derivatives chemically modified at the 5- and 2'-positions of pyrimidines. U.S. Patent No. 5,580,737, *supra,* describes highly specific nucleic acid ligands containing one or more nucleotides modified with 2'-amino (2'-NH₂), 2'-fluoro (2'-F), and/or 2'-methoxy (2'-OMe). U.S. Patent Application Serial No. 08/264,029, filed June 22, 1994, entitled "Novel Method of Preparation of Known and Novel 2' Modified Nucleosides by Intramolecular Nucleophilic Displacement," describes oligonucleotides containing various 2'-modified pyrimidines.

The SELEX method encompasses combining selected oligonucleotides with other selected oligonucleotides and non-oligonucleotide functional units as described in U.S. Patent No. 5,637,459, entitled "Systematic Evolution of Ligands by Exponential Enrichment: Chimeric SELEX," and U.S. Patent No. 5,683,867, entitled "Systematic Evolution of Ligands by Exponential Enrichment: Blended SELEX," respectively. These applications allow the combination of the broad array of shapes and other properties, and the efficient amplification and replication properties of oligonucleotides with the desirable properties of other molecules.

The SELEX method further encompasses combining selected nucleic acid ligands with lipophilic compounds or non-immunogenic, high molecular weight compounds in a diagnostic or therapeutic complex as described in U.S. Patent No. 6,011,020, entitled "Nucleic Acid Ligand Complexes." Each of the above described patent applications which describe modifications of the basic SELEX procedure are specifically incorporated herein by reference in their entirety.

It is an object of the present invention to obtain nucleic acid ligands to HGF using the SELEX process.

It is a further object of the invention to obtain nucleic acid ligands that act as inhibitors of HGF.

It is a further object of the invention to provide therapeutic and diagnostic agents for tumorigenic conditions in which HGF and c-met are implicated.

It is yet a further object of the invention to use nucleic acid ligands to HGF and c-met to diagnose and treat hypertension, arteriosclerosis, myocardial infarction, and rheumatoid arthritis.

It is an even further object of the invention to use nucleic acid ligands to HGF singly or in combination with other nucleic acid ligands that inhibit VEGF and/or bFGF, and/or possibly other angiogenesis factors.

### Summary of the Invention

Methods are provided for generating nucleic acid ligands to HGF. The methods use the SELEX process for ligand generation. The nucleic acid ligands to HGF provided by the invention are useful as therapeutic and diagnostic agents for a number of diseases.

### Brief Description of the Drawings

Figure 1 illustrates the template and primer oligonucleotides used in the 2'-F-pyrimidine RNA SELEX experiments. The 5' fixed region of the template and primers contains a T7 promoter to facilitate transcription of RNA by T7 RNA polymerase.
Figure 2 illustrates RNaseH cleavage primers used in hybridization truncate SELEX. Bases depicted in bold-type are 2'-Ome modified and bases underlined are deoxyribonucleosides. The random region is designated as "N". Upon treatment with RNaseH, the fixed regions are removed at the positions indicated by the carets. Note that the there are two possible cleavage sites at the 5'-end of the fixed region, resulting in RNA which has one or two fixed G residues.
Figure 3 illustrates binding of SELEX pools to HGF. Figure 3A shows HGF SELEX 1 30N7 pools and Figure 3B shows HGF SELEX 2 30N8 pools.
Figure 4 illustrates two methods of evaluating HGF SELEX 3 30N7 pool binding to HGF. In Figure 4A, heparin competes with RNA pools for binding to 2.7 nM HGF and Figure 4B illustrates conventional pool binding.
Figure 5 illustrates two methods of evaluating HGF SELEX 3 30N7 pool binding to HGF. Figure 5A shows that tRNA competes with RNA pools for binding to 2.7 nM HGF and Figure 5B shows conventional pool binding.
Figure 6 illustrates inhibition of 10 ng/mL HGF stimulation of starved HUVECs by aptamers. Figure 6A shows a 1^{st} set of aptamers and Figure 6B illustrates a 2^{nd} set of aptamers.
Figure 7 illustrates truncates of aptamer 8-102 (SEQ ID NO: 12). Figure 7A shows predicted two-dimensional structures of full-length and truncated sequences and Figure 7B shows binding of full-length and truncated aptamers to HGF.
Figure 8 illustrates truncates of aptamer 8-17 (SEQ ID NO:68). Figure 8A shows a predicted two-dimensional structures of full-length and truncated sequences and Figure 8B shows binding of full-length and truncated aptamers to HGF.
Figure 9 illustrates binding of HGF truncate SELEX pools. Figure 9A shows the HGF SELEX 4 30N7 series and Figure 9B shows the HGF SELEX 5 30N7 series.
Figure 10 shows aptamer inhibition of 100 ng/mL HGF stimulation of 4MBr-5 cells.
Figure 11 illustrates aptamer inhibition of 50 ng/mL HGF stimulation of 4MBr5 cells. Figure 11A shows the effect of PEGylation of 36mer and Figure 11B shows a comparison of PEGylated 36mer to best full-length inhibitor 8-17.
Figure 12 shows aptamer inhibition of 50 ng/mL HGF stimulation of 4MBr-5 cells.
Figure 13 shows HUVEC mitogenesis by 10 ng/mL HGF, 10 ng/mL VEGF, or both HGF and VEGF.
Figure 14 illustrates aptamer-mediated inhibition of HUVEC mitogenesis. Figure 14A shows stimulation by both HGF and VEGF inhibited by either HGF or VEGF aptamers or both. Figure 14B illustrates stimulation by HGF alone inhibited by either HGF or VEGF aptamer or both. Figure 14C illustrates stimulation by VEGF alone inhibited by either HGF or VEGF aptamer or both.
Figure 15 depicts ratios of selected to unselected partially 2'-OMe substituted purines in aptamer NX22354.
Figure 16 illustrates 2'-OMe substituted derivatives of NX22354 binding to HGF (average of two experiments).
Figure 17 illustrates binding of SELEX pools to c-met. Figure 17A shows c-Met SELEX 40N7; Figure 17B shows c-Met SELEX 30N8; and Figure 17C shows both SELEXes: a, c pools, 40N7; b, d pools, 30N8.
Figure 18 illustrates binding of c-met SELEX pools to c-met and KDR Ig fusion proteins.
Figure 19 shows binding of c-met 40N7 cloned aptamers to c-met and KDR Ig fusion proteins. Figure 19A shows clone 7c-1 and Figure 19B shows clone7c-3.
Figure 20 illustrates the binding of affinity-enriched RNA pools to immobilized αᵥβ₃. 5'-biotinylated RNA pools were incubated at varying concentrations in 96-well microtiter plates coated with integrin αᵥβ₃. Bound RNAs were detected via the biotin moiety by a chromogenic assay. Data are expressed in absorbance units at 405 nm as a function of input RNA concentration.
Figure 21 illustrates cross-reactivity of aptamer 17.16 (SEQ ID NO:249) to purified integrin β_{IIb}β₃. 5'-biotinylated aptamer 17.16 was incubated at varying concentrations in microtiter wells coated with either integrin αᵥβ₃ or α_{IIb}β₃. Bound RNA was detected via the biotin moiety using a chromogenic assay. Data are expressed as the per cent of the maximum signal to normalize for differences in protein coating.
Figure 22 illustrates cross-reactivity of aptamer 17.16 (SEQ ID NO:249) to purified integrin αᵥβ₅. 5'-biotinylated aptamer 17.16 or a control RNA of similar length and base composition were incubated at varying concentrations in microtiter wells coated with either αᵥβ₃ or αᵥβ₅. Bound RNAs were detected via the biotin moiety by a chromogenic assay. Data are expressed in absorbance units at 405 nm as a function of input RNA concentration.

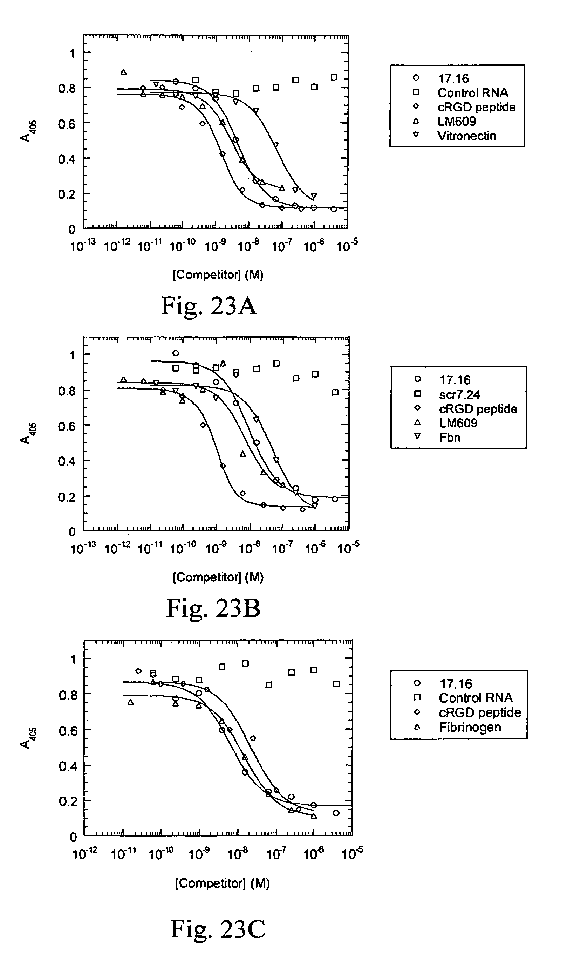

Figure 23 illustrates β3 aptamer inhibition of integrin ligand binding. Biotinylated fibrinogen or vitronectin were incubated in microtiter wells coated with either integrin αᵥβ₃ or α_{IIb}β₃ in the presence or absence of varying concentrations of ligand binding competitors. Competitors included aptamer 17.16 (SEQ ID NO:249), a control RNA of similar length and base composition, a cyclic RGD peptide (cRGD, see Materials and Methods), an αᵥβ₃-specific monoclonal antibody (LM609), or unmodified fibrinogen or vitronectin. Bound ligands were detected via biotin using a chromogenic assay. Data are expressed in absorbance units at 405 nm as a function of input competitor concentration. Figure 23A illustrates competition of vitronectin binding to immobilized αᵥβ₃; Figure 23B illustrates competition of fibrinogen binding to immobilized αᵥβ₃; and Figure 23C illustrates competition of fibrinogen binding to immobilized α_{IIb}β₃. An estimate of the maximum absorbance value was determined for each ligand/integrin pair in the absence of competitor. The baseline absorbance value was determined by adding 5 mM EDTA to the incubation buffer. The maximum and minimum values so determined were (a) 0.914/0.113; (b) 1.042/0.122; and (c) 0.889/0.128.

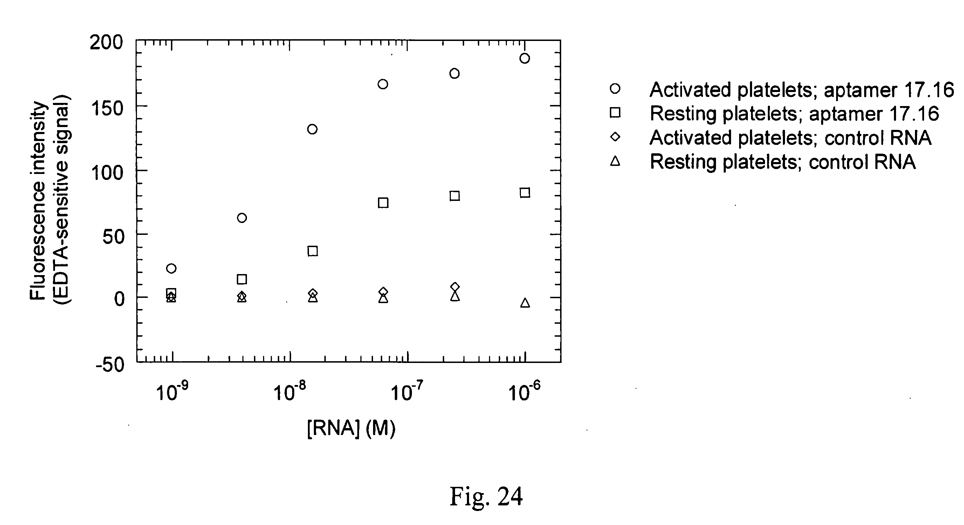

Figure 24 illustrates binding of aptamer 17.16 (SEQ ID NO:249) to activated or resting human platelets. 5'-fluorescein-conjugated aptamer 17.16 or a control RNA of similar length and base composition were incubated at various concentrations with resting or thrombin-activated human platelets (10⁶/mL). Incubations were at room temperature in buffered saline containing divalent cations, 0.1% BSA and 0.01% sodium azide. Mean fluorescence intensity of the sample was determined by flow cytometry, both before and after the addition of EDTA to 5 mM final concentration. The difference in fluorescence intensity between the two samples (the EDTA-sensitive signal) is shown as a function of the concentration of aptamer or control RNA.

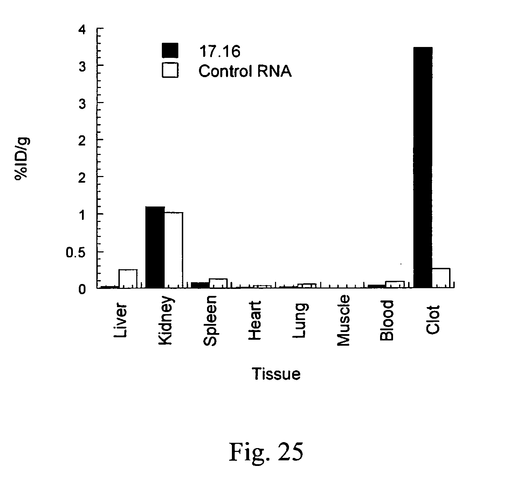

Figure 25 illustrates biodistribution of [^{99m}Tc]-aptamer 17.16 (SEQ ID NO:249) or control RNA in a rabbit venous clot model. A clot derived from human platelet-rich plasma was generated *in situ* by temporary isolation of the jugular vein of an anesthetized rabbit. After restoration of circulation over the clot, [^{99m}Tc]-labeled aptamer or control RNA were injected into the bloodstream of the rabbit via the ipsilateral ear vein. After one hour, the animal was sacrificed and tissues were weighed and counted in a gamma counter. Accumulation of radioactivity in various tissues is reported as the percentage of the injected dose per gram wet weight of tissue.

### Detailed Description of the Preferred Embodiments

The central method utilized herein for identifying nucleic acid ligands is called the SELEX process, an acronym for Systematic Evolution of Ligands by Exponential enrichment. As applied to identifying ligands to HGF and c-met the method comprises: a) contacting the candidate mixture of nucleic acids with HGF or c-met, or expressed domains or peptides corresponding to HGF or c-met; b) partitioning between members of said candidate mixture on the basis of affinity to HGF or c-met; and c) amplifying the selected molecules to yield a mixture of nucleic acids enriched for nucleic acid sequences with a relatively higher affinity for binding to HGF or c-met. As applied to identifying integrins the method comprises: a) contacting the candidate mixture of nucleic acids with integrins, or expressed domains or peptides corresponding to integrins; b) partitioning between members of said candidate mixture on the basis of affinity to integrins; and c) amplifying the selected molecules to yield a mixture of nucleic acids enriched for nucleic acid sequences with a relatively higher affinity for binding to integrins.

### Definitions

Various terms are used herein to refer to aspects of the present invention. To aid in the clarification of the description of the components of this invention, the following definitions are provided.

As used herein, **"nucleic acid ligand"** is a non-naturally occurring nucleic acid having a desirable action on a target. Nucleic acid ligands are often referred to as **"aptamers"**. The term aptamer is used interchangeably with nucleic acid ligand throughout this application. A desirable action includes, but is not limited to, binding of the target, catalytically changing the target, reacting with the target in a way which modifies/alters the target or the functional activity of the target, covalently attaching to the target as in a suicide inhibitor, facilitating the reaction between the target and another molecule. In the preferred embodiment, the action is specific binding affinity for a target molecule, such target molecule being a three dimensional chemical structure other than a polynucleotide that binds to the nucleic acid ligand through a mechanism which predominantly depends on Watson/Crick base pairing or triple helix binding, wherein the nucleic acid ligand is not a nucleic acid having the known physiological function of being bound by the target molecule. In the present invention, the targets are HGF, c-met and integrins or portions thereof. Nucleic acid ligands include nucleic acids that are identified from a candidate mixture of nucleic acids, said nucleic acid ligand being a ligand of a given target, by the method comprising: a) contacting the candidate mixture with the target, wherein nucleic acids having an increased affinity to the target relative to the candidate mixture may be partitioned from the remainder of the candidate mixture; b) partitioning the increased affinity nucleic acids from the remainder of the candidate mixture; and c) amplifying the increased affinity nucleic acids to yield a ligand-enriched mixture of nucleic acids.

As used herein, **"candidate mixture"** is a mixture of nucleic acids of differing sequence from which to select a desired ligand. The source of a candidate mixture can be from naturally-occurring nucleic acids or fragments thereof, chemically synthesized nucleic acids, enzymatically synthesized nucleic acids or nucleic acids made by a combination of the foregoing techniques. In a preferred embodiment, each nucleic acid has fixed sequences surrounding a randomized region to facilitate the amplification process.

As used herein, **"nucleic acid"** means either DNA, RNA, single-stranded or double-stranded, and any chemical modifications thereof. Modifications include, but are not limited to, those which provide other chemical groups that incorporate additional charge, polarizability, hydrogen bonding, electrostatic interaction, and fluxionality to the nucleic acid ligand bases or to the nucleic acid ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Modifications can also include 3' and 5' modifications such as capping.

**"SELEX"** methodology involves the combination of selection of nucleic acid ligands that interact with a target in a desirable manner, for example binding to a protein, with amplification of those selected nucleic acids. Optional iterative cycling of the selection/amplification steps allows selection of one or a small number of nucleic acids which interact most strongly with the target from a pool which contains a very large number of nucleic acids. Cycling of the selection/amplification procedure is continued until a selected goal is achieved. In the present invention, the SELEX methodology is employed to obtain nucleic acid ligands to HGF, c-met and integrins or portions thereof. The SELEX methodology is described in the SELEX Patent Applications.

**"SELEX target"** or **"target"** means any compound or molecule of interest for which a ligand is desired. A target can be a protein, peptide, carbohydrate, polysaccharide, glycoprotein, hormone, receptor, antigen, antibody, virus, substrate, metabolite, transition state analog, cofactor, inhibitor, drug, dye, nutrient, growth factor, etc. without limitation. In this application, the SELEX targets are HGF, c-met and integrins. In particular, the SELEX targets in this application include purified integrins, HGF and c-met, and fragments thereof, and short peptides or expressed protein domains comprising HGF or c-met. Also includes as targets are fusion proteins comprising portions of HGF or c-met and other proteins.

As used herein, "**solid support**" is defined as any surface to which molecules may be attached through either covalent or non-covalent bonds. This includes, but is not limited to, membranes, microtiter plates, magnetic beads, charged paper, nylon, Langmuir-Bodgett films, functionalized glass, germanium, silicon, PTFE, polystyrene, gallium arsenide, gold, and silver. Any other material known in the art that is capable of having functional groups such as amino, carboxyl, thiol or hydroxyl incorporated on its surface, is also contemplated. This includes surfaces with any topology, including, but not limited to, spherical surfaces and grooved surfaces.

As used herein, "**HGF**" refers to hepatocyte growth factor/scatter factor. This includes purified hepatocyte growth factor/scatter factor, fragments of hepatocyte growth factor/scatter factor, chemically synthesized fragments of hepatocyte growth factor/scatter factor, derivatives or mutated versions of hepatocyte growth factor/scatter factor, and fusion proteins comprising hepatocyte growth factor/scatter factor and another protein. "HGF" as used herein also includes hepatocyte growth factor/scatter factor isolated from species other than humans.

As used herein "**c-met**" refers to the receptor for HGF. This includes purified receptor, fragments of receptor, chemically synthesized fragments of receptor, derivatives or mutated versions of receptor, and fusion proteins comprising the receptor and another protein. "c-met" as used herein also includes the HGF receptor isolated from a species other than humans.

As used herein "**integrin**" refers to any integrin. This includes purified integrins, fragments of integrins, chemically synthesized fragments of integrins, derivatives or mutated versions of integrins, and fusion proteins comprising the integrin and another protein. "Integrins" as used herein also includes integrins isolated from a species other than humans.

Note that throughout this application, various references are cited. Every reference cited herein is specifically incorporated in its entirety.

### SELEX

In the preferred embodiment, the nucleic acid ligands of the present invention are derived from the SELEX methodology. The SELEX process is described in U.S. Patent Application Serial No. 07/536,428, entitled "Systematic Evolution of Ligands by Exponential Enrichment," now abandoned, U.S. Patent No. 5,475,096, entitled "Nucleic Acid Ligands" and U.S. Patent No. 5,270,163 (see also WO 91/19813), entitled "Methods for Identifying Nucleic Acid Ligands." These applications, each specifically incorporated herein by reference, are collectively called the SELEX Patent Applications.

The SELEX process provides a class of products which are nucleic acid molecules, each having a unique sequence, and each of which has the property of binding specifically to a desired target compound or molecule. Target molecules are preferably proteins, but can also include among others carbohydrates, peptidoglycans and a variety of small molecules. SELEX methodology can also be used to target biological structures, such as cell surfaces or viruses, through specific interaction with a molecule that is an integral part of that biological structure.

In its most basic form, the SELEX process may be defined by the following series of steps:
1) A candidate mixture of nucleic acids of differing sequence is prepared. The candidate mixture generally includes regions of fixed sequences (i.e., each of the members of the candidate mixture contains the same sequences in the same location) and regions of randomized sequences. The fixed sequence regions are chosen either: a) to assist in the amplification steps described below; b) to mimic a sequence known to bind to the target; or c) to enhance the concentration of a given structural arrangement of the nucleic acids in the candidate mixture. The randomized sequences can be totally randomized (i.e., the probability of finding a base at any position being one in four) or only partially randomized (e.g., the probability of finding a base at any location can be selected at any level between 0 and 100 percent).
2) The candidate mixture is contacted with the selected target under conditions favorable for binding between the target and members of the candidate mixture. Under these circumstances, the interaction between the target and the nucleic acids of the candidate mixture can be considered as forming nucleic acid-target pairs between the target and those nucleic acids having the strongest affinity for the target.
3) The nucleic acids with the highest affinity for the target are partitioned from those nucleic acids with lesser affinity to the target. Because only an extremely small number of sequences (and possibly only one molecule of nucleic acid) corresponding to the highest affinity nucleic acids exist in the candidate mixture, it is generally desirable to set the partitioning criteria so that a significant amount of the nucleic acids in the candidate mixture (approximately 5-50%) are retained during partitioning.
4) Those nucleic acids selected during partitioning as having the relatively higher affinity for the target are then amplified to create a new candidate mixture that is enriched in nucleic acids having a relatively higher affinity for the target.
5) By repeating the partitioning and amplifying steps above, the newly formed candidate mixture contains fewer and fewer unique sequences, and the average degree of affinity of the nucleic acids to the target will generally increase. Taken to its extreme, the SELEX process will yield a candidate mixture containing one or a small number of unique nucleic acids representing those nucleic acids from the original candidate mixture having the highest affinity to the target molecule.

The basic SELEX method has been modified to achieve a number of specific objectives. For example, U.S. Patent Application Serial No. 07/960,093, filed October 14, 1992, now abandoned, and U.S. Patent No. 5,707,796, both entitled "Method for Selecting Nucleic Acids on the Basis of Structure," describe the use of the SELEX process in conjunction with gel electrophoresis to select nucleic acid molecules with specific structural characteristics, such as bent DNA. U.S. Patent Application Serial No. 08/123,935, filed September 17, 1993, entitled "Photoselection of Nucleic Acid Ligands," now abandoned, U.S. Patent No. 5,763,177 and U.S. Patent No. 6,001,577, both entitled "Systematic Evolution of Ligands by Exponential Enrichment: Photoselection of Nucleic Acid Ligands and Solution SELEX" all describe a SELEX based method for selecting nucleic acid ligands containing photoreactive groups capable of binding and/or photocrosslinking to and/or photoinactivating a target molecule. U.S. Patent No. 5,580,737, entitled "High-Affinity Nucleic Acid Ligands That Discriminate Between Theophylline and Caffeine," describes a method for identifying highly specific nucleic acid ligands able to discriminate between closely related molecules, termed Counter-SELEX. U.S. Patent No. 5,567,588, entitled "Systematic Evolution of Ligands by Exponential Enrichment: Solution SELEX," describes a SELEX-based method which achieves highly efficient partitioning between oligonucleotides having high and low affinity for a target molecule. U.S. Patent No. 5,496,938, entitled "Nucleic Acid Ligands to HIV-RT and HIV-1 Rev," describes methods for obtaining improved nucleic acid ligands after SELEX has been performed. U.S. Patent No. 5,705,337, entitled "Systematic Evolution of Ligands by Exponential Enrichment: Chemi-SELEX," describes methods for covalently linking a ligand to its target.

The SELEX method encompasses the identification of high-affinity nucleic acid ligands containing modified nucleotides conferring improved characteristics on the ligand, such as improved *in vivo* stability or improved delivery characteristics. Examples of such modifications include chemical substitutions at the ribose and/or phosphate and/or base positions. SELEX-identified nucleic acid ligands containing modified nucleotides are described in U.S. Patent No. 5,660,985, entitled "High Affinity Nucleic Acid Ligands Containing Modified Nucleotides," that describes oligonucleotides containing nucleotide derivatives chemically modified at the 5- and 2'-positions of pyrimidines. U.S. Patent No. 5,637,459, *supra,* describes highly specific nucleic acid ligands containing one or more nucleotides modified with 2'-amino (2'-NH₂), 2'-fluoro (2'-F), and/or 2'-O-methyl (2'-OMe). U.S. Patent Application Serial No. 08/264,029, filed June 22, 1994, entitled "Novel Method of Preparation of Known and Novel 2' Modified Nucleosides by Intramolecular Nucleophilic Displacement," describes oligonucleotides containing various 2'-modified pyrimidines.

The SELEX method encompasses combining selected oligonucleotides with other selected oligonucleotides and non-oligonucleotide functional units as described in U.S. Patent No. 5,637,459, entitled "Systematic Evolution of Ligands by Exponential Enrichment: Chimeric SELEX," and U.S. Patent No. 5,683,867, entitled "Systematic Evolution of Ligands by Exponential Enrichment: Blended SELEX," respectively. These applications allow the combination of the broad array of shapes and other properties, and the efficient amplification and replication properties, of oligonucleotides with the desirable properties of other molecules.

In U.S. Patent No. 5,496,938, methods are described for obtaining improved nucleic acid ligands after the SELEX process has been performed. This patent, entitled "Nucleic Acid Ligands to HIV-RT and HIV-1 Rev," is specifically incorporated herein by reference in its entirety.

One potential problem encountered in the diagnostic use of nucleic acids is that oligonucleotides in their phosphodiester form may be quickly degraded in body fluids by intracellular and extracellular enzymes such as endonucleases and exonucleases before the desired effect is manifest. Certain chemical modifications of the nucleic acid ligand can be made to increase the *in vivo* stability of the nucleic acid ligand or to enhance or to mediate the delivery of the nucleic acid ligand. See, e.g., U.S. Patent Application Serial No. 08/117,991, filed September 8 1993, now abandoned and U.S. Patent No. 5,660,985, both entitled "High Affinity Nucleic Acid Ligands Containing Modified Nucleotides," and the U.S. Patent Application Serial No. 09/362,578, filed July 28, 1999, entitled "Transcription-free SELEX," each of which is specifically incorporated herein by reference. Modifications of the nucleic acid ligands contemplated in this invention include, but are not limited to, those which provide other chemical groups that incorporate additional charge, polarizability, hydrophobicity, hydrogen bonding, electrostatic interaction, and fluxionality to the nucleic acid ligand bases or to the nucleic acid ligand as a whole. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, unusual base-pairing combinations such as the isobases isocytidine and isoguanidine and the like. Modifications can also include 3' and 5' modifications such as capping. In preferred embodiments of the instant invention, the nucleic acid ligands are RNA molecules that are 2'-fluoro (2'-F) modified on the sugar moiety of pyrimidine residues.

The modifications can be pre- or post-SELEX process modifications. Pre-SELEX process modifications yield nucleic acid ligands with both specificity for their SELEX target and improved *in vivo* stability. Post-SELEX process modifications made to 2'-OH nucleic acid ligands can result in improved *in vivo* stability without adversely affecting the binding capacity of the nucleic acid ligand. Other modifications are known to one of ordinary skill in the art. Such modifications may be made post-SELEX process (modification of previously identified unmodified ligands) or by incorporation into the SELEX process.

In some embodiments, the nucleic acid ligands become covalently attached to their targets upon irradiation of the nucleic acid ligand with light having a selected wavelength. Methods for obtaining such nucleic acid ligands are detailed in U.S. Patent Application Serial No. 08/123,935, filed September 17, 1993, entitled "Photoselection of Nucleic Acid Ligands," now abandoned, U.S. Patent No. 5,763,177 and U.S. Patent No. 6,001,577, both entitled "Systematic Evolution of Nucleic Acid Ligands by Exponential Enrichment: Photoselection of Nucleic Acid Ligands and Solution SELEX," each of which is specifically incorporated herein by reference in its entirety.

The nucleic acid ligands of the invention are prepared through the SELEX methodology that is outlined above and thoroughly enabled in the SELEX applications incorporated herein by reference in their entirety.

### Nucleic acid ligands to HGF and c-met

The SELEX process can be performed using purified HGF or c-met, or fragments thereof as a target. Alternatively, full-length HGF or c-met, or discrete domains of HGF or c-met, can be produced in a suitable expression system. Alternatively, the SELEX process can be performed using as a target a synthetic peptide that includes sequences found in HGF or c-met. Determination of the precise number of amino acids needed for the optimal nucleic acid ligand is routine experimentation for skilled artisans.

In preferred embodiments, the SELEX process is carried out using HGF or c-met attached to a solid support. A candidate mixture of single stranded RNA molecules is then contacted with the solid support. In especially preferred embodiments, the single stranded RNA molecules have a 2'-fluoro modification on C and U residues, rather than a 2'-OH group. After incubation for a predetermined time at a selected temperature, the solid support is washed to remove unbound candidate nucleic acid ligand. The nucleic acid ligands that bind to the HGF or c-met protein are then released into solution, then reverse transcribed by reverse transcriptase and amplified using the Polymerase Chain Reaction. The amplified candidate mixture is then used to begin the next round of the SELEX process.

In the above embodiments, the solid support can be a nitrocellulose filter. Nucleic acids in the candidate mixture that do not interact with the immobilized HGF or c-met can be removed from this nitrocellulose filter by application of a vacuum. In other embodiments, the HGF or c-met target is adsorbed on a dry nitrocellulose filter, and nucleic acids in the candidate mixture that do not bind to the HGF or c-met are removed by washing in buffer. In other embodiments, the solid support is a microtiter plate comprised of, for example, polystyrene.

In still other embodiments, the HGF or c-met protein is used as a target for Truncate SELEX, described in U.S. Patent Application Serial No. 09/275,850, filed March 24 1999, entitled "Truncation SELEX Method," incorporated herein by reference in its entirety.

In preferred embodiments, the nucleic acid ligands thus obtained are assayed for their ability to inhibit the HGF/c-met interaction. In one embodiment, this is accomplished by performing a cell migration assay. Certain cell types, such as A549 lung carcinoma cells, will show increased migration through a Matrigel-coated filter insert (Becton Dickinson) in the presence of HGF. Thus, the degree of inhibition of HGF activity in the presence of an HGF or c-met nucleic acid ligand can be assayed by determining the number of cells that have migrated through the filter in the presence of HGF.

### Methods and compositions for using nucleic acid ligands to HGF and c-met to treat and diagnose disease

Given that elevated levels of c-met and HGF are observed in hypertension, arteriosclerosis, myocardial infarction, and rheumatoid arthritis, nucleic acid ligands will serve as useful therapeutic and diagnostic agents for these diseases. In some embodiments, inhibitory nucleic acid ligands of HGF and c-met are administered, along with a pharmaceutically accepted excipient to an individual suffering from one of these diseases. Modifications of these nucleic acid ligands are made in some embodiments to impart increased stability upon the nucleic acid ligands in the presence of bodily fluids. Such modifications are described and enabled in the SELEX applications cited above.

In other embodiments, nucleic acid ligands to HGF and c-met are used to measure the levels of these proteins in an individual in order to obtain prognostic and diagnostic information. Elevated levels of c-met and HGF are associated with tumors in the liver, breast, pancreas, lung, kidney, bladder, ovary, brain, prostrate, and gallbladder. Elevated levels of HGF and c-met are also associated with myeloma.

In other embodiments, nucleic acid ligands that inhibit the HGF/c-met interaction are used to inhibit tumorigenesis, by inhibiting, for example, angiogenesis and motogenesis.

In one embodiment of the instant invention, a nucleic acid ligand to HGF is used in combination with nucleic acid ligands to VEGF (vascular endothelial growth factor) and/or bFGF (basic fibroblast growth factor) to inhibit tumor metastasis and angiogenesis. The use of multiple nucleic acid ligands is likely to have an additive or synergistic effect on tumor suppression. Nucleic acid ligands that inhibit VEGF are described in U.S. Patent No. 5,849, 479, U.S. Patent No. 5,811,533 and U.S. Patent Application Serial No. 09/156,824, filed September 18, 1998, each of which is entitled "High Affinity Oligonucleotide Ligands to Vascular Endothelial Growth Factor," and each of which is specifically incorporated herein by reference in its entirety. Nucleic acid ligands to VEGF are also described in U.S. Patent No. 5,859,228, U.S. Patent No. 6,051,698, U.S. Patent Application Serial No. 08/870,930, filed June 6, 1997 and U.S. Patent Application Serial No. 09/254,968, filed March 16 1999, each of which is entitled "Vascular Endothelial Growth Factor (VEGF) Nucleic Acid Ligand Complexes, and each of which is specifically incorporated by reference in its entirety. Nucleic acid ligands to bFGF are described in U.S. Patent No. 5,639,868 and U.S. Patent Application Serial No. 08/442,423, filed May 16, 1995, both entitled "High Affinity RNA ligands for Basic Fibroblast Growth Factor," each of which is specifically incorporated herein by reference in its entirety.

### Nucleic acid ligands to integrins

The SELEX process can be performed using purified integrins, or fragments thereof as a target. Alternatively, full-length integrins, or discrete domains of integrins, can be produced in a suitable expression system. Alternatively, the SELEX process can be performed using as a target a synthetic peptide that includes sequences found in an integrin. Determination of the precise number of amino acids needed for the optimal nucleic acid ligand is routine experimentation for skilled artisans.

The SELEX process is carried out using integrins attached to polystyrene beads. A candidate mixture of single stranded RNA molecules is then contacted with the beads. In especially preferred embodiments, the single stranded RNA molecules have a 2'-fluoro modification on C and U residues, rather than a 2'-OH group. After incubation for a predetermined time at a selected temperature, the beads are washed to remove unbound candidate nucleic acid ligands. The nucleic acid ligands that bind to the integrin are then released into solution, reverse transcribed by reverse transcriptase and amplified using the Polymerase Chain Reaction (PCR). The amplified candidate mixture is then used to begin the next round of the SELEX process. Example 5 illustrates one possible way of performing the SELEX process using integrins as targets.

The nucleic acid ligands thus obtained are assayed for their ability to inhibit the interaction of the integrin with its cognate ligand. In one embodiment, this is accomplished by first coating microtiter plates with the appropriate integrin(s). A ligand for the integrin, such as vitronectin or fibrinogen, is then biotinylated and contacted with the coated integrin in the presence of the nucleic acid ligand to be assayed. After incubation for a suitable period of time, the microtiter plate is washed, and the amount of vitronectin or fibrinogen binding to integrin is quantitated by adding a streptavidin-alkaline phosphatase conjugate, followed by a colorimetric substrate for alkaline phosphatase, such as p-nitrophenyl phosphate. The alkaline phosphatase signal in each well of the plate is thus inversely proportional to the effectiveness of the nucleic acid ligand as an inhibitor of the interaction between the bound integrin and its cognate ligand.

The nucleic acid ligands are analyzed using binding to human platelets as an assay. This can be done, for example, by fluorescently labeling the nucleic acid ligand by any of the numerous techniques known in the art. The fluorescent nucleic acid ligand is then contacted with platelets, and the amount of nucleic acid ligand is be quantitated using Fluorescence Activated Cell Sorting (FACS).

The distribution of the nucleic acid ligands can also be studied *in vivo*. Nucleic acid ligands are labeled with a radiolabel used in the art of radioimaging. For example, a nucleic acid ligand can be conjugated to the isotope ^{99m}Tc using one of a number of techniques known in the art. The radiolabelled nucleic acid can then be studied in an animal model of venous thrombosis. For example, a human blood clot can be generated in rabbit vein by first isolating the vein *in situ* by ligation, and then infusing the vein with human platelet-rich plasma and heparin to induce the formation of a blood clot. Blood flow through the vein is then re-established, and the radiolabelled nucleic acid ligand is introduced into the animals blood supply. The distribution of the radiolabelled nucleic acid ligand can then be studied in the rabbit's tissues to determine whether the nucleic acid ligand has accumulated in the clot, rather than in other areas.

### Methods and composition for using nucleic acid ligands to integrins to treat and diagnose disease

The nucleic acid ligands provided have a number of potential uses as therapeutic and diagnostic agents. Nucleic acid ligands that inhibit the interaction between platelet-expressed integrins and their cognate ligands are administered, along with pharmaceutically accepted excipients, in order to prevent the formation of blood clots in patients susceptible to deep vein thrombosis. The nucleic acid ligands are used to treat acute thrombosis formation during and following percutaneous coronary intervention. The nucleic acid ligands are used to treat patients with acute coronary syndromes such as unstable angina or myocardial infarction.

Radiolabelled nucleic acid ligands to platelet-expressed integrins are administered to individuals who are to undergo major surgery, or have suffered major trauma. Such nucleic acid ligands can function as imaging agents for the detection of thrombi, by showing sites in the body where large aggregations of platelets are present. If a thrombosis is detected by radioimaging at a critical site in the body, then anticoagulant and thrombolytic treatment -including treatment with the inhibitory nucleic acid ligands of the instant invention-- can be given locally. The advantage of using such a nucleic acid ligand imaging agent is that the anticoagulant and thrombolytic treatmentswhich can cause harm if administered prophylactically by allowing internal bleeding to continue without efficient clotting-- can be given only to those individuals who definitely have a dangerous thrombosis. Moreover, these treatments can be specifically injected at the site where the thrombosis has been detected by the nucleic acid ligand, instead of injecting higher concentrations into the bloodstream in the hope that some active agent will be carried to all potential sites of thrombosis.

Nucleic acid ligands to αᵥβ₃ integrin can be used to inhibit tumor growth and metastasis. They can also be used to treat ocular diseases including, but not limited to, diabetic retinopathy, retinopathy of prematurity, and macular degeneration. Other diseases for which αᵥβ₃ nucleic acid ligands are useful therapeutic agents include, but are not limited to, endometriosis, psoriasis, rheumatoid arthritis, stroke, osteoporosis, and restenosis.

The following examples are given by way of illustration only. They are not to be taken as limiting the scope of the invention in any way.

### Examples

### Example 1. Generation of nucleic acid ligands to HGF and c-met

### Materials and Methods

In the sections below entitled "Results: HGF" and "Results: c-met", the following materials and methods were used.

Proteins. The HGF protein and c-met-IgG₁-His₆ fusion protein, which were used in the SELEX process, and the KDR-IgG₁-His₆ proteins were purchased from R&D Systems, Inc. (Minneapolis, MN). The human c-met-IgG₁-His₆ fusion protein --described from the amino to the carboxyl terminus-- consists of 932 amino acids from the extracellular domains of the α and β chains of c-met, a factor Xa cleavage site, 231 amino acids from human IgG₁ (Fc domain), and a (His)₆ tag. This protein is referred to in the text and figures as c-met. A similar fusion protein containing the vascular endothelial growth factor receptor KDR will be referred to as KDR.

Anti-HGF monoclonal antibody MAB294 was purchased from R&D Systems, Inc. Human IgG₁ was produced in-house by stable expression from Chinese hamster ovary cells.

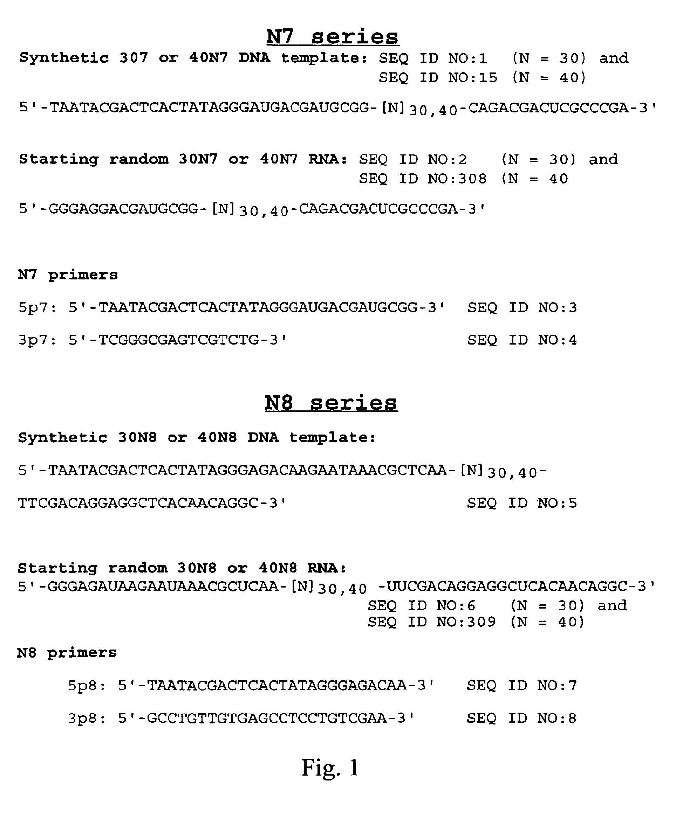

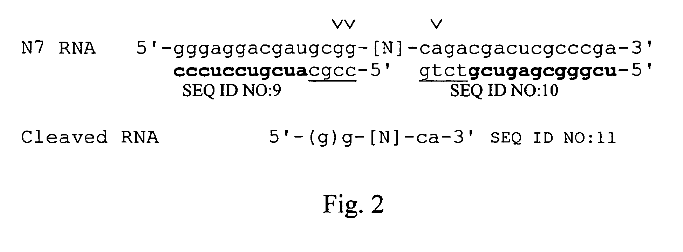

SELEX templates and primers. Standard SELEX templates carrying 30 or 40 random nucleotides flanked by fixed regions of the N7 or N8 series and associated primers (Figure 1) were used as described (Fitzwater and Polisky (1996) Methods Enzymol. 267:275-301). Truncate SELEX was done by the hybridization method described in U.S. Patent Application Serial No. 09/275,850, filed March 24 1999, entitled "Truncation SELEX Method," incorporated herein by reference in its entirety, using RNaseH cleavage primers (Figure 2).

SELEX methods. Initial HGF SELEX experiments were done by two closely-related partitioning methods, both involving separating free from bound RNA on nitrocellulose filters. Conventional SELEX involves mixing target protein and RNA library in HBSMC buffer (hepes-buffered saline, 25 mM hepes, 137 mM NaCl, 5 mM KCl plus 1 mM CaCl₂, 1 mM MgCl₂, pH 7.4), followed by filtration on nitrocellulose under vacuum. Maintaining vacuum, the filter is washed in buffer, followed by vacuum release and RNA extraction. In spot filter SELEX, the protein is applied to a dry nitrocellulose 13 mm filter, allowed to adsorb for several minutes, then pre-incubated in Buffer S (HBSMC buffer plus 0.02% each of ficoll, polyvinylpyrrolidone, and human serum albumin) for 10 minutes at 37°C to remove unbound protein. The wash buffer is removed, and then the RNA library is added in the same buffer, and incubated with the protein-bound filter. The filters are washed by repeated incubations in fresh buffer, followed by RNA extraction.

SELEX was initiated with between 1 and 5 nmoles of 2'-fluoro-pyrimidine RNA sequence libraries containing either a 30 or 40 nucleotide randomized region sequence (Figure 1). The RNA libraries were transcribed from the corresponding synthetic DNA templates that were generated by Klenow extension (Sambrook, Fritsch *et al.* (1989) 3:B.12). The DNA templates were transcribed in 1 mL reactions, each containing 0.25 nM template, 0.58 µM T7 RNA polymerase, 1 mM each of ATP and GTP, 3 mM each of 2'-F-CTP and 2'-F-UTP, 40 mM Tris-HCl (pH 8.0), 12 mM MgCl₂, 1 mM spermidine, 5 mM DTT, 0.002% Triton X-100 and 4% polyethylene glycol (w/v) for at least 4 hours at 37°C. The full-length transcription products were purified by denaturing polyacrylamide gel electrophoresis. Radiolabeled RNA was obtained from transcription reactions as described above, but containing 0.2 nM ATP and 100 µCi of α-³²P-ATP. Alternatively, radiolabeled RNA was obtained by labeling the 5'-end of RNA with α-³²P -ATP (NEN-DuPont), catalyzed by T4 polynucleotide kinase (New England Biolabs). To prepare RNA containing 5'-OH groups for kinase reactions, transcription reactions included 5 mM guanosine.

For conventional filter SELEX, radiolabeled RNA pools were suspended in HBSMC buffer to which HGF protein was added, and incubated at 37°C for 30 minutes to 3 hours depending on the round. Binding reactions were then filtered under suction through 0.45 µm nitrocellulose filters (Millipore), pre-wet with binding buffer. The filters were immediately washed with at least 5 mL of HBSMC buffer. For each binding reaction, a protein-minus control reaction was done in parallel in order to determine the amount of background binding to the filters. The amount of RNA retained on the filters was quantified by Cherenkov counting, and compared with the amount input into the reactions. Filter-retained RNA was extracted with phenol and chloroform, and isolated by ethanol precipitation in the presence of 1-2 µg glycogen.

The isolated RNA was subsequently used as a template for avian myeloblastosis virus reverse transcriptase (AMV-RT, Life Sciences) to obtain cDNA. One hundred pmoles of the 3'-primer (Figure 1) was added to the RNA and annealed by heating for 3 minutes at 70°C, followed by chilling on ice. The 50 µL reaction contained 5 U AMV-RT, 0.4 mM each of dNTPs, 50 mM Tris-HCl (pH 8.3), 60 mM NaCl, 6 mM Mg(OAc)₂, and 10 mM DTT, which was incubated for 45 minutes at 48°C. The cDNA was amplified by PCR with the 5'- and the 3'-primers (Figure 1), and the resulting DNA template was transcribed to obtain RNA for the next round of SELEX.

To minimize selection of undesirable nitrocellulose-binding sequences, beginning in round three, pools were pre-soaked with nitrocellulose filters before incubating with the target protein. This treatment worked well to control background binding and helped ensure that each SELEX round had a positive signal/noise ratio. The progress of SELEX was monitored by nitrocellulose filter-binding analysis of the enriched pools (see below).

Truncate SELEX was performed by the hybridization method described in U.S. Patent Application Serial No. 09/275,850, filed March 24 1999, entitled "Truncation SELEX Method," incorporated herein by reference in its entirety. Briefly, 2'-F-RNA pools were body-labeled during transcription and cleaved by RNaseH using specific cleavage primers to remove the fixed sequences from the SELEX pool (Figure 2). This RNA was then bound to target protein HGF and recovered following partitioning as in a conventional filter SELEX experiment. The recovered RNA was then biotinlyated at its 3' end and hybridized overnight under appropriate conditions with single-stranded full-length complementary strand DNA obtained from the starting SELEX pool, from which the RNA had been transcribed. The RNA/DNA complexes were then captured on streptavidin-coated magnetic beads and extensively washed to remove non-hybridized DNA. The bound DNA in the captured RNA/DNA complexes was then eluted by heat denaturation and amplified using conventional SELEX PCR primers. To complete the cycle, the resulting DNA was then used as a transcription template for generating RNA to be cleaved by RNaseH, and used in the next round of truncate SELEX.

For plate SELEX, a polystyrene well was pre-blocked in 400 µL of blocking agent for 60 minutes at 37°C. The blocking agent was removed and the desired amount of RNA in 100 µL binding buffer was added and incubated for 60 minutes at 37°C. White, polystyrene breakaway wells (catalog #950-2965) used for partitioning were from VWR (Denver, CO). The blocking agents, I-block and Superblock, were purchased from Tropix (Bedford, MA) and Pierce (Rockford, IL), respectively. The preadsorbtion was done to remove any nucleic acids which might bind to the well or the blocking agent. The random and round one libraries were not preadsorbed to plates to avoid loss of unique sequences. C-met protein was diluted in HBSMCK (50 mM HEPES, pH 7.4, 140 mM NaCl, 3 mM KCl, 1 mM CaCl₂, 1 mM MgCl₂), and was adsorbed to polystyrene wells by incubating 100 µL of diluted protein per well for 60 minutes at 37°C. The wells were each washed with three 400 µL aliquots of HIT buffer (HBSMCK, 0.1% I-block, 0.05 % Tween 20), and then blocked in 400 µL of blocking agent for 60 minutes at 37°C. SELEX was initiated by incubating 100 µL of RNA in the protein-bound well for 60 minutes at 37°C. The RNA was removed and the wells were washed with 400 µL aliquots of HIT buffer. Increasing numbers of washes were used in later rounds. The wells were then washed twice with 400 µL water. RNA bound to c-met was eluted by adding 100 µL water and heating at 95°C for 5 minutes and then cooled on ice, followed by reverse transcription.

Nitrocellulose filter-binding. In binding reactions, RNA concentrations were kept as low as possible --between 1 and 20 pM-- to ensure equilibrium in conditions of protein excess. Oligonucleotides were incubated for 15 minutes at 37°C with varying amounts of the protein in 43 µL of the binding buffer. Thirty-two microliters of each binding mixture was placed on pre-wet 0.45 µm nitrocellulose filters under suction. Each well was immediately washed with 0.5 mL binding buffer. The amount of radioactivity retained on the filters was quantitated by imaging. The radioactivity that bound to filters in the absence of protein was used for background correction. The percentage of input oligonucleotide retained on each filter spot was plotted against the corresponding log protein concentration. The nonlinear least square method was used to obtain the dissociation constant (K*_{d}*; reference Jellinek, Lynott et al. (1993) Proc. Natl. Acad. Sci. USA. 90:11227-31).

Competitor titration curves were generated essentially as a standard binding curve, except that the protein and RNA concentrations were kept constant, and the competitor concentration was varied. Competitors were also added at a fixed concentration in binding experiments to increase stringency for purposes of comparing pool binding affinities. In these experiments, the competitor concentration was chosen based on the results from the competitor titration curves.

Molecular cloning and DNA sequencing. To obtain individual sequences from the enriched pools, the PCR products from the final SELEX rounds were cloned using one of two blunt-end cloning kits: Perfectly Blunt (Novagen, Madison, WI), or PCR-Script (Stratagene, La Jolla, CA). Clones were sequenced with the ABI Prism Big Dye Terminator Cycle Sequencing kit (Perkin-Elmer Applied Biosystems, Foster City, CA). Sequences were obtained from an automated ABI sequencer, and text files were collated and analyzed by computer alignment and inspection.

Boundary determinations. 5' and 3' boundaries of RNA aptamers were determined by the method of partial alkaline hydrolysis as described (Jellinek, Green et al. (1994) Biochemistry. 33:10450-6).

Cell assays. Standard cell culture procedures were employed in the course of performing *in vitro* experiments to test aptamer-mediated inhibition of HGF activity. For cell migration assays, monolayers of A549 (lung carcinoma) cells were grown on the topsides of Matrigel-coated filter inserts (Becton Dickinson, Franklin Lakes, NJ) in 24-well plates. The cells adhere to the upper surface of the filter, which is placed in growth medium containing HGF. After two days, the cells are physically removed from the top surface of the filter. The filter is then removed from the insert and stained with crystal violet. Since all cells on the top of the filter are gone, the only cells that remain are those that are have migrated to the bottom of the filter. In the presence of HGF, significantly more cells are found on the bottom of the filter compared to controls without HGF.

Oligonucleotide synthesis and modification. RNA was routinely synthesized by standard cyanoethyl chemistry as modified (Green, Jellinek et al. (1995) Chem Biol. 2:683-95). 2'-Fluoro-pyrimidine phosphoramidite monomers were obtained from JBL Scientific (San Luis Obispo, CA); 2'-OMe purine, 2'-OH purine, hexyl amine, and the dT polystyrene solid support were obtained from Glen Research (Sterling, VA).

For addition of 40K(40,000)-PEG, RNA oligomers were synthesized with an amino-linker at the 5'-position. This was subsequently reacted with NHS-ester 40K-PEG manufactured by Shearwater Polymers, Inc. (Huntsville, AL), and purified by HPLC on a reverse-phase preparative column.

2'-O-methyl purine substitution. Determination of which 2'-OH-purines can be substituted by 2'-OMe purine was done as described (Green (1995) Chem Biol. 2:683-95). Briefly, a set of oligonucleotides was synthesized with a mixture of 2'-OMe amidites and 2'-OH amidites at defined purine positions. The set was designed so that each oligonucleotide contains a subset of partially-substituted purines, and the complete set encompasses all purines. Each aptamer was 5'-end labeled and subjected to limited alkaline hydrolysis followed by binding to HGF protein at two different concentrations, 50 and 100 pM. Following binding, protein-bound RNA was separated by standard nitrocellulose filtration. Bound RNA was recovered and analyzed by high-resolution gel electrophoresis. The fragmented alkaline-hydrolyzed aptamers which were not exposed to HGF were run to establish the cleavage patterns of the unselected aptamers. Hydrolysis occurs only at 2'-OH-purines. If a given position requires 2'-OH for optimal binding to HGF, it appears as a relatively darker band compared to the unselected aptamer at that position.

### Example 2. Results - HGF

Five HGF SELEX experiments were done in total. The first three were done by conventional filter SELEX, while the latter two were done by the hybridization truncate SELEX method described in U.S. Patent Application Serial No. 09/275,850, filed March 24 1999, entitled "Truncation SELEX Method," incorporated herein by reference in its entirety. HGF SELEX 1 was done with 30N7 2'-F-RNA for thirteen rounds of conventional filter binding. HGF SELEX 2 was done with 30N8 2'-F-RNA for thirteen rounds of conventional filter binding. HGF SELEX 3 was done with 30N7 2'-F-RNA for seven rounds by spot filter binding, followed by eight rounds of filter binding. HGF SELEX 4 was done by hybridization filter SELEX for three rounds, starting with pool 8 from HGF SELEX 1. HGF SELEX 5 was done by hybridization filter SELEX for three rounds, starting with pool 11 from HGF SELEX 3. HBSMC buffer was used in conventional SELEX reactions, and in spot filter SELEX, blocking agents were added as described in Materials and Methods.

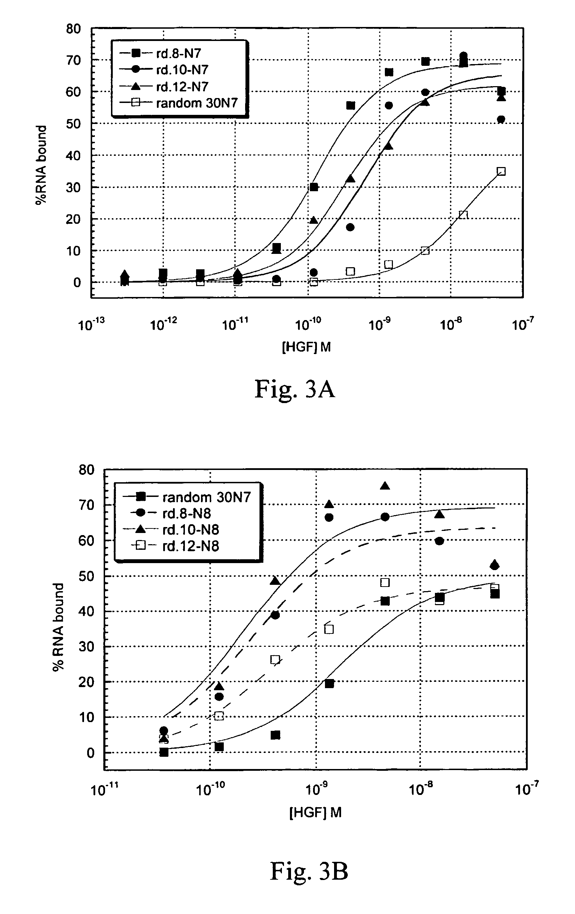

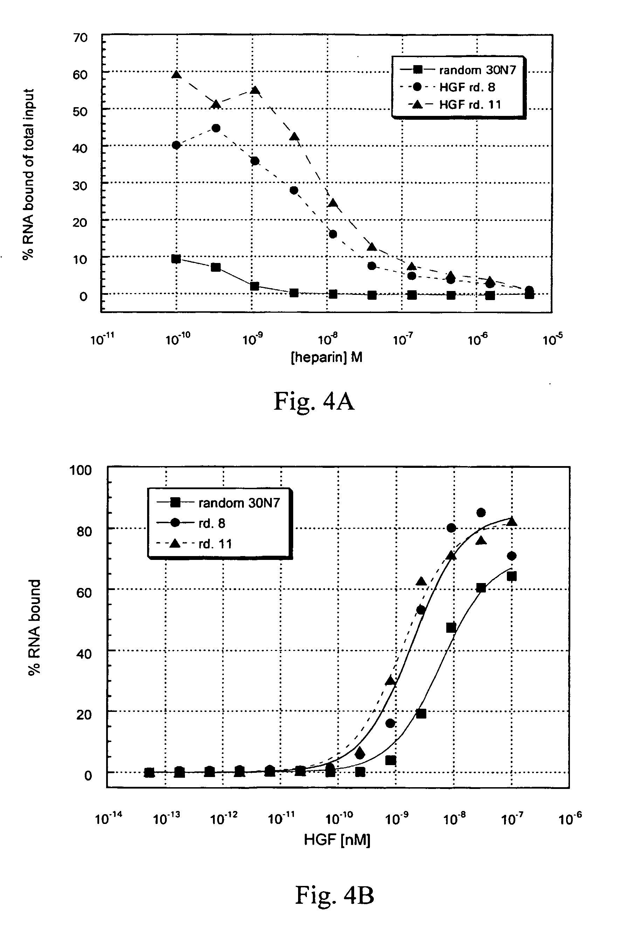

RNA pool binding with and without competitors heparin and tRNA. To evaluate SELEX progress, binding curves with purified HGF protein were routinely done with evolved pools during the course of these experiments. Representative binding curves are shown for HGF SELEX experiments 1 and 2 (Figure 3). These data were used to ascertain when SELEX was complete in that further progress was not likely to occur by performing additional rounds. HGF SELEX 1 reached its maximal binding by round 8, with a binding affinity of approximately 0.1 nM' (Figure 3A; earlier rounds and round 9 were examined in other experiments). HGF SELEX 2 reached its maximal binding by round 10, with a binding affinity of approximately 0.1 nM (Figure 3B). HGF SELEX 3 reached its maximal binding by round 11, after seven rounds of spot filter partitioning followed by four rounds of conventional filter SELEX (see Figure 4B). A SELEX experiment which was deemed complete was characterized by cloning and sequencing (see below).

HGF, like other proteins which have large clusters of positively charged amino acids, exhibits a high degree of non-specific binding to polyanionic compounds. For example, random RNA pools bind to HGF with low nanomolar affinity, similar to the value reported for HGF binding to heparin, a polyanionic sulfated polysaccharide known to have an important biological role in HGF function (Zioncheck, Richardson et al. (1995) J Biol Chem. 270:16871-8). Competition binding to heparin as well as the non-specific competitor tRNA was done to provide an additional means of evaluating SELEX progress. This was done because the binding of random and evolved RNA pools to HGF occurs in a high-affinity range that makes it difficult to monitor progress. In other words, random RNA binds so well to HGF that the affinity enhancement of the evolved pools may not be adequately assessed in conventional binding experiments in the absence of competitor.

RNA pools from HGF SELEX 3 were subjected to competition with heparin (Figure 4A). This experiment demonstrates that random RNA is considerably more sensitive to competition for binding to HGF than are the evolved pools. These data are compared to those obtained from a binding curve with the same three RNA pools (Figure 4B). In the absence of heparin competition, binding of random RNA to HGF is nearly as good as that of the evolved pools, whereas the heparin competition reveals that the evolved pools are significantly different in composition from random RNA. In addition, while rounds 8 and 11 are indistinguishable in conventional binding curves, round 11 exhibits improved binding based on increased resistance to heparin competition. These data contributed to the choice of round 11 as the maximally binding pool from which we cloned and sequenced.

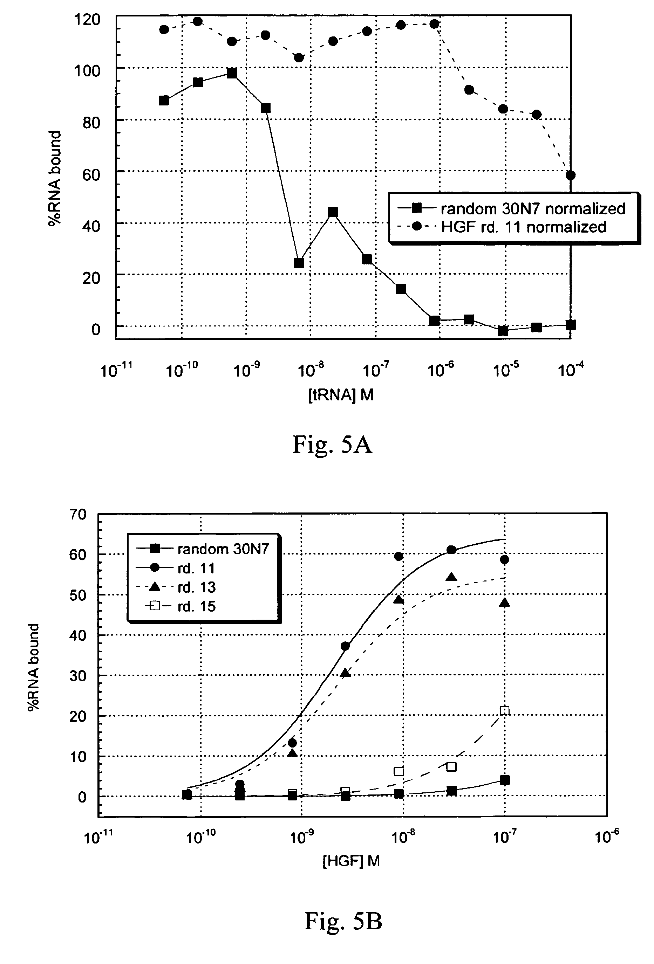

A similar, but more pronounced, effect was observed with tRNA as the competitor (Figure 5A). These data indicate that the round 11 pool from HGF SELEX 3 are at least four orders of magnitude more resistant to competition for binding to HGF than is random RNA. From these curves, it was determined that 800 nM tRNA is the maximum concentration at which complete binding of evolved RNA persists. Therefore, binding curves were done at this tRNA concentration to compare the binding of different evolved pools (Figure 5B). These curves were useful in determining that further SELEX rounds beyond round 11 did not improve binding.

Typical data from a similar set of binding competition experiments done for latter rounds of HGF SELEX 1 are summarized in Table 1.

Cloning and sequence analysis of HGF SELEXes 1, 2 and 3. Following determination of pool binding affinities for HGF, the optimal SELEX pools were cloned and sequenced in order to isolate and characterize individual aptamers. Data from 30N7 HGF SELEX 1 and 3 are summarized in Table 2, including binding affinities for many of the aptamers. A similar data set was generated for 30N8 HGF SELEX 2 (Table 3). Sequences from HGF SELEX 1, 2 and 3 are designated 8-seq. number, 10-seq. number, and 11-seq. number, respectively, referring to the total number of SELEX rounds each cloned pool was subjected to. Sequences were analyzed and organized into groups with significant homology. Motifs were analyzed and predicted structures were drawn in order to analyze key features responsible for binding to HGF.

Inhibition of HGF-mediated stimulation of cell proliferation. HGF, while not a potent mitogen, does stimulate moderate proliferation of many cell lines, which can be measured by incorporation of ³H-thymidine. The inhibitory activity of HGF aptamers was assayed by measuring their effect on proliferation of human umbilical vein endothelial cells (HUVECs), or monkey bronchial epithelial (4MBr-5) cells. Based on the binding data and sequence family analysis, fourteen aptamers were chosen for analysis *in vitro* because they bind to HGF with high affinity and are representative of different sequence families. The sequences are shown in Table 4, aligned by a rough consensus that contains bases in common to several families. All sequences are 30N7 except 10-2 which is 30N8.

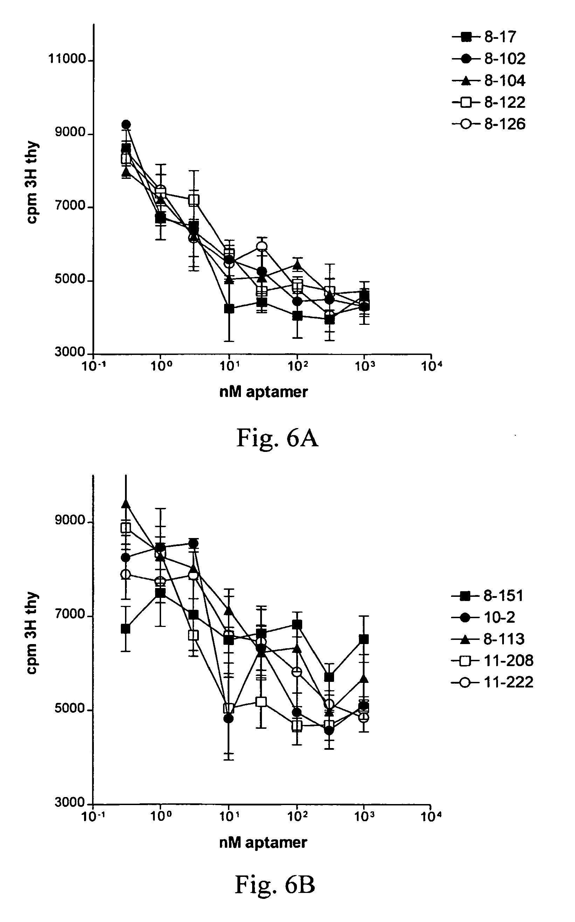

HGF stimulates proliferation of HUVECs by about two-to-three-fold (data not shown). The initial experiment indicated that aptamers 8-17, 8-102, 8-104, 8-122, 8-126, 10-2 and 11-208 were effective inhibitors of HGF-induced HUVEC proliferation with K*ᵢ* values in the low nanomolar range (Figure 6). Aptamers 8-113 and 11-222 were less effective and 8-151 exhibited little or no concentration-dependent inhibition. The latter observation is consistent with the fact that aptamer 8-151 does not bind HGF with high affinity and actually binds worse than the random pool.

The following approaches were taken to reduce the length of aptamers which retained significant inhibition of HGF: 1) boundary determinations by biochemical separation of partially hydrolyzed aptamers; 2) sequence motif analysis and educated guessing; and 3) truncate SELEX.

Boundaries and truncation. Boundary determinations were done for a subset of aptamers that demonstrated *in vitro* inhibition of HGF activity. Using a standard alkaline hydrolysis procedure with 5'-end-labeled RNA, the 3'-boundaries of aptamers 8-17, 8-102, 8-104, 8-126, 10-1 and 10-2 were examined. Additionally, 3'-end-labeled RNA was used for 5'-boundary experiments with aptamers 8-17 and 8-102. These experiments were mostly uninformative, probably because the high degree of non-specific binding of RNA fragments, regardless of size, obscured the binding of truncated high-affinity aptamers to HGF. Non-specific binding of virtually all fragments gave no boundary information, and reducing the protein concentration did not help. Instead, we tried to use polyanionic competitors tRNA and heparin to eliminate nonspecific binding to reveal the actual boundaries. The competitors reduced non-specific binding, and HGF was predominantly bound only by full-length aptamers, revealing no boundary information beyond the possibility that full-length aptamers are strongly preferred.

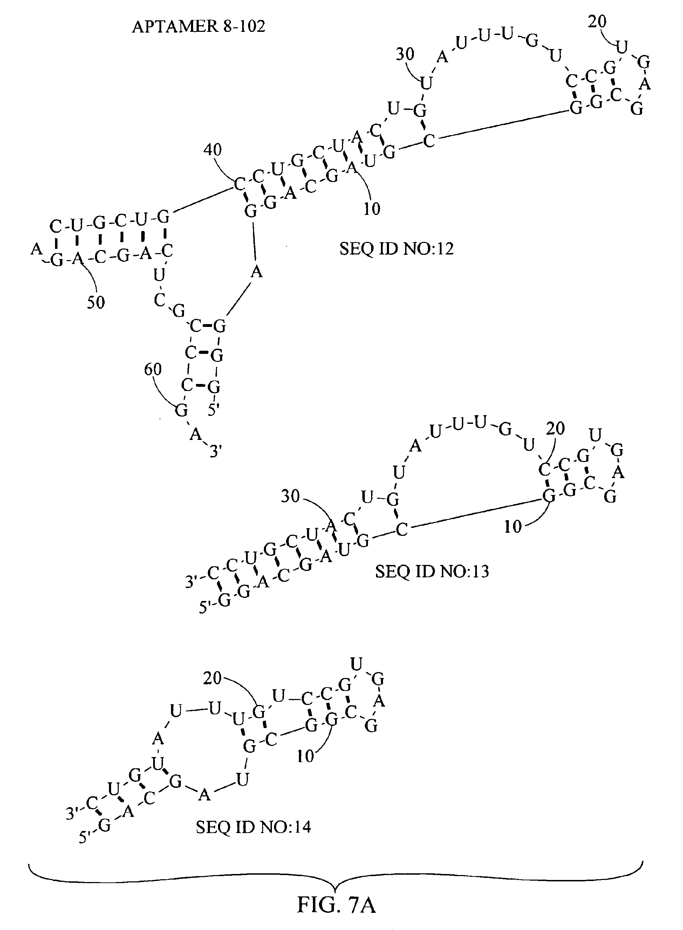

The sole exception was aptamer 8-102 which had a plausible 3'-boundary between two possible endpoints which made sense with respect to computer-predicted structures (Figure 7A). Based on the boundary data and structural data, two truncates of aptamer 8-102 were synthesized and analyzed for binding to HGF. The sequence of the full-length aptamer and the two truncates are shown, with fixed regions underlined:
gggaggacgaugcggcgagugccuguuuaugucaucguccgucgucagacgacucgcccga (8-102) (SEQ ID NO:12)
ggacgaugcggcgagugccuguuuaugucaucgucc (36mer) (SEQ ID NO: 13)
gacgaugcggcgagugccuguuuauguc (28mer) (SEQ ID NO:14)

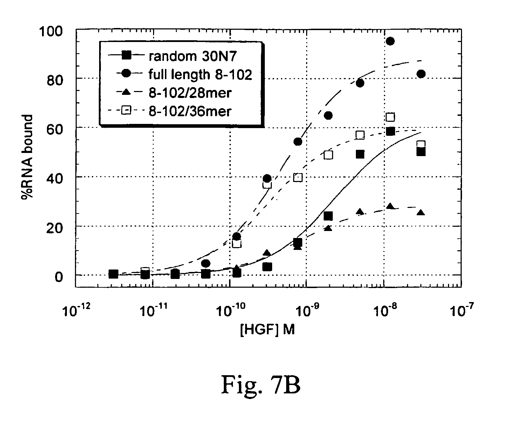

In binding to HGF, the 36mer bound almost as well as the full-length aptamer, while the 28mer bound no better than random 30N7 (Figure 7B), suggesting that the boundary data were correct.

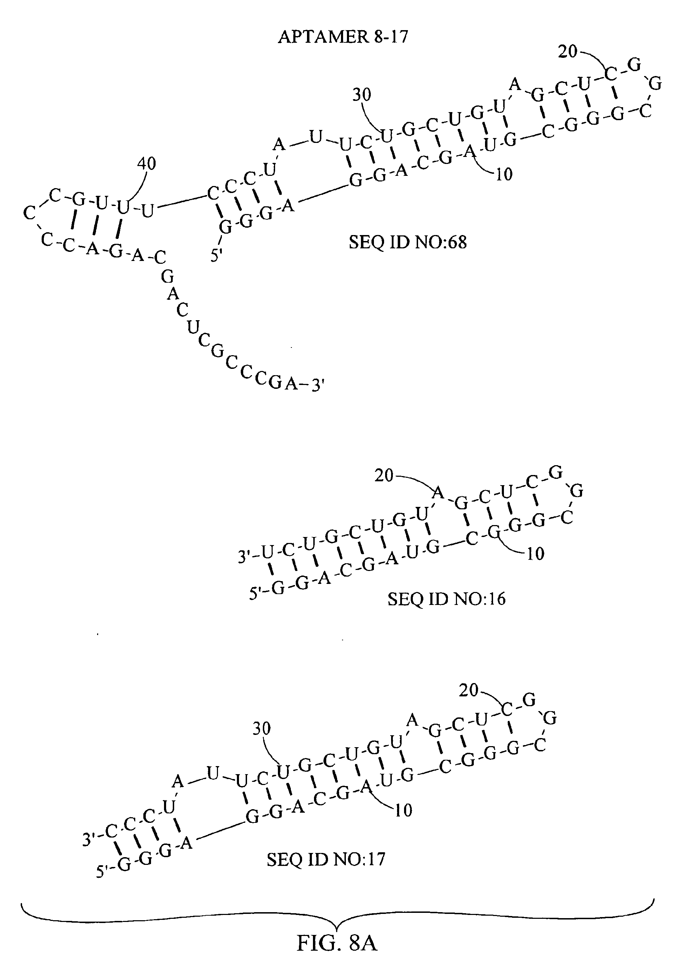

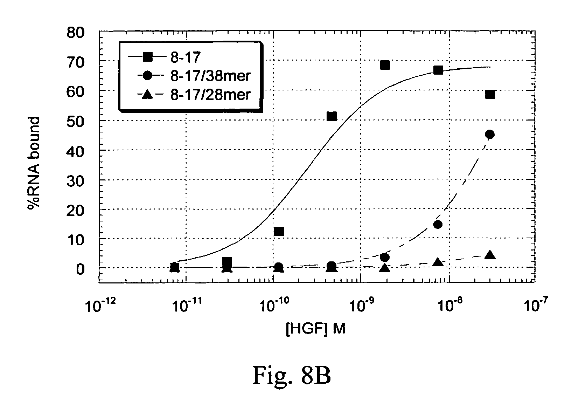

Truncation by sequence structure prediction. Several attempts were made to base truncation on motif analysis and predicted structures, but these did not succeed in producing truncates which retained binding to HGF. For example, aptamer 8-17 folded into a reasonable predicted structure which suggested two obvious points of truncation from its 3' terminus, into a 38mer or 28mer (Figure 8A). However, binding analysis revealed that neither of these truncates retained significant binding to HGF (Figure 8B). These data suggest either that the predicted structure is incorrect or that some of the 3' region past base 38 is critical for high-affinity binding of aptamer 8-17 to HGF. These two hypotheses are not mutually exclusive. Nevertheless, we did not succeed in obtaining a useful truncate of 8-17 by boundary and structural prediction.

Truncate SELEX. In order to generate additional short aptamers, advanced rounds of the earlier SELEX experiments were subjected to additional rounds of truncate SELEX, using the Truncation SELEX method described in U.S. Patent Application Serial No. 09/275,850, filed March 24 1999, entitled "Truncation SELEX Method," incorporated herein by reference in its entirety. Binding of RNaseH cleaved pools was examined to determine which were the appropriate rounds to use to initiate truncate SELEX (data not shown). None of the RNaseH-cleaved evolved pools was clearly superior to another in binding to HGF, therefore, the pools which had been previously cloned were chosen to use in truncate SELEX. The encouraging result from this experiment was that after RNaseH treatment, the evolved pools bound better to HGF than did random RNA, suggesting that even in the absence of the fixed regions, significant binding affinity was retained. This observation was sufficient evidence to suggest that truncate SELEX could enrich for sequences that bound to HGF in the absence of fixed regions.

Three rounds of hybridization truncate SELEX were done in parallel, using as starting pools HGF SELEX 1 round 8 and HGF SELEX 3 round 11. The truncate SELEX rounds were done at equi-molar RNA and protein, starting at 1 nM and decreasing to 0.5 and 0.1 nM. Signal-to-noise ratios were very high during selection. Subsequent manipulations were satisfactory even though the amount of recovered RNA was sub-picomolar.

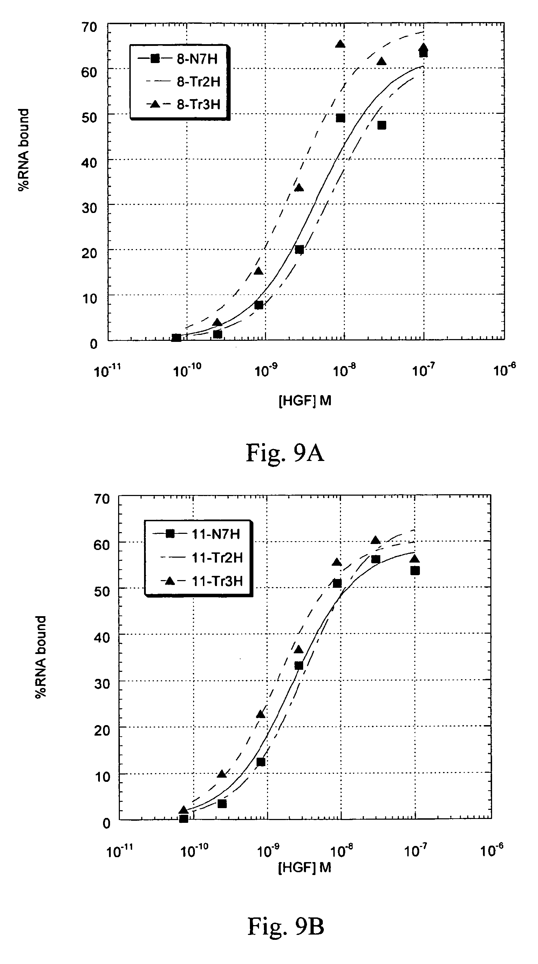

To evaluate the progress of the SELEX, binding affinities of truncate rounds two and three were determined and compared to those of the RNaseH-cleaved starting pools (Figure 9). For both SELEXes, the third round pools bound with improved affinity for HGF compared with the earlier rounds. Interestingly, the second rounds did not bind HGF better than the staring material. The dissociation constants for the third round truncate SELEX pools are 1-2 nM, representing a 2-3 fold improvement. While the magnitude of this improvement is not large, it is probably significant since HGF as a target did not easily yield affinity enrichment, probably because of its intrinsically high affinity for RNA.

The two pools were cloned and sequenced, and binding affinities were determined (Table 5). The truncated aptamer with the best binding affinity, Tr51, is among several sequences which are novel, that is, they were not found in the clones sequenced from the full-length SELEX pools. The emergence of novel sequences suggests that the truncate SELEX succeeded in amplifying aptamers that were relatively rare in the full-length pools. Aptamer Tr51 appeared more frequently than any other sequence, consistent with the observation that it has better binding affinity than any other truncate. Other sequences that appeared multiple times also tend to be those with binding affinities near or better than the pool K*_{d}* of 1-2 nM.

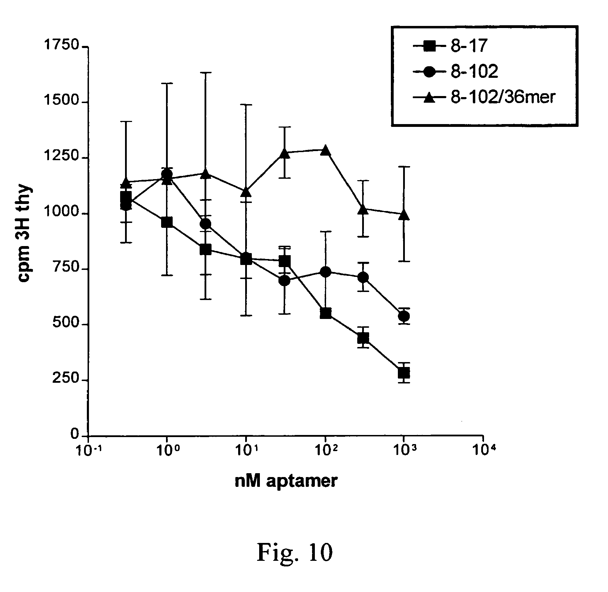

HGF inhibition by the 36mer aptamer modified with 40K-PEG. The 36mer derivative of aptamer 8-102, described above, was tested for inhibition *in vitro* in a 4MBr-5 cell proliferation assay (Figure 10). Although the 36mer retained high-affinity binding to HGF, it did not retain inhibitory activity *in vitro* comparable to its parent aptamer 8-102 and aptamer 8-17 (Figure 10).

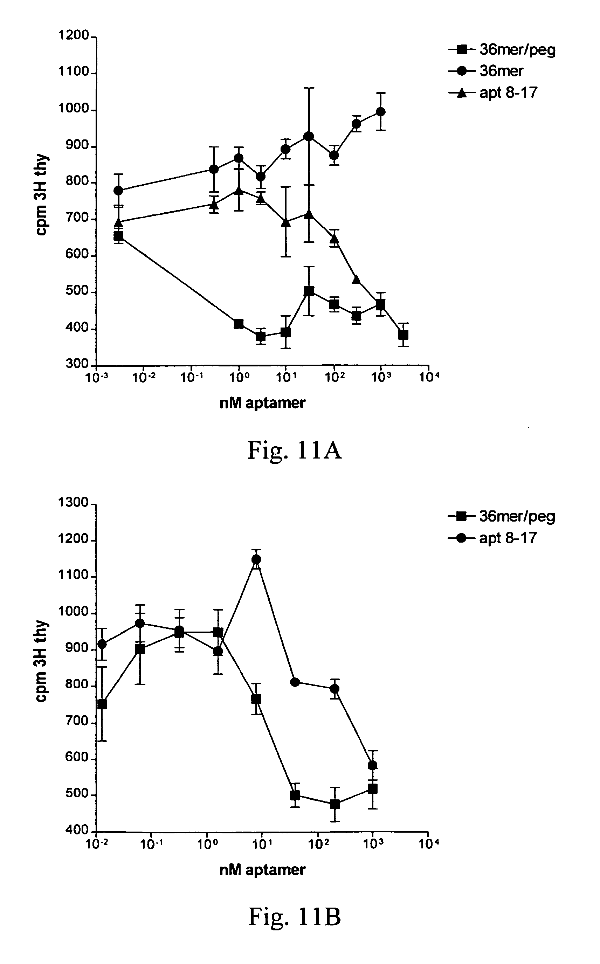

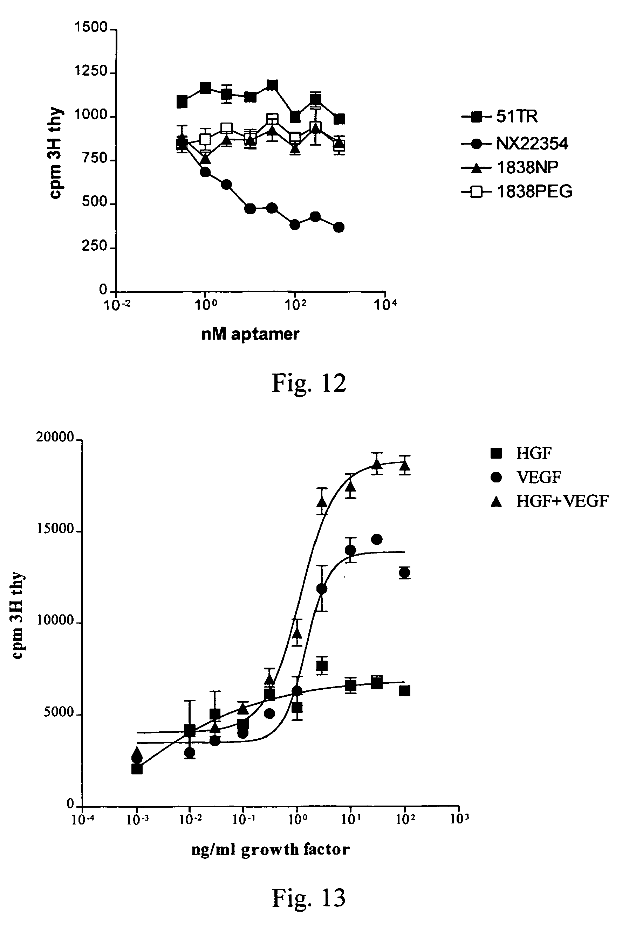

In order to improve the activity of the 36mer, it was tested in a formulation with a 3'-dT cap and 5'-40K PEG. The modified aptamer, designated NX22354, was tested for inhibition of HGF-mediated proliferation 4MBr-5 cells (Figure 11A). The data indicate that the 36mer-PEG aptamer inhibits HGF, and that it performs at least as well as the full-length aptamer 8-17, which had previously exhibited the strongest inhibition of all aptamers tested. As expected, the non-PEGylated 36mer did not inhibit HGF, suggesting that the addition of PEG and/or the 3'-cap contribute to the aptamer's bioactivity. This experiment was also done at lower aptamer concentrations, supporting the previous result and showing more clearly that the 36mer-PEG aptamer is a better inhibitor that the 8-17 full-length aptamer (Figure 11B). Also tested by this assay was a non-binding aptamer containing a 3'-dT cap and 5'-40K PEG, the VEGF aptamer NX1838, which had no effect on HGF stimulation (Figure 12). In this same experiment, a non-PEGylated version of NX1838 and the truncate SELEX aptamer Tr51 were shown to have no inhibitory effect on HGF (Figure 12). This suggests that Tr51, similar to the 36mer base aptamer of NX22354, may require 5'-40K-PEG to inhibit HGF function.

Inhibition of HGF-mediated stimulation of cell migration. HGF readily stimulates cell movement, hence the name, scatter factor. The inhibitory effect of HGF aptamers was assayed by measuring their effect on A549 cell migration across a Matrigel coated membrane with 8.0 micron pores as described in Materials and Methods (Table 6). The NX22354 aptamer fully inhibited HGF-mediated migration at both 1 and 0.2 µM concentrations, but at 0.04 µM, the effect was negligible. The monoclonal antibody control (sample 3) was moderately effective at the 1 µg/mL dose, which is above its published EC₅₀ value of 0.1-0.3 µg/mL for inhibition of 4MBr-5 cell proliferation.

Combined inhibitory effect of HGF and VEGF aptamers on HUVEC proliferation. It was reported that VEGF and HGF have an additive stimulatory effect on HUVEC proliferation (Van Belle (1998) Circulation. 97:381-90). This effect was observed when VEGF and HGF were added, singly and in combination, to HUVECs, and incorporation of ³H-thymidine was measured (Figure 13). As expected, stimulation by HGF was relatively weak compared with that of VEGF and together, the stimulatory effect was greater than that elicited by VEGF alone.

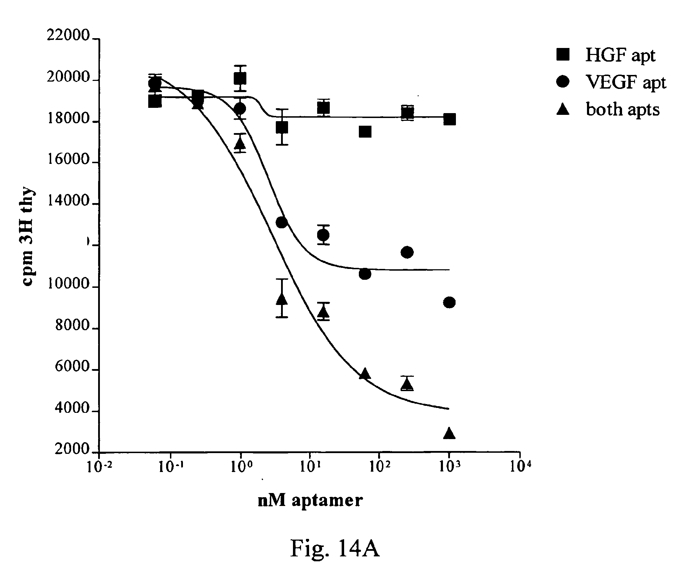

Based on these curves, we chose to add each cytokine at 10 ng/mL for optimal stimulation in the aptamer inhibition experiments. The effect of adding one or both aptamers to the doubly-stimulated cells in the presence of both growth factors was then tested (Figure 14A). It was observed that each aptamer partially inhibits the stimulation and that both aptamers result in complete inhibition. Interestingly, the magnitude of the inhibitory effect of each aptamer roughly corresponds with the magnitude of the stimulation conferred by each cytokine. This observation suggests that the stimulatory effect of each cytokine can be inhibited independently, and that the two cytokines stimulate HUVECs independently.

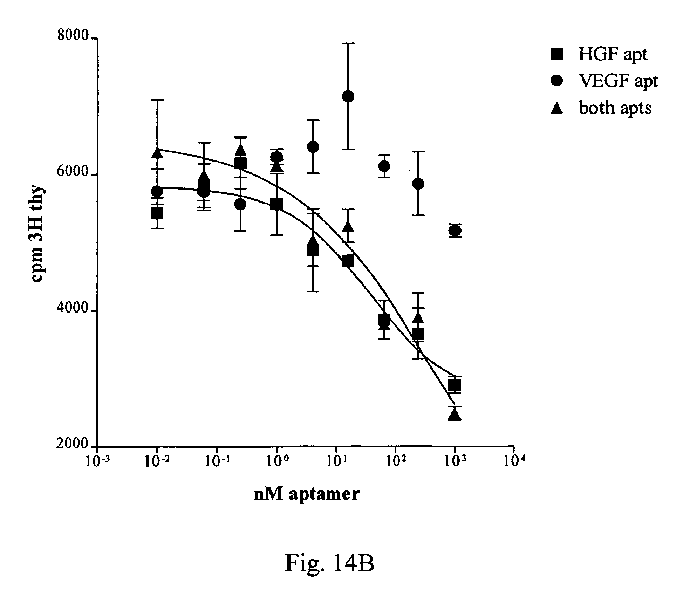

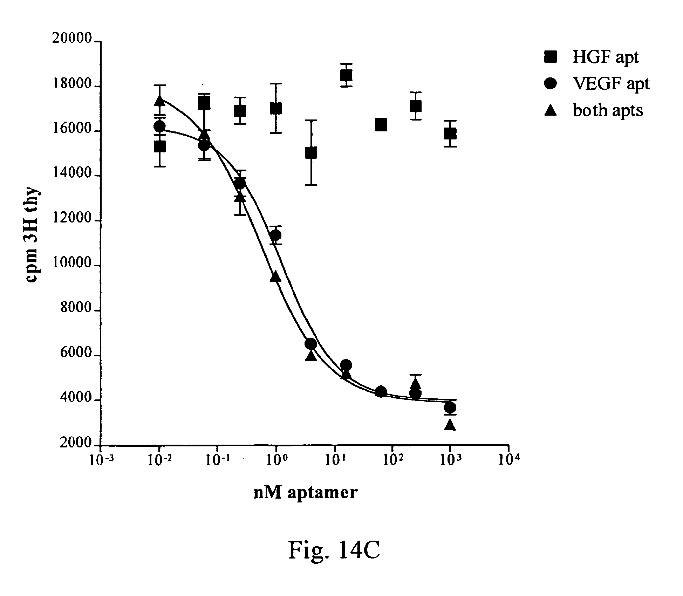

Figures 14B and C depict controls in which each cytokine was administered separately, demonstrating that the HGF and VEGF aptamers do not cross-react, that is, each aptamer affects only the cytokine against which it was selected. For the HGF stimulated cells, inhibition by the HGF aptamer NX22354 was observed, but not by the VEGF aptamer NX1838 (Figure 14B). Conversely, stimulation by VEGF was inhibited by the VEGF aptamer NX1838, but was unaffected by the HGF aptamer NX22354 (Figure 14C).

These data, along with the fact that HGF, like VEGF, is an angiogenesis factor make it intriguing to consider dual administration of VEGF and HGF aptamers to treat tumors. Furthermore, the availability of aptamers which inhibit other growth factors suggests further combinations of the VEGF or the HGF aptamer in combination with other aptamers, for example, aptamers that inhibit bFGF, platelet-derived growth factor (PDGF), transforming growth factor beta (TGF), keratinocyte growth factor (KGF), and/or their receptors allowing for the possibility that any combination of these inhibitors may be relevant. The goal is to have an array of aptamer-inhibitors of cytokines and their receptors and to be able to tailor combination treatments for specific disease states.

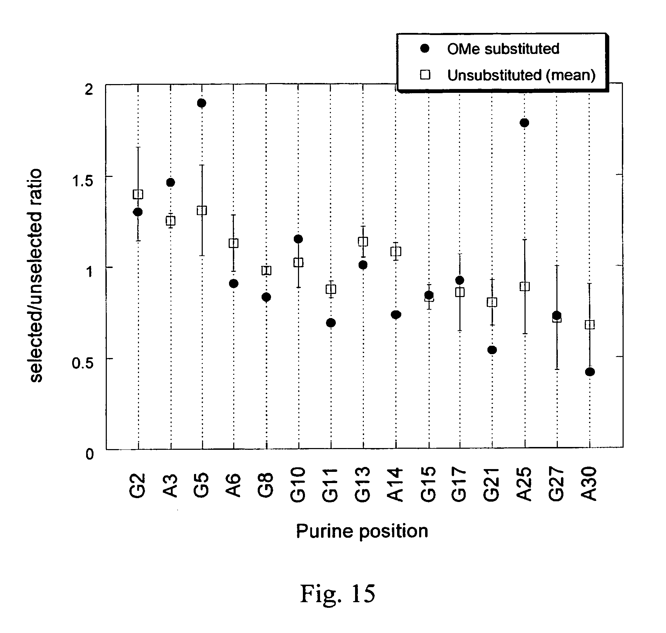

2'-O-methyl-purine substitution of HGF aptamer NX22354. To improve the stability and pharmacokinetics of NX22354, we determined which of the 17 2'-OH purines could be replaced. This was done by synthesizing four partially substituted 2'-OMe purine variants of the base sequence of NX22354, followed by analysis as described above in the Materials and Methods section. The four partially-substituted oligonucleotides were synthesized with a 1:1 ratio of 2'-OMe amidite:2'-OH amidite (Table 7). The data analysis measures the ratios of the selected to unselected RNA at each substituted purine position, based on quantitation of bands from the gel. The data are summarized by position (Figure 15). At each position, the three unsubstituted aptamers provide an important comparison, which is expressed as an average of the three unsubstituted aptamers with standard deviation represented by the error bars. Points that occur at ratios higher than that of the nearby positions are likely to require 2'-OH for binding.

The data strongly indicate that two positions, G5 and A25, do not tolerate 2'-OMe substitution. Two other positions, A3 and G10, show a slight preference above the standard deviation of the unselected RNA.

The set of OMe aptamers were also examined for binding to HGF (data not shown). The binding data indicate that the OMe1 and OMe3 bind as well as the parent unsubstituted 36mer, whereas OMe2 and OMe4 bind do not bind as well. This suggests that the substitutions in OMe2 and OMe4 are less well tolerated with respect to HGF binding in solution, consistent with the fact that OMe2 and OMe4 are substituted at A25 and G5, respectively.

To confirm these results, two aptamers were synthesized which are fully 2'-OMe substituted at the apparently well-tolerated positions. The sequences are shown below, with the 2'-OH-purines shown underlined. All other purines have 2'-OMe and the pyrimidines are 2'-fluoro substituted.
4x Sub 2'-OH. GGACGAUGCGGCGAGUGCCUGUUUAUGUCAUCGUCC
   (SEQ ID NO:186)
2x Sub 2'-OH. GGACGAUGCGGCGAGUGCCUGUUUAUGUCAUCGUCC
   (SEQ ID NO:187)
Sequence 4x Sub 2'-OH contains all four of the 2'-OH-purines in question, while 2x Sub 2'-OH has only the two 2'-OH-purines most likely to be required.

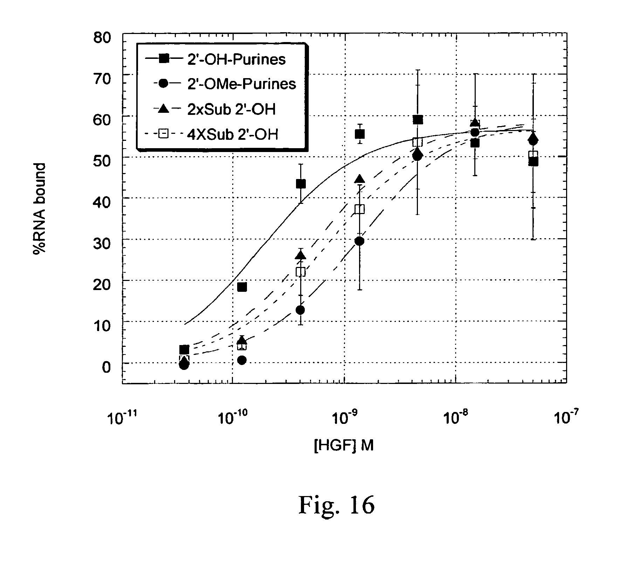

Binding of these oligomers to HGF was examined compared to the unsubstituted parent and the fully 2'-OMe substituted RNA (Figure 16). Based on these binding curves, NX22354 tolerates 2'-OMe substitution at all purines except G5 and A25 (aptamer 2x Sub 2'-OH) with minimal loss of binding affinity. The other two positions in question apparently are not required to be 2'-OH since aptamer 4x Sub 2'-OH binds no better than aptamer 2x Sub 2'-OH.

Two aptamers have been synthesized with 5'-40K-PEG and a 3'-dT cap: one is fully 2'-OMe substituted and the other contains 2'-OH at positions G5 and A25. One of these will presumably supplant NX22354 as the lead HGF aptamer for further testing *in vitro* and *in vivo.*

### Example 3. Results - c-met

c-met SELEX. In the c-met plate SELEX experiments, the concentration of nucleic acids was lowered initially, but then raised in later rounds so that the ratio of the nucleic acid to protein would be very high. This was done in order to create conditions of high stringency which may select for higher affinity aptamers. Stringency was also applied by increasing the number of washes.

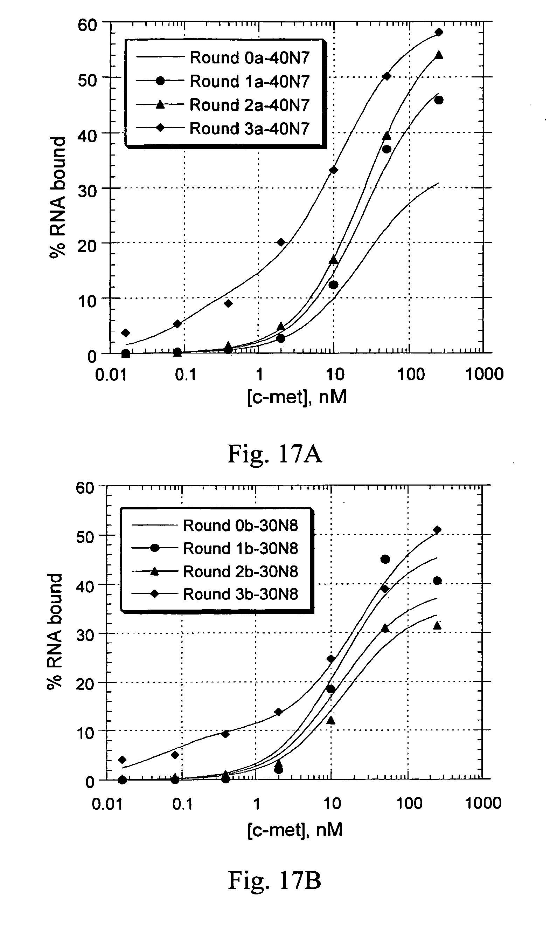

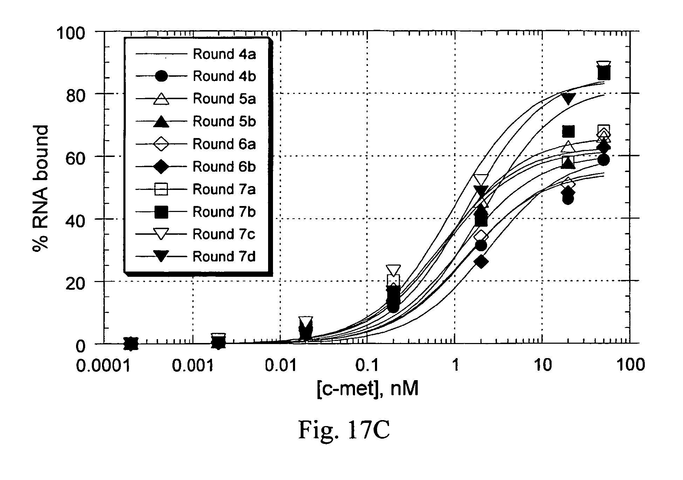

SELEX pool binding. Binding of SELEX pools to c-met was assessed through round 7 (Figure 17). The binding data indicate that the SELEX resulted in about a 20 fold improvement in K*_{d}* from 20 nM to 1 nM for both (40N7) (Figure 17A) and (30N8) (Figure 17B) pools.

Since the c-met protein used in SELEX is an IgG fusion protein, random 40N7 and round 7c RNA pools, were tested for binding to human IgG₁ and c-met. The binding dissociation constants are set forth in Table 8. The affinity of round 7c RNA for both IgG₁ and c-met proteins improved about 50-fold. There are several interpretations of this result. Aptamers may have been selected which bind with better affinity to both proteins. This assumes that the difference in binding between IgG₁ and c-met is due to c-met specific aptamers. However, the two proteins were made in different cell lines that may have different glycosylation patterns which could influence binding. Thus, if the differences in affinity are due to differences between the free IgG₁ protein and the IgG₁ domain in c-met, then there might be few if any c-met specific aptamers in the round 7 pool.

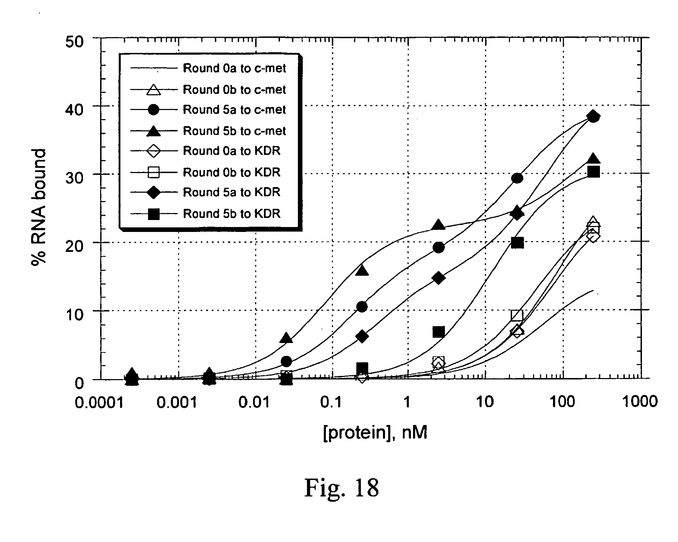

In order to address these issues further, random and round 5 RNA pools from both libraries were examined for binding to the c-met and KDR proteins (Figure 18). Both of these proteins were made in the same cell line and contain the same IgG₁-His₆ sequence. Random RNA from both libraries binds about the same to each protein (K*_{d}*= ~50 nM). Round 5 from both libraries of c-met SELEX binds better to c-met than to KDR (~100-fold better for the 30N8 pool and 3-fold better for the 40N7 pool). However, round 5 RNA pools do bind better than random RNA to KDR. These results imply that, while there are probably aptamers that bind to human IgG₁ or (HIS)₆ tag in the round 5 pools, there may also be c-met aptamers.

Detection of IgG aptamers by PCR. Another approach for determining if IgG₁ aptamers are present in the SELEX pools was to subject them to PCR. Predominant IgG₁ aptamers have been isolated from N7 type libraries that have a known sequence (Nikos Pagratis and Chinh Dang, personal communication). For the PCR, a DNA oligonucleotide:
ML-124; 5'-ACGAGTTTATCGAAAAAGAACGATGGTTCCAATGGAGCA-3' (SEQ ID NO: 188), was used that is complementary to the most prevalent N7-series human IgG₁ aptamer sequence, and differs by only a few bases from most other IgG₁ aptamers. This PCR primer is the same length as the selected sequence of the major IgG₁ so that it can tolerate mismatches and hybridize to similar sequences.

The ML-124 3'-primer: ML-34; 5'-CGCAGGATCCTAATACGACTCACTATA-3' (SEQ ID NO: 189), was used with a 5'-primer containing the T7-promoter sequence present in all cloned aptamers to amplify 40N7 series nucleic acids pools: random, 1a, 2a, 3a and 4a (data not shown). Since IgG₁ aptamers have not been isolated from an N8 type library, this analysis was not done for the 30N8 SELEX. PCR of random and c-met SELEX round 1a pools yielded no signal after 20 cycles. However, rounds 2a, 3a, and 4a had steadily increasing signals that were easily detectable after 10 PCR cycles. Thus IgG₁ aptamers appeared relatively early in the 40N7 SELEX experiment. For a negative control, PCR was done with a nucleic acid pool from a SELEX known to lack IgG₁ aptamers. For positive controls, PCR was done with pools from either an N7-based IgG₁ or CTLA4-IgG₁ SELEX. IgG₁ aptamers were first isolated from both of these SELEXes. The negative control had no detectable IgG₁ aptamers after 20 PCR cycles. The positive controls had detectable signals after 10 PCR cycles.

C-met aptamers. The sequences of 19 clones from round 7c-40N7 fall into five families with two sequences each, a group with three unrelated members, and six sequences closely related to known IgG₁ aptamer sequences (Table 9). Thus, at least 6 of the 19 clones (32 %) are human IgG₁ aptamers. This confirms the results of previous analysis that indicated the presence of IgG₁ aptamers in this SELEX experiment.

Of the 13 clones sequenced from round 7b-30N8, six are almost identical, another five are closely related, and two are distinct (Table 10).

Nine clones were tested for binding to c-met or KDR, six from the 40N7 series and three from the 30N8 series. These clones were chosen for the following reasons. Clone 7b-4 is the most frequent clone in Family 1 and is representative of almost all of the sequences isolated from the 7b-30N8 library. Clones 7b-10 and 7b-12 are the two clones from the 7b-30N8 library that had different sequences. From the 7c-40N7 pool, the chosen representatives were: Family 1 (clone 7c-1); Family 2 (clone 7c-4); Family 3 (clone 7c-23); Family 4 (clone 7c-26); Family 5 (clone 7c-25); and the presumed IgG1 Family (clone 7c-3).

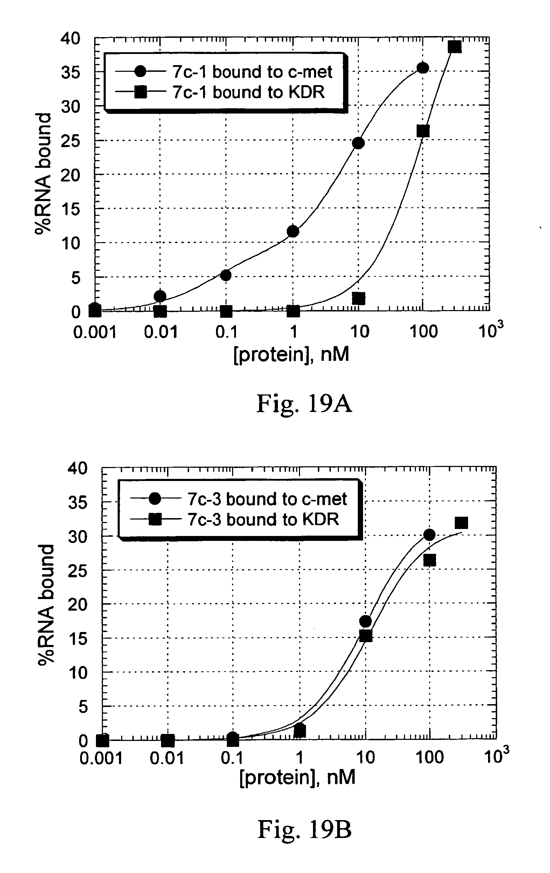

Results are shown for only two clones, including 7c-1, which was the only one observed to bind to c-met better than KDR (Figure 19A). Clone 7c-1, which appeared twice in the 40N7 series, may exhibit biphasic binding behavior with a high affinity binding K*_{d}* of ~50 pM and a lower affinity binding K*_{d}* of ~5 nM. All eight other clones bound to KDR as well as to c-met, including 7c-3, which is shown here as representative example (Figure 19B). Clone 7c-3 and all others besides 7c-1 are presumed to be IgG₁ aptamers.

In summary, two clones (identical to 7c-1) out of 32 apparently bind c-met specifically and with high affinity. The remaining clones appear to be IgG₁ aptamers.

### Example 4. Isolation of integrins and integrin ligands

αᵥβ₃ integrin was isolated from human placenta and purified by immunoaffinity chromatography essentially as described by (Smith and Cheresh (1988) J. Biol. Chem. 263:18726-31). In brief, human placentas were diced and the tissue fragments were extracted in a buffer containing 100 mM octyl-β-D-glucopyranoside detergent (Calbiochem, San Diego, CA). The extract was cleared by centrifugation and applied to an immunoaffinity column (αᵥβ₃-specific monoclonal antibody LM609 affixed to Affi-Gel 10, (Chemicon International, Inc., Temecula, CA)). Protein bound to the column was eluted with a low-pH buffer and fractions were immediately neutralized and analyzed for integrin content by SDS-polyacrylamide gel electrophoresis. Integrin-containing fractions were pooled and aliquots of the purified material were stored at -80°C. Purified human αᵥβ₃ was also purchased from Chemicon International, Inc, as was human αᵥβ₅ integrin. α_{IIb}β and fibrinogen were purchased from Enzyme Research Laboratories, Inc. (South Bend, IN). Vitronectin was purified from outdated human plasma according to the procedure of (Yatohgo et al. (1988) Cell Struct. Func. 13:281-92), using heparin affinity chromatography.

### Example 5. Generation of nucleic acid ligands to integrins

A DNA template library of sequence: was prepared by chemical synthesis. The italicized nucleotides correspond to a T7 RNA polymerase promoter. There are 40N residues (a, g, t or c). A short DNA primer "3N8": 5'- gcctgttgtgagcctcctgtcgaa-3' (SEQ ID NO:191)
was annealed to the template and extended using Klenow DNA polymerase (New England Biolabs, Beverly, MA). The double-stranded DNA product served as a product for T7 RNA polymerase transcription (enzyme obtained from Enzyco, Inc., Denver, CO) to generate a library of random-sequence RNAs. 2'-fluoro-CTP and -UTP were used in place of the 2'-OH-pyrimidines.

For application of the SELEX process to αᵥβ₃ integrin, the purified protein was diluted 1000-fold from detergent-containing storage buffer into 50 mM MES (2-[N-morpholino]ethanesulfonic acid), pH 6.1, 150 mM NaCl, 2 mM CaCl₂, to a final concentration of approximately 0.2 µg/mL. 3.2 µ polystyrene particles (IDEXX Laboratories, Inc., Westbrook, ME) were added to the diluted protein and the mixture was rotated overnight at 4°C. The beads were collected by centrifugation and blocked by incubation in 3% BSA in MES buffer (above) for one hour at room temperature. Blocked beads were washed several times by resuspension in binding buffer (50 mM Tris-HCl, pH 7.4 (at 37°C), 145 mM NaCl, 4 mM KCl, 1 mM MgCl₂, 2 mM CaCl₂, 0.1 mM MnCl₂, 0.01% BSA). For one round of selection, integrin-coated beads were mixed with RNA and rotated at 37°C for 4 hours to allow equilibration of the RNA with the immobilized protein. The beads were then collected by centrifugation and washed at least 5 times in binding buffer by rapid resuspension and pelleting, without additional incubation. RNAs that remained bound to the beads were eluted overnight at 37°C in binding buffer plus 100 µM cyclic RGD peptide ("cRGD") (GPenGRGDSPCA, Life Technologies, Gibco BRL, Gaithersburg, MD). Eluted RNAs were extracted with phenol, then chloroform:isoamyl alcohol (24:1), and ethanol precipitated. The RNA pellet was resuspended and annealed to primer 3N8 for reverse transcription using avian myeloblastosis virus reverse transcriptase (Life Sciences, Inc., St. Petersburg, FL). The cDNA pool was amplified by the polymerase chain reaction using the 3N8 primer and primer "5N8":
5'-taatacgactcactatagggagacaagaataaacgctcaa-3' (SEQ ID NO: 192)
and T. aquaticus DNA polymerase (Perkin Elmer-Cetus, Foster City, CA). Transcription of the PCR product generated an RNA pool to initiate a new round of selection. For the first round of selection 1 nmol of RNA (approximately 6 x 10¹⁴ sequences) was incubated at 2 µM concentration with a volume of bead suspension equivalent to 50 pmol of protein (assuming all the integrin had adsorbed to the beads). In subsequent rounds, the concentration of RNA and protein-coated beads were both reduced to demand higher affinity binding interactions.

The affinity of individual RNAs and RNA pools for αᵥβ₃ was determined by titration of biotinylated RNA with a small quantity of immobilized integrin. Bound RNA was detected through the biotin moiety. Biotinylated RNA was prepared according to standard transcription protocols, but including a 5-fold molar excess of a 5'-biotin-modified GMP over GTP in the reaction mixture. Methods for synthesizing 5'-biotin-modified guanosine nucleotides are described in WO 98/30720 entitled "Bioconjugation of Oligonucleotides," specifically incorporated herein by reference in its entirety. The modified nucleotide is incorporated at the 5' end of the transcript in proportion to its representation in the guanosine pool. 96-well microtiter plates (Immulon 2, Dynatech Laboratories, Inc., Chantilly, VA) were coated overnight at 4°C with 100 µL purified αᵥβ₃ at a concentration of 0.25 µg/mL in 20 mM Tris-HCl, pH 7.5, 150 mM NaCl, 1 mM MgCl₂, 2 mM CaCl₂, 0.1 mM MnCl₂. Coating concentrations were 0.8 µg/mL for α_{IIb}β₃ and 0.3 µg/mL for αᵥβ₃. Wells were blocked with 200 µL of a solution of 3% BSA in the same buffer (1 hour at room temperature) then rinsed 3 times with 200 µL binding buffer (50 mM Tris-HCl, pH 7.5, 137 mM NaCl, 2.7 mM KCl, 1 mM MgCl₂, 2 mM CaCl₂, 0.1 mM MnCl₂, 0.1% BSA). Individual RNAs or RNA pools were denatured briefly at 93°C in binding buffer without divalent cations or BSA, then serially diluted in the same buffer. 50 µL binding buffer containing 2X-concentrations of divalent cations and BSA were added to each well, followed by 50 µL RNA dilution.

RNAs were allowed to incubate in the integrin-coated wells at 37°C for 30-60 minutes. Unbound RNAs were removed by 3 rapid washes in binding buffer. To detect bound RNA, 100 µL of a 1:2500 dilution in binding buffer of streptavidin-alkaline phosphatase conjugate (Calbiochem) were incubated in each well for 30 minutes at room temperature, followed by three rapid washes, as above. 100 µL/well p-nitrophenyl phosphate (Sigma Chemical Co., St. Louis, MO) was added and incubated at room temperature for 30 minutes. Color development was monitored by absorbance at 405 nm. Binding data were fit to an equation that describes the fraction of RNA or protein bound as a function of K_{D}, and the total concentrations of RNA and protein in the binding reaction for both monophasic and biphasic binding behavior (Green et al. (1996) Biochem. 35:14413-24). A control RNA corresponding to a sequence-scrambled version of aptamer 7.24: was used to monitor non-specific binding of RNA under the conditions of the assay.

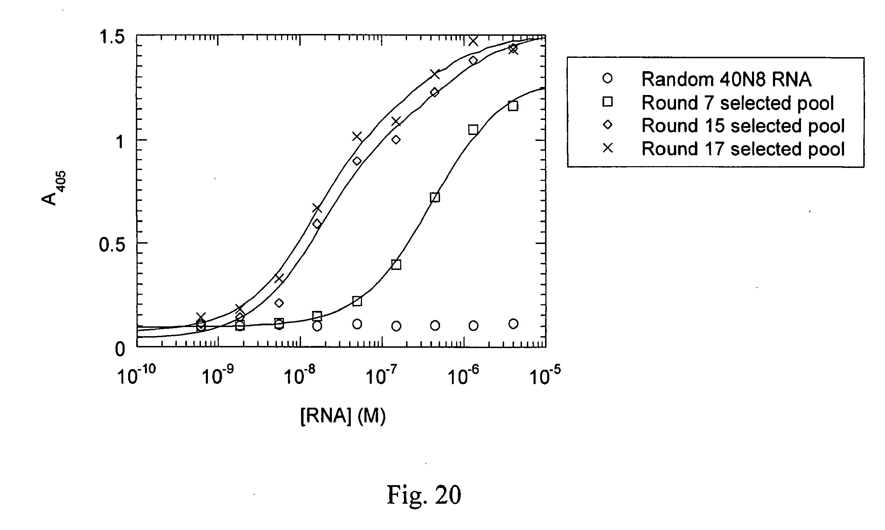

After seven rounds of the SELEX process, the amount of RNA specifically bound to the integrin-coated beads had increased substantially (data not shown). Although immobilized αᵥβ₃ showed no detectable affinity for random sequence RNA, the round 7 RNA pool bound with an equilibrium dissociation constant (K_{D}) of approximately 4 x 10-⁷ M (Figure 20). The round 7 affinity-enriched pool was cloned and sequences were determined for individual molecules in the mixture. Of 92 sequences obtained, 35 (38%) were very highly related to one another, in many cases differing at no more than a single base position. These sequences are collectively referred to as "Family 1." It is likely that many if not most of these RNAs derived from a single precursor as a result of errors in replication during the RT and PCR steps. Another 25 sequences (27%) shared a short motif (CCUGCC) that defined a second sequence family ("Family 2"). The remaining 32 sequences (35%) were not obviously related to sequences in Families 1 or 2 and were thus termed "orphan" sequences. The large percentage of orphan sequences in the round 7 pool suggested that a great deal of sequence complexity remained in the population. Therefore, the SELEX process was continued in the hope of further enriching for high affinity sequences whose representation in the round 7 pool may have been low. Indeed, a substantial improvement in the affinity of the RNA pool was observed after 8 additional rounds of affinity selection (round 15, Figure 20). No further improvement was seen after two more rounds of selection (round 17, Figure 20), so clones were isolated from the round 15 and round 17 RNA pools and the sequences of individual isolates were compared to those obtained at round 7. Twenty-seven of 39 sequences derived from the round 15 pool (69%) were members of the highly conserved sequence family, Family 1. Three sequences (8%) could be grouped with Family 2 and 9 sequences (23%) were orphans. All of the 18 sequences isolated from the round 17 pool were members of sequence Family 1. Thus, in this case, additional rounds of the SELEX process served to focus the RNA population on a single high-affinity sequence family that was already predominate at round 7.

Table 11 shows the sequences of the major family of 2'-F-pyrimidine RNAs with high affinity for αᵥβ₃ (Family 1). Clone names indicate the selected RNA pool from which each sequence was derived (round 7, round 15 or round 17) followed by a unique clone number. Note that in several cases identical sequences were isolated from different RNA pools; in these cases, both clone names are given. (Clones 17.12A and B were isolated as end-to-end inserts in a single plasmid.) Numbers in parentheses indicate the frequency with which a particular sequence was isolated; if no number is given the clone was obtained only once from the selected RNA pool. Sequences of the 5' and 3' fixed sequence regions common to all of the clones are shown at the top in lower case letters. Gaps have been inserted into many of the sequences to highlight the strong sequence conservation among most of the clones. The length of the random sequence region is shown for each RNA, as well as an estimate of the K_{D} for binding to immobilized αᵥβ₃, where it was determined (ND = not determined). The K_{D} value provided is generally based on one or the average of two determinations. Family 2 sequences isolated from the αᵥβ₃ SELEX are shown in Table 12. The short motif (CCUGCC) held in common among all the sequences is indicated in boldface letters. In Table 13, sequences with no obvious relationship to Families 1 or 2 are shown. Groups of similar sequences with only two (7.41 (SEQ ID NO:279) and 7.93 (SEQ ID NO:291)) or three (7.11 (SEQ ID NO:276), 7.82 (SEQ ID NO:288) and 7.101 (SEQ ID NO:294)) members are also included in Table 13.

The substantial affinity improvement between rounds 7 and 15 must be due in part to the loss of lower affinity species from the population; however, the introduction of and selection for higher affinity sequence variants of Family 1 may also have contributed to the overall affinity enrichment of the pool. While the affinity of relatively few sequences from the round 7 pool were measured, their affinities for immobilized αᵥβ₃ were generally less than that of RNAs derived from rounds 15 and 17 (Tables 11-13).

### Example 6. Specificity of the nucleic acid ligands to integrins

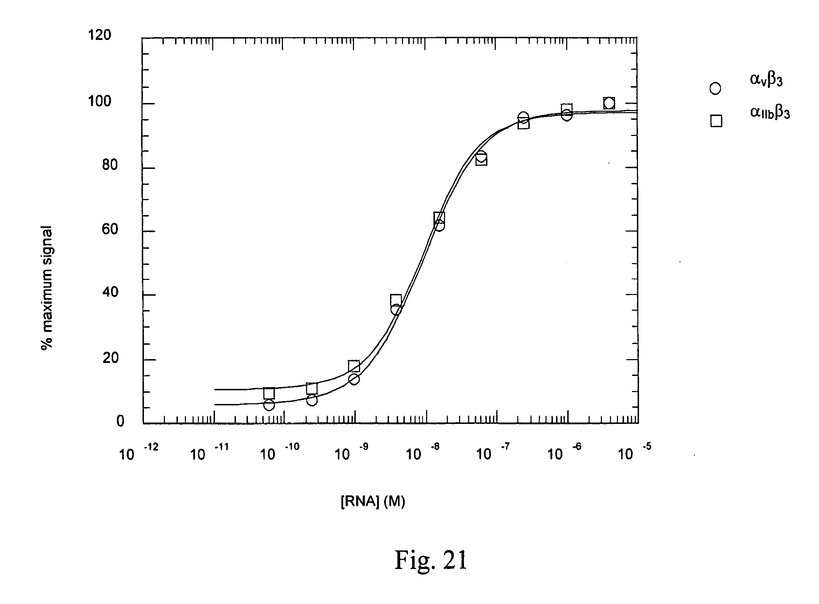

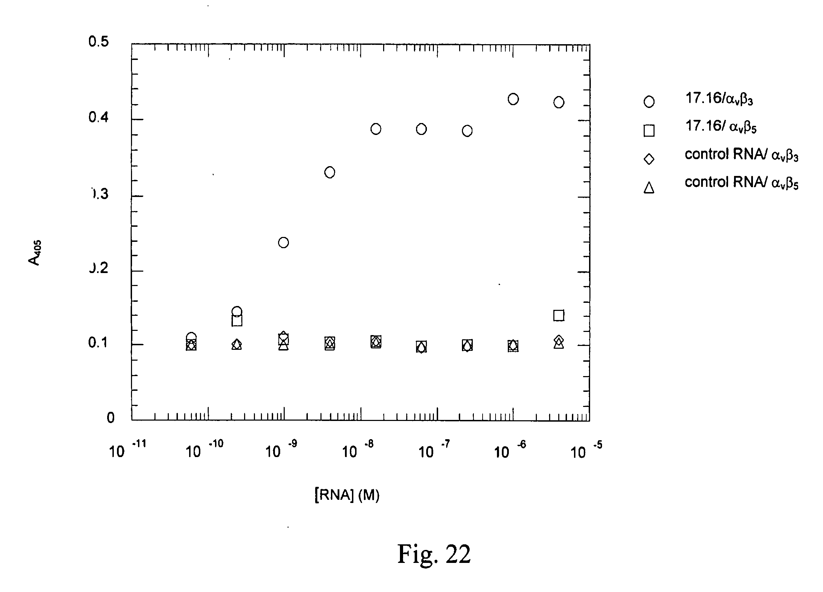

In general, aptamers selected for high-affinity binding to a particular target protein show relatively weak binding to other related proteins, except in cases where the degree of homology is very high (for example, see (Green et al. (1996) Biochem. 35:14413-24; Ruckman et al. (1998) J. Biol. Chem. 273:20556-67)). Significant homology exists within the families of integrin alpha and beta sub-units, and both alpha and beta sub-units are shared among members of the integrin superfamily. Thus, it was of interest to assess the relative affinity of the αᵥβ₃ aptamers for closely related integrins. The affinities were determined using the methods described above. The Family 1 aptamer 17.16 (SEQ ID NO:249), was chosen as a representative of the major sequence family. Figure 21 shows that aptamer 17.16 bound with identical affinity to purified, immobilized αᵥβ₃ and to the platelet integrin, αᵥβ₃ in a 96-well plate binding assay. Although these two proteins share the β₃ sub-unit in common, an alignment of the αᵥ and α_{IIB} amino acid sequences shows only 36% overall sequence identity (Fitzgerald et al. (1987) Biochem. 26:8158-65). Short stretches of exact sequence identity, 5 to 9 amino acids in length, do occur, primarily within four putative calcium-binding domains of each α sub-unit. Binding of aptamer 17.16 to integrin αᵥβ₅ was also tested. The β₅ sub-unit shares 56% sequence identity with β₃ and is more closely related to β₃ than other members of the beta sub-unit family (McLean et al. (1990) J. Biol. Chem. 265:17126-31; Suzuki et al. (1990) Proc. Nat. Acad. Sci. 87:5354-8). No aptamer binding to immobilized integrin αᵥβ₅ was observed (Figure 22), although an αᵥ-specific antibody detected the presence of αᵥβ₅ protein adsorbed to the surface of the well (data not shown). Together, these data strongly suggest that aptamer 17.16, and by extension the other members of sequence Family 1, bind primarily to the β₃ sub-unit of αᵥβ₃. Furthermore, the high-affinity binding of the aptamer to the platelet integrin, α_{IIb}β₃ extends its range of potential application to indications involving detection of platelets or inhibition of their function.

### Example 7. Aptamer inhibition of ligand binding to purified integrins

While the SELEX process identifies RNA sequences with high affinity for a particular target, the procedure used in this example was designed to bias for the recovery of ligand binding site inhibitors by the inclusion of a cRGD peptide competitor in the elution buffer. To test whether aptamer 17.16 (SEQ ID NO:249) could block the ligand binding site of αᵥβ₃ or α_{IIb}β₃, purified vitronectin and fibrinogen were biotinylated and incubated with one or both of the immobilized integrins in the presence or absence of varying concentrations of the aptamer or a non-binding control RNA. This was done as follows: purified integrin ligands, vitronectin and fibrinogen, were biotinylated according to (Smith et al. (1990) J. Biol. Chem. 265:12267-71). Briefly, proteins were dialyzed into 0.1 M NaHCO₃ and 0.1 M NaCl. N-hydroxysuccinimido-LC-biotin (Pierce) was dissolved at 1 mg/mL in DMSO and added to the protein at a ratio of 0.1 mg biotin per 1 mg protein. The reaction was allowed to rotate at room temperature for 2 hours. Biotinylated proteins were dialyzed into phosphate-buffered saline and their concentrations determined by absorbance at 280 nm. 96-well microtiter plates were coated as described above with either αᵥβ₃ or α_{IIb}β₃ and blocked with BSA. A fixed concentration of biotinylated ligand (fibrinogen: 6 nM final; vitronectin: 10 nM final) was pre-mixed in binding buffer (see "Measurement of Aptamer Binding Affinities," above) with varying concentrations of aptamer, control RNA, cyclic RGD peptide, antibody, or unmodified ligand. The mixtures were incubated in the integrin-coated wells for 60 minutes at room temperature. After washing, bound biotinylated ligand was detected by addition of 100 µL /well 1:500 dilution streptavidin-alkaline phosphatase conjugate (Calbiochem) (30 minutes at room temperature) followed by 100 µL /well p-nitrophenyl phosphate, as described above. Absorbance was read at 405 nm. The data were fit to an equation that describes mutually exclusive binding of two ligands to a single target species (Gill et al. (1991) J. Mol. Biol. 220:307-24). The concentration of competitor that inhibited 50% of the maximum signal above background (IC₅₀) was determined from the fitted curve.

Known ligand binding inhibitors, including an RGD peptide and the αᵥβ₃-specific antibody LM609, were included as positive controls for the assay. Figure 23A shows inhibition of biotinylated vitronectin binding to immobilized αᵥβ₃. Aptamer 17.16 inhibited the binding interaction with an IC₅₀ of 4.7 nM while the control RNA showed no inhibition. By comparison, the IC₅₀ of RGD peptide inhibition was 1.4 nM and that of LM609 was 2.7 nM. Unmodified vitronectin inhibited the binding of the biotinylated material with an IC₅₀ of 59 nM. Similar data were obtained for aptamer inhibition of fibrinogen binding to αᵥβ₃ (Figure 23B) and for fibrinogen binding to α_{IIb}β₃ (Figure 23C). IC₅₀ values for the data in Figure 23B were: 17.16, 9.5 nM; control RNA, not measurable; RGD peptide, 1.0 nM; LM609, 6.3 nM; unmodified fibrinogen, 43 nM. IC₅₀ values for Figure 23C were: 17.16, 6.5 nM; control RNA, not measurable; RGD peptide, 21 nM; unmodified fibrinogen, 15 nM. Thus, aptamer 17.16 is an effective competitor of β₃ integrin ligand binding and, on a molar basis, has an inhibitory potency nearly equivalent to that of a bivalent antibody.

### Example 8. Nucleic acid ligand binding to human platelets

Aptamer 17.16 (SEQ ID NO:249) was selected for binding to purified human αᵥβ₃ adsorbed to the surface of a polystyrene bead. *In vitro* assays to measure the affinity of the aptamer for purified β₃ integrins were also done in the context of hydrophobically-adsorbed protein. Thus, an important test of aptamer function was to determine its capacity to bind to native protein on the surface of cells. Human platelets were chosen for this purpose because of their ease of isolation and their high level of expression of integrin α_{IIb}β₃. Because α_{IIb}β₃ undergoes a conformational change upon platelet activation, binding of the aptamer to both resting and thrombin-activated platelets was tested. This was done as follow: fluorescein-conjugated RNA was prepared according to (Davis et al. (1998) Nuc. Acids Res. 26:3915-24). Briefly, RNA was transcribed in the presence of a 5-fold molar excess of the initiator nucleotide guanosine-5'-O-(2-thiodiphosphate) (Calciochem), followed by conjugation of the gel-purified RNA to 5-iodoacetamidofluorescein (Pierce, Rockford, IL). Platelet-rich plasma was prepared from freshly-drawn citrated human blood by centrifugation at 1000 rpm for 15 minutes in a table top centrifuge. For activated platelets, cells were incubated for 15 minutes at room temperature at 2 x 10⁷/mL in calcium- and magnesium-free Dulbecco's PBS with 2.5 U/mL thrombin and 5 mM Gly-Pro-Arg-Pro (GPRP) to inhibit platelet aggregation. Cells were diluted 1:10 into binding buffer (20 mM HEPES, pH 7.5, 111 mM NaCl, 5 mM KCl, 1 mM MgCl₂, 1 mM CaCl₂, 0.1 % BSA, 0.01 % NaN₃). Resting cells were diluted similarly, without exposure to thrombin or GPRP. The activation state of resting and thrombin-treated cells was monitored by staining with fluorophore-conjugated antibodies to CD61 (β₃ integrin subunit), which binds to all platelets, and to CD62 (P-selectin), a marker of platelet activation. Antibodies were obtained from Becton-Dickinson Immunocytometry Systems, San Jose, CA. Fluorescein-conjugated RNAs were diluted in water to 4 µM and denatured briefly at 93°C, then diluted to 2 µM with 2X-concentrated binding buffer. RNAs were then serially diluted in binding buffer. Each dilution was mixed 1:1 with resting or activated platelets and allowed to incubate in the dark at room temperature for 30 minutes. The incubation mixtures were applied directly to a Becton Dickinson FACSCalibur flow cytometer to determine the mean fluorescence intensity of the sample. Under such equilibrium binding conditions, an estimate of the K_{D} for aptamer binding to the cell surface integrin could be obtained.

Non-specific RNA binding to platelets was measured using a control RNA of similar length and base composition to aptamer 17.16. Non-specific binding became significant at concentrations above approximately 100 nM. Specific binding of the aptamer was distinguished from non-specific binding by the addition of 5 mM EDTA to the sample: EDTA had no effect on the binding of the control RNA but reduced aptamer binding to the level of the control. Specific binding of the aptamer was thus defined as the difference between the fluorescence intensity of the sample before the addition of EDTA (specific + non-specific) and the fluorescence intensity after the addition of EDTA (non-specific only).

Figure 24 shows representative data for the EDTA-sensitive component of aptamer binding to both resting and thrombin-activated human platelets. The maximum binding signal is approximately 2-fold higher to activated platelets, consistent with the slightly higher level of α_{IIb}β₃ on such cells (Wagner et al. (1996) Blood 88:907-14). However, the estimated K_{D} for aptamer binding to platelets was approximately 10 nM for both cell populations, equivalent to the value determined for binding *in vitro* to purified α_{IIb}β₃. Furthermore, aptamer 17.16 binds to both resting and activated platelets with an affinity equivalent to that reported for Reopro (abciximab, chimeric 7E3 Fab), an approved α_{IIb}β₃ antagonist (Mousa et al. (1998) J. Pharm. Exp. Ther. 286:1277-84).

### Example 9. Nucleic acid ligand biodistribution in rabbit venous clot model

To explore the application of a β₃-specific aptamer in clot imaging, aptamer 17.16 was labeled at the 5' end with technitium-99m (^{99m}Tc) and its biodistribution was monitored in a rabbit model of venous thrombosis. In this model, a clot is generated *in situ* in the isolated jugular vein of a rabbit from human platelet-rich plasma. Blood flow across the clot is re-established and the radiolabeled aptamer (or a non-binding control RNA) are introduced into the bloodstream via the ipsilateral ear vein. The distribution of the radiolabel into various tissues is reported as the per cent of the injected dose per gram of tissue.

The experiment was performed as follows: Aptamer 17.16 and a control RNA of similar length and base composition were transcribed using a 5-fold molar excess of 5'-(O-hexylamino) guanosine monophosphate. Each RNA was conjugated to Hi₁₅ at 50 mg/mL aptamer in 30% dimethylformamide with 5 molar equivalents of Hi₁₅-NHS buffered in 100 mM NaBorate pH 9.3, for 30 minutes at room temperature. The conjugation reactions were washed over a 30,000 molecular weight cut-off filter (Microcon 30, Amicon, Inc., Beverly, MA) to remove excess Hi₁₅ cage. The RNAs were then labeled with ^{99m}Tc in the following manner: to 1 nmol Hi₁₅₋aptamer was added 200 µL of 100 mM sodium phosphate buffer, pH 8.5, 23 mg/mL NaTartrate, and 50 µL [^{99m}Tc] pertechnetate (5.0 mCi) eluted from a ⁹⁹Mo column (Syncor, Denver) within 12 hours prior to use. The labeling reaction was initiated by the addition of 10 µL 5 mg/mL SnCl₂. The reaction mixture was incubated for 15 minutes at 90° C. Unreacted ^{99m}Tc was removed by spin dialysis through a 30,000 molecular weight cut-off membrane (Centrex, Schleicher & Schuell) with two 300 µL washes. This labeling protocol results in 30-50% of the added ^{99m}Tc being incorporated with a specific activity of 2-3 mCi/nmol RNA.

For biodistribution studies, rabbits were anesthetized with isofluorane. A two centimeter section of the right jugular vein was isolated *in situ* and all the branches were ligated. A catheter was inserted into the facial vein. The isolated vein segment was temporarily ligated above and below the catheter. The vein segment was flushed with saline. 1000 USP units of heparin was administered intravenously. 300-400 µL of fresh human platelet-rich plasma (citrate) activated with calcium and thrombin was instilled into the isolated vein segment and allowed to clot. After 30 minutes the ligatures were removed and blood flow over the thrombus was re-established (confirmed by the injection of 200 µL of air into the ipsilateral ear vein). [^{99m}Tc]-conjugated aptamer or control RNA was injected into the ipsilateral ear vein. At 1 hour the rabbit was exsanguinated and tissues were weighed and counted in a Wallac 1470 gamma counter. The aptamer and control RNA were tested at 1 nmol/kg (approximately 0.03 mg/kg).

For aptamer 17.16, radiolabel accumulated in the clot to a significant degree by one hour after injection, while similar accumulation was not observed with the control RNA (Figure 25). Blood clearance of the radiolabel was apparently rapid and mediated primarily by a renal mechanism as judged by moderate accumulation of radioactivity in the kidney for both the aptamer and control RNA. Thus, aptamers specific for α_{IIb}β₃ or for other proteins expressed at high levels on the surface of platelets or within the matrix of a clot will serve as useful agents for rapid imaging of thrombi.

**Table 1. Binding affinities of HGF SELEX 1 pools with and without competitor tRNA.**

| RNA pool | K*_{d}* (nM) | K*_{d}* (nM) w/tRNA |
|---|---|---|
| Random 30N7 | 1.6 | 550 |
| HGF SELEX 1 Rd.8 | 0.07 | 0.35 |
| HGF SELEX 1 Rd.9 | 0.09 | 0.42 |

**Table 2. HGF 30N7 aptamer sequences and binding affinities.**

| Seq. no.*^{a}* | | 30N7 random region*^{b}* | SEQ ID No: | K*_{d}* (nM) |
|---|---|---|---|---|
| 8-122 | (2,1) | CGGUGUGAACCUGUUUAUGUCCGCGUACCC | 18 | 0.097 |
| 8-108 | | CGGUGUGGACCUGUUUAUGUCCGCGUACCC | 19 | ND*^{c}* |
| 8-115 | | AGUGAUCCUAUUUAUGACAUCGCGGGCUGC | 20 | ND |
| 8-125 | | UGUGAACCUGUUUAUGUCAUCUUUUGUCGU | 21 | 0.075 |
| 8-155 | (1,1) | UGUGAACCUAUUUAUGCCAUCUCGAGUGCC | 22 | 0.093 |
| 8-162 | | CGUGAGCCUAUUUAUGUCAUCAUGUCUGUC | 23 | ND |
| 8-165 | | CGAGAGCCUAUUUAUGUCAUCAUGCCUGUG | 24 | 0.100 |
| 8-171 | | CGGGAGCCUUUUUAUGUCAUCAUGUCUGUG | 25 | 0.120 |
| 8-114 | (4,2) | CGUGAGCCUAUUUAUGUCAUCAUGUCUGUG | 26 | 0.071 |
| 8-203 | | CGCGAGCCUAUUUAUGUCAUCAUGUCUGUG | 27 | 0.140 |
| 8-215 | | CGUGAGCCUAUUUAUGUCAUCAUGUCUGGU | 28 | 0.077 |
| 8-217 | | CGUGAGCCUAUUUACGUCAUCAUGUCUGUG | 29 | ND |
| 8-222 | | UGUGAACCUAUUUAUGCCAUUAUGUCUGUG | 30 | 0.130 |
| 8-225 | | CGUGAGCCUAUUUAUGUCAUCAAGUCUGUG | 31 | ND |
| 8-102 | | CGAGUGCCUGUUUAUGUCAUCGUCCGUCGU | 12 | 0.060 |
| 11-9 | | CGUGAGCCUGUUUAUGACCUCGUCCAUGGC | 32 | 0.074 |
| 11-58 | | CGUGAGCCUAUUUAUGACAUGUCCCUCGAG | 33 | ND |
| 11-59 | | CGUGAGCCUGUAUAUGUCAUUGUUCUCCGG | 34 | 0.110 |
| 11-57 | | UGAGUACCUGUUUAUGUCACCACUUUCCCC | 35 | ND |
| 11-103 | | UGAUUACCUA UUAUGUC UCGCCCUCUC | 36 | 0.200 |
| 11-110 | | UGAUUACCUAUUUAUGUCAUGCUCCUCCCC | 37 | 0.086 |
| 11-65 | | UGAUAACCUGUUUAUGCCAUCGUGCUGGGC | 38 | 0.110 |
| 11-167 | | UGAUAACCUGUUUAUGUCAUCGUGCUGGGC | 39 | ND |
| 11-201 | | UGAGAACCUAUUUAUGUCAUCGUGUCUGGC | 40 | ND |
| 11-162 | | UGAUAACCUAUUUAUGACGUCGUGGCUCCC | 41 | ND |
| 11-202 | | UGGGAACCUAUUUAUGUCAUC UCCGUCCC | 42 | ND |
| 11-106 | | CGAUGAUGCCUGUUUAUGUCGAUGUCCCCC | 43 | 0.120 |
| 11-158 | | CGAUAGCCUAUUUAUGACCUCGUCCCCGUG | 44 | 0.170 |
| 11-112 | | CGUGAGCCUAUUUAUGACAUCGUUCUUGGC | 45 | ND |
| 11-124 | | CGUGAGCCUAUCUAUGUCAUCGUGUGUGCC | 46 | ND |
| 11-122 | | UGAGUACUAUUUAUGUCGUCGUUCGUGCC | 47 | ND |
| 11-217 | | CGUGAGCCUUCCAAUGACGUCGUCCUUGGC | 48 | 0.071 |
| 8-104 | | GCGACUCAAUCUGAAUCGUCUUGUCCCGUG | 49 | 0.050 |
| 11-76 | UCAGCGGCGCGAGCCUGUUUAUGUC UGCUG | | 50 | 0.076 |
| "consensus" | | CGUGAGCCUAUUUAUGUCAUCGU-C-UG | 51 | |
| | | | | |
| 11-8 | | UCAGUAUGACU UUUAUAGCA CGUUCGCCC | 52 | 0.150 |
| 11-153 | | ACAGGUAGUCU UCUAUAGCA CUUCCUCCCC | 53 | 0.190 |
| 11-157 | | UCAGAAUGACU UUCAUAGCA CGCUUUCCC | 54 | 0.260 |
| 11-222 | | ACAUAAGUCU UCUAUAGC UCGUCCUUUGUG | 55 | 0.077 |
| 11-223 | | UCAGUAUGGCU UCUAUAGC UCGUUCCUCGG | 56 | 0.120 |
| 8-126 | (3,1) | GUGACUCAAAAUGGUGAUCCUCG UUUCCGC | 57 | 0.099 |
| 8-101 | | GUGACUCAAAAUGGUGAUCCUCGAUUUCCGC | 58 | 0.095 |
| 8-105 | | GUGACUCAAAAUGGUGAUCCUCGAUUGCCGC | 59 | ND |
| 8-103 | | GCCGAAAAU UCGUCGACAUCUCCCUGUCUG | 60 | 0.120 |
| 8-118 | | GGCGACUUUCCUCCAAUUCUCACCUCUGCA | 61 | 0.160 |
| 8-119 | | GCCAUUCGAUCGA UUCUCCGCCGGAUCGUG | 62 | 0.110 |
| "consensus " | | CGUGAGCCUAUUUAUGUCAUCGU-C-UG | 51 | |
| | | | | |
| 8-3 | (2) | AUCCCGCGAC CAGGGCGUU UCUUCCUCGUCC | 63 | 0.130 |
| 8-112 | (3) | UCCCGAAUUUAAGUGCGUU UCCUCCGCGUC | 64 | 0.130 |
| 8-154 | (3) | UCCCAAGAUUCAGGGCGUU UCUUCCUCGUC | 65 | 0.120 |
| 8-117 | | UCCCAAGUUUCAGGGCGUU UCUUCCUCGUC | 15 | 0.130 |
| 8-123 | | UCCCGAGUUUGAGGGCGUU UCUUCUUCGUC | 66 | 0.210 |
| 11-121 | | UCCCAGUUUCAgGGGCGAU UCCUCUUCGUC | 67 | ND^{c} |
| | | | | |
| 8-17 | (7,1) | GCGGCU CGAUG UCGUCUUAUCCCUUUGCCC | 68 | 0.095 |
| 8-16 | | GCGGGCU CGAUG UCGUCUUAUCCCCUUUGCCCC | 69 | ND |
| 8-158 | | CCGGCU CGAUG UCGUCUUA CCCCUUUGCCC | 70 | 0.310 |
| 11-104 | | GUUUGAG UGAUG UCGUCUUGUCCCGCCUGC | 71 | 0.091 |
| 11-111 | | GUUAGAG UUUUG UCGUCUUGUCCCAUGUG | 72 | ND |
| 11-163 | | GCUUGAGUC UUUG AUCGUCUUAUCCCUCGU | 73 | 0.082 |
| 11-208 | | GUUUGAG UGACG AUCGUCUUGUCCCAUGUG | 74 | 0.060 |
| 11-212 | | GUUUGAG UUAAA CAUCGGUUUUCUCCUG | 75 | 0.075 |
| 11-6 | | GACGCG UUGAUU CAUCGUCUUAUCCUGCUG | 76 | 0.240 |
| 11-126 | | GUUUGGGUCU UGAUC UCGUCUUGUCCCGUG | 77 | 0.170 |
| 11-165 | | gUUGAUAGG AGUCAU CAUCGUCUUGUCCGC | 78 | 0.073 |
| 11-215 | | GUAGUGAG UUUUCAUU GUCUUGUCCCCGUG | 79 | 0.091 |
| 11-151 | | UGAGUCAUAGUGUUG AUCGUCGUAUCCCGU | 80 | 0.170 |
| 11-7 | | GUGGAGUCAA AUCGUCUUGUCCCUUGUCCU | 81 | 0.110 |
| 11-166 | | GUUUGAG UUCUGACA CGUCUUGUCCCAUGC | 82 | 0.079 |
| 11-17 | | GUUAGAGC GUGACAG UCGUCUUAUCCCGGGUCA | 83 | 0.130 |
| | | | | |
| 8-113 | (2) | UGAAUUCCUCUGGCUGAAAAU GACUUGUGC | 84 | 0.083 |
| 8-60 | | UGAAUUCCUUUGGCUGAAAAU GACUUGUGC | 85 | ND |
| 11-221 | | GCAGAGCGAAAAUCGUCUUGUCCCCGACGC | 86 | 0.062 |
| | | | | |
| ORPHANS | | | | |
| 11-123 | | GUGACUCAAAAUGGUGAUCCUCGUUUCGC | 87 | 0.090 |
| 8-151 | | AGGACUAAUCCCUAAGGAAUAGCUUGCCCG | 88 | 8 |
| 8-174 | | UCGAGCUUCUGAGUUAAA CUGGGGCCUCCU | 89 | 0.230 |
| 8-160 | | GUCCCCGAAUUUAAAGUGCGUUUUCCUCCGGG | 90 | 0.150 |
| 11-203 | | GGUUUUUCUUUUCWGUUCUCUUCUUUCCCC | 91 | 0.260 |
| 11-224 | | ACAGCGGCGACUAGCCUGUUCAUGCCUGCC | 92 | 0.110 |
| 11-107 | | GUUCUGUGUGUCCACGUUCUUACCCCUGUG | 93 | 0.140 |

| | | | | |
|---|---|---|---|---|
| *^{a}*Clone series 8 is from HGF SELEX 1; series 11 is from HGF SELEX 3. Numbers in parentheses refer to repeat occurrences of the same exact sequence. For the series 8 clones, a second number refers to an exact match which was isolated in series 11. *^{b}*N7 fixed sequences are not shown. (5'-GGGAGGACGAUGCGG-N-CAGACGACUCGCCCGA-3') *^{c}*ND, not determined. | | | | |

**Table 3. HGF 30N8 aptamer sequences and binding affinities.**

| Seq. no*^{a}* | | 30N8 random region*^{b}* | SEQ ID NO: | K*_{d}* (nM) |
|---|---|---|---|---|
| 10-28 | | CCUGUUCUGAAC GCAAAAUGGCGUGGUGGC | 94 | 0.860 |
| 10-40 | | UGUCGUUAGUUUAUUGACAAGGCCCGAAG | 95 | 0.350 |
| | | | | |
| 10-52 | | UCUUAUUGUGUCCAGCUUCUCCCUGCAGGC | 96 | 0.160 |
| 10-72 | | UGUGGCAC UGUUGUCCACAAGGGCCUCA | 97 | 0.450 |
| 10-8 | | UUGACAAGGUACCUGUUGCCUGGCGUUUCU | 98 | 0.920 |
| | | | | |
| 10-76 | | AGUUAGGCUUUAAAGC ACG AUAAUCAGCA | 99 | 0.170 |
| 10-47 | | GUCAAGAGG AAAUGACACGG CUCCACUUUUA | 100 | 0.390 |
| 10-2 | (10) | GCCUGAGUUAAACAUGACGG UUUGUGACCC | 101 | 0.069 |
| 10-3 | | GCCUGAGUUAAACAUGACGGGUUUUGUGACCCCU | 102 | 0.072 |
| 10-23 | (4) | GUCUGAGUUGGACACAACGC AUUGAGACCC | 103 | 0.330 |
| 10-24 | | GUCUGAGUUgGUCACAACGC AUUGAGACCC | 104 | ND^{c} |
| 10-37 | | GUCUGAGUCCGU AGGGCGA UUUGUGUCCC | 105 | 3.05 |
| 10-7 | | UGCCUUAAGAGCGGAA CUCCCUGACCCACC | 106 | 1.45 |
| 10-13 | | GAUCUGUUGGCGU GU CUACCCGACCCUCCU | 107 | 0.720 |
| 10-17 | | AACCCUGUUGGCGU GA CGUCCCGACCCUCC | 108 | 0.560 |
| 10-36 | | CGUUAGCAUCUGAACGAUGCCCAGCCUCAA | 109 | 1.94 |
| 10-62 | | GUUAGACUCAACAUGAGUCCCAGCCUCAA | 110 | 0.440. |
| 10-29 | | UCUGUUGGCGUCGU UCUCCUGACCCUCCUC | 111 | 1.75 |
| 10-48 | | GAGUUCCCUGUUGAC UCGC UCUCCUGACCC | 112 | 0.310 |
| 10-16 | | UACAGCGUGUUGGUCCCGGACGGGGACUUAU | 113 | 0.210 |
| | | | | |
| 10-11 | | CGCCUGGACCGUUUGUUUAUCCCCGUAGUC | 114 | 0.610 |
| 10-18 | | CGUGAUUCCUACCAUCA GGUACCUAUCUUG | 115 | 0.300 |
| 10-1 | (2) | AGUGAUGUGAGAG CGUGCCUCUAGUCGGUG | 116 | 0.094 |
| 10-57 | | CGAGCCUCCUACCGUUU AGGUACC AUCUUG | 117 | 0.140 |
| 10-27 | | UUAGCCUCCGACCG UAA GGUCCUUUUCUUG | 118 | 0.830 |
| 10-53 | | GGCCUCCAACCGCUAAA GGUUCCAUUCUUG | 119 | 0.310 |
| 10-49 | | CCCGACCUCCUGUAACUGGUUGA GGCACUA | 120 | 0.240 |
| 10-31 | (2) | GGGUUCCUGAUUGACCCUGUCUCUAGACCC | 121 | 1.90 |
| 10-58 | | GGGGAGGCCCUUCAGCCGUCUCCUUGACCC | 122 | 0.440 |
| 10-63 | | UGUGAUGUGAGGGC GUGCUUCCUAACGGUG | 123 | 0.190 |
| | | | | |
| | | 40N8 "hitchhiker" sequences | | |
| | | | | |
| 10-19 | | UUCAUUAUGCAUCGAACAGUAUACCACAGGUGUUCAUGUG | 124 | ND |
| 10-35 | | AUCCAAAUUCUGGUCAUGAGGCGCUGCAGAUACUGCUGCG | 125 | 2.33 |
| 10-38 | | UCUGCGGACGGUGAGGUUAAGUUGCAACGACUGCUUGGCG | 126 | 7.38 |
| 10-42 | | CAGACCGUGCAAACCCCCCUUAGAGGGUUUUGUCAUUUAC | 127 | ND |
| 10-56 | | CCUUAGGGCUCCCAAAAAUCGGGCC CGUCGGGCCGAUCAC | 128 | 0.280 |
| 10-68 | | CGCGGGAUUCUCUGAGGACGAGGCACGUGUGGGUAAUUCG | 129 | 1.00 |
| 10-67 | | UCGGGCUUGGAUGUGGACGUGUAUUUCUAGCUGUGUACGC | 130 | 0.640 |
| 10-4 | | UUGGGUCGGGACUCGAAAGGAUUUGAUAGGAUACAUGAAU | 131 | 0.610 |

| | | | | |
|---|---|---|---|---|
| *^{a}*clone series 10 is from HGF SELEX 2. Numbers in parentheses refer to repeat occurrences of the same exact sequence. *^{b}*N8 fixed sequences are not shown. (5'-GGGAGAUAAGAAUAAACGCUCAA-N-UUCGACAGGAGGCUCACAACAGGC-3') *^{c}*ND, not determined. | | | | |

**Table 4. List of HGF aptamers and their binding affinities which were tested in vitro for inhibition of activity.**

| Seq. No. | random region | K*_{d}* (nM) | SEQ ID NO: |
|---|---|---|---|
| "consensus" | CGUGAGCCUAUUUAUGUCAUCGU-C-UG | | |
| 8-17 | GCGGCU CGAUG UCGU CUUAUCCCUUUGCCC | 0.095 | 68 |
| 8-102 | CGAGUGCCUGUUUAUGUCAUCGUCCGUCGU | 0.060 | 12 |
| 8-104 | GCGACUCAAUCUGAAUCGUCUUGUCCCGUG | 0.050 | 49 |
| 8-112 | UCCCGAAUUUAAGUGCGUU UCCUCCGCGUC | 0.130 | 64 |
| 8-113 | UGAAUUCCUCUGGCUGAAAAUGA CUUGUGC | 0.083 | 84 |
| 8-122 | CGGUGUGAACCUGUUUAUGUCCGCGUACCC | 0.097 | 18 |
| 8-126 | GUGACUCAAAAUGGUGAUCCUCG UUUCCGC | 0.099 | 57 |
| 11-8 | UCAGUAUGACU UUUAUAGCA CGUUCGCCC | 0.150 | 52 |
| 11-76 | UCAGCGGCGCGAGCCUGUUUAUGUC UGCUG | 0.076 | 50 |
| 11-166 | GUUUGAG UUCUGACA CGUCU UGUCCCAUGC | 0.079 | 82 |
| 11-208 | GUUUGAG UGACG AUCGUCU UGUCCCAUGUG | 0.060 | 74 |
| 11-222 | ACAUAAGUCU UCUAUAGC UCGUCCUUUGUG | 0.077 | 55 |
| 10-2^{*} | GCCUGAG UUAAACAUGACG GUUUGUGACCC | 0.069 | 101 |
| 8-151 | AGGACUAAUCCCUAAGGAAUAGCUUGCCCG | 8 | 88 |

| | | | |
|---|---|---|---|
| * 10-2 contains N8 fixed sequences; all others are N7. | | | |

**Table 5. HGF truncate SELEX 30N sequences.**

| **Trunc** **Seq #*^{a}*** | **# of hits** | **Sequence of random region** **(G)G-30N-CA** | **Identity to full-length** *^{b}* | **K*_{d}*** **(nM)** | **SEQ ID No:** |
|---|---|---|---|---|---|
| | | | | | |
| | | GGACGAUGCGGCGAGUGCCUGUUUAUGUCAUCGUCC | NX22354 | 0.1 | 13 |
| Tr7 | **(5)** | CGGUGUGAACCUGUUUAUGUCCGCGUACCC | 8-122 | 0.67 | 132 |
| Tr45 | **(3)** | UGGGAACCUAUUUAUGUCAUCUCCGUCCC | 11-202 | 1.7 | 133 |
| Tr70 | | UGGGAACCUAUUUAUGUCAUCGUCUGUGCC | New | 2.4. | 134 |
| Tr6 | | CGUGAGCCUAUUUAUGUCAUCAUGUCUGUG | 8-114 | 9.0 | 135 |
| Tr20 | | UGUGAACCUGUUUAUGCCAUCUCGAGUCCC | New | 3.4 | 136 |
| Tr23 | | UGUGAACCUAUUUAUGCCAUCUCGAGUGCC | 8-155 | ND^{c} | 137 |
| Tr42 | | UGAUAACCUAUUUAUGACGUCGUGGCUCCC | 11-162 | 6.1 | 138 |
| Tr44 | | AGUGAUCCUAUUUAUGCCGUCGCUUCUCGC | New | 6.5 | 139 |
| Tr65 | | AGAGNUCCUAUUUAUGACAUCCCAUGCCCC | New | 1.4 | 140 |
| Tr48 | | UGAUCACCUGUUUAUGCCAUCGUUCUGGGC | 11-65 | 1.8 | 141 |
| Tr28 | | GGUGACCCUUUUUAUGACAUCGCGUCUGGC | New | 4.0 | 142 |
| | | | | | |
| Tr51 | **(6)** | AAUCACAGGAAUCAACUUCUAUUCCCGCCC | New | 0.06 | 143 |
| Tr67 | | AAUCACAGGAAUCGACUUUUAUUCCUGCCC | New | ND | 144 |
| | | | | | |
| Tr17 | | GC GGCUCGAUGUCGUCUUAUCCCUUUGCCC | 8-17 | 3.0 | 145 |
| Tr27 | | UC GGCUCGUUGUCGUCUUAUCCCUUUGCCC | New | ND | 146 |
| Tr18 | | GCUGGCUCGAUGUCAGGUUAUCCCUUUGCCC | New | ND | 147 |
| | | | | | |
| Tr4 | **(4,2)*^{d}*** | GUGACUCAAAAUGGUGAUCCUCGUUUCCGC | 8-126 | 1.4 | 148 |
| Tr31 | **(2)** | UGAAUUCCUCUGGCUGAAAAUGACUUGUGC | 8-113 | 9.2 | 149 |
| Tr15 | | GUUUGAGUGACGAUCGUCUUGUCCCAUGUG | 11-208 | 8.8 | 150 |
| Tr1 | | AUUGAUUCACUGCAUCCUUGACUCUUCCCC | New | 7.3 | 151 |
| Tr5 | | CAGACGACUCGCCCGAAGGACGAUGCGG | New | 28 | 152 |
| Tr14 | | GAGUUAUAUUUCGUCACCCGUUCCUUUGCCC | New | 2.2 | 153 |
| Tr59 | | ACAGUUUGUCUUCUAUAGCUCGUCGCCCC | New | 7.2 | 154 |
| Tr71 | | UCAGAAUGACUUUCAUAGCUCGCUUUCCCC | New | 7.7 | 155 |

| | | | | | |
|---|---|---|---|---|---|
| *^{a}*Tr1-36 and Tr37-72 clones are from series which were carried through 8 and 11 conventional rounds, respectively. *^{b}*sequences indicated are identical to full length aptamers derived from series 8 or 11; NX22354 is a synthetic truncate based on boundary experiments, derived from sequence 8-102, shown here for comparative purposes. *^{c}*ND, not determined. *^{d}*(4,2) refers to 4 occurrences in the first series and two in the second series. | | | | | |

**Table 6. Invasion of A549 cells through Matrigel is inhibited by HGF aptamer NX22354.**

| Sample | HGF 10 ng/mL | Inhibitor | Cells migrated |
|---|---|---|---|
| 1 | - | - | 40 |
| 2 | + | - | 240 |
| 3 | + | mAb*^{a}*, 1 µg/mL | 120 |
| 4 | + | NX22354, 1 uM | 40 |
| 5 | + | NX22354, 0.2 uM | 25 |
| 6 | + | NX22354, 0.04 uM | 200 |

| | | | |
|---|---|---|---|
| *^{a}*Anti-HGF antibody was MAB294 from R&D Systems, Inc. | | | |

**Table 7. Partially 2'-O-methyl substituted variants of NX22354.**

| | SEQUENCE | SEQ. ID. No. |
|---|---|---|
| NX22354 (parent) | | 13 |
| HGFOMe1 | GGACGAUGCGGCGAGUGCCUGUUUAUGUCAUCGUCCg | 156 |
| HGFOMe2 | GGACGAUGCGGCGAGUGCCUGUUUAUGUCAUCGUCCg | 157 |
| HGFOMe3 | GGACGAUGCGGCGAGUGCCUGUUUAUGUCAUCGUCCg | 158 |
| HGFOMe4 | GGACGAUGCGGCGAGUGCCUGUUUAUGUCAUCGUCCg | 159 |

| | | |
|---|---|---|
| Parent 36mer sequence of NX22354 (purines marked with asterisks). The substituted positions are indicated by underlines. The OMe1 sequence has five substitutions while the others have four. For technical reasons, a G residue was added at the 3'-end of each aptamer. | | |

**Table 8. Binding and Dissociation Constants**

| SELEX Round | Protein | K*_{d}* |
|---|---|---|
| random | IgG₁ | ~1 µM |
| 7c | IgG₁ | 23 nM |
| random | c-met | 100 nM |
| 7c | c-met | 2 nM |

**Table 9. 40N7 sequences isolated from a plate SELEX on human c-met.**

| Clone name: (number of isolates). | | Sequence*^{a}* | SEQ ID NO: |
|---|---|---|---|
| FAMILY 1: | | | |
| 7C -1: | (2) | UUUGACUAUGUCUGACGGGUCUGUGGUCAAUUCCGCCCC | 160 |
| | | | |
| FAMILY 2 | | | |
| 7C -4: | (1) | AUCCGUGUUGAUGUCCAUAUAACCUUAUCCCGUCGCUCCC | 161 |
| 7C -5: | (1) | GUGUUGACUUCUAGCCAGAAUAACAUUUUGUACCCCUCCC | 162 |
| | | | |
| FAMILY 3 | | | |
| 7C -2: | (1) | UCGUUGAGCUUUUGAUAGGGCUUGUUCUUCGAGCGUCCC | 163 |
| 7C-23: | (1) | UGAUCUUGGGUUUGAUCGUAAUUACUUCACCCUCCGUCCC | 164 |
| | | | |
| FAMILY 4 | | | |
| 7C-26: | (2) | CUCCUUUUCCGCUAAACAAGACCACUUUGAGCCCUGCCCC | 165 |
| | | | |
| FAMILY 5 | | | |
| 7C-25: | (1) | CCACCUCGUUACGUACUGAUUUUGGCAUCGCAGUUUGCCC | 166 |
| 7C-27: | (1) | GGGCACCUCGAUACGUACUGAUUUUGAAUAUCAGUUAGCCCC | 167 |
| | | | |
| OTHERS | | | |
| 7C-21: | (1) | CGAUUCGUCGUAUAGAAAUGAUUUGAAUGCACCUCCUCCC | 168 |
| 7C-24: | (1) | UGUGUUUGUGUGUUGUGUUUGUUAUUCCUGUUUGUGUCCU | 169 |
| 7C-32: | (1) | UCGGUCGUAAAAAAAUCGUUGGUGUCUAUCUAUUGUUCUCCC | 170 |
| | | | |
| Presumed IgG₁ aptamers | | | |
| 7C -3: | (1) | UGCUCCAGAGGAACCAUCGUUUACUUCAUUUAUUCGCCC | 171 |
| 7C-22: | (1) | UGCUCCUUAGGAACCAUCGUCUAUAUCCCAUUCUGACUGCC | 172 |
| 7C-30: | (1) | UGCUCCUCAGGAACCAUCGUUUUUCCCAUGUCCUUCUGCC | 173 |
| 7C-29: | (3) | UGCUCCUUGGAUUACCAAGGAACCAUUUUCCUCUACCCCC | 174 |

| | | | |
|---|---|---|---|
| *^{a}*N7 fixed sequences are not shown. (5'-GGGAGGACGAUGCGG-N-CAGACGACUCGCCCGA-3') | | | |

**Table 10. 30N8 sequences isolated from a plate SELEX on human c-met.**

| Clone name: (number of isolates). | | Sequence*^{a}* | SEQ ID NO: |
|---|---|---|---|
| FAMILY 1: | | | |
| 7b-1: | (4) | GUGCUCAUUACGAACUUGACCGAUGCCUA | 175 |
| 7b-9: | (1) | GGUGCUCAUUACGAACUUGACCGAAGCCUA | 176 |
| 7b-18: | (1) | GGUGCUCAUUACGAACUUGACCGAUGCCUA | 177 |
| 7b-3: | (1) | AGUGCUCCAAUGAACUUUGCUCGCUGA | 178 |
| 7b-8: | (1) | GGUGCUCCGUUUGGAACUUGAUCGGUAGGA | 179 |
| 7b-7: | (1) | GUGCUCAUUCAGAACUUGACGUAUAACCA | 180 |
| 7b-14 | (1) | GGUGCUCCUUAGGAACWGACCGUCCGCCA | 181 |
| 7b-16: | (1) | GUGGUGCUCCACUAACCAAGUGGAACCUUG | 182 |
| consensus: | | GUGCUC-UU--GAACUUGACCG | 183 |
| | | | |
| OTHERS: | | | |
| 7b-10: | (1) | ACGAUAAGUGGGAGUGAGUAAGUUUGAGUA | 184 |
| 7b-12: | (1) | CCUAGACCCCCAGGUUCCUCCCCACUAGUC | 185 |

| | | | |
|---|---|---|---|
| *^{a}*N8 fixed sequences are not shown. (5'-GGGAGAUAAGAAUAAACGCUCAA-N-UUCGACAGGAGGCUCACAACAGGC-3') | | | |

**Table 11. αᵥβ₃ Family 1 aptamer sequences.**

| Clone name (# of isolates) | Sequence of variable region 5'-gggagacaagaauaaacgcucaa [40N] uucgacaggaggcucacaacaggc-3' | Seq. length | K_{D} (nM) | SEQ ID NO: 310 |
|---|---|---|---|---|
| 7.3 (2) | Uucuacgu uguuuaagggcuuauaugagcgcauuauaccc | 40 | 22 | 194 |
| 7.6; | Uucaacgc uguuuaagggcuuauaugagcgcguuauaccc | 40 | ND | 195 |
| 17.12A | | | | |
| 7.12 | Uucaacgc uguuuaagggcuuauaugagcgcguuacaccc | 40 | ND | 196 |
| 7.24(5) | Uucaacgc uguucaagggcuuauaugagcgcguuauaccc | 40 | 170 | 197 |
| 7.25 | Uucaacga uguuuaagggcuuauaugagcgcguuauaccc | 40 | ND | 198 |
| 7.34 | uucau gaa guccaagggcuuauaugagcgcguuauaccc | 39 | ND | 199 |
| 7.36 (3) | Uucaacgc ugucaaagggcuuauaugagcgcguuauaccc | 40 | ND | 200 |
| 7.37(2) | uu aacgu uguucaaggguuuauaugagugcguuauaccc | 39 | ND | 201 |
| 7.38(2) | Uucaacgc uguccaagggcuuauaugagcgcguuauaccc | 40 | 49 | 202 |
| 7.49 | Uucaacggauguccaagggcuu uaugagcgcguuauaccc | 40 | ND | 203 |
| 7.53 | Uucgacgc uguucaagggcuuauaugagcgcauuauaucc | 40 | ND | 204 |
| 7.54(2) | Uucgacgc uguucaagggcuuauaugagcgcguuauaccc | 40 | 230 | 205 |
| 7.57(2) | Uucgacga uguccaagggcuuauaugagcgcauuauaccc | 40 | ND | 206 |
| 7.63 | Uucaacgc uguucaagggcuuauaugagcgcguuacaccc | 40 | ND | 207 |
| 7.64 | uuc auga uguucaagggcuuauaugagcgcauuauaccc | 39 | ND | 208 |
| 7.77(2) | Uucaacga uguugaggggcuuauaugagcgcauuauaccc | 40 | 770 | 209 |
| 7.80 | Uucaacga uguccaagggcuuauaugagcgcauuauaccc | 40 | ND | 210 |
| 7.86 | Uucaacgc uguucaagggcuuaugugagcgcguuauaccc | 40 | ND | 211 |
| 7.91 | Uucaacgu uguccaagggcuuauaugagcgcauuauaccc | 40 | ND | 212 |
| 7.115 | Uucaacgc uguucaagggcuuauaugagcgcauuauaccc | 40 | ND | 213 |
| 7.121 | Uucaacga uguccaagggcuuauaugagcggauua ccc | 38 | ND | 214 |
| 7.124 | Uucaacac ugu gaagggcuuauaugagcgcgucauaccc | 39 | ND | 215 |
| 7.127 | Uucaacgu uguucaagggcuuauaugagcgcguuauaccc | 40 | ND | 216 |
| 15.2 | Uucaacgu ugucaaagggcuuauaugagcggauua ccc | 38 | 6 | 217 |
| 15.3 (3); | Uucaacgu uguccaagggcuuauaugagcggauua ccc | 38 | 8 | 218 |
| 17.17 | | | | |
| 15.7 | Uucuacga ugucaaagggcuuauaugagcggauua ccc | 38 | 5 | 219 |
| 15.8 | Uucgacgc uguugaagggcuuauacgagcggauua ccc | 38 | 5 | 220 |
| 15.10 | Uucaacgc uguucaagggcuuauaugagcggauua ccc | 38 | 20 | 221 |
| 15.14 | Uucaacau uguccaagggcuuauaugagcggauua ccc | 38 | 6 | 222 |
| 15.17 | Uucaacgu ugucaaagggcuuauacgggcggauua ccc | 38 | 4 | 223 |
| 15.18 | Uucaacgc ugug aagggcuuauaugagcggauua ccc | 37 | 2 | 224 |
| (2) | | | | |
| 15.20 | Uucaacgc uguccaagggcuuauaugagcgcauuauaccc 40 20 225 | | | |
| 15.27 | Uucgacua uguccaagggcuuauaugagcggauua ccc | 38 | ND | 226 |
| 15.28 | Uucgacga ugucuaagggcuuauaugagcggauua ccc | 38 | ND | 227 |
| 15.40; | Uucaacgc uguugaagggcuuauacgagcggauua ccc | 38 | ND | 228 |
| 17.12B | | | | |
| 15.41 | Uucaacgu uguccaagggcuuauacgagcggauua ccc | 38 | ND | 229 |
| 15.42; | Uucaacgc uguccaagggcuuauacgagcggauua ccc | 38 | ND | 230 |
| 17.14 | | | | |
| (2) | | | | |
| 15.46; | Uucgacgc ugug aagggcuuauaugagcggauua ccc | 37 | 40 | 231 |
| 17.20 | | | | |
| 15.47 | Uucaacgu ugucaaagggcuuauacgagcggauua ccc | 38 | ND | 232 |
| 15.48 | Uucaacgc uguugaagggcuuauaugagcggauua ccc | 38 | ND | 233 |
| 15.49 | Uucuacgu ugucuaagggcuuauaugagcggauua ccc | 38 | ND | 234 |
| 15.50; | Uucgacgc ugug aagggcuuauacgagcggauua ccc | 37 | 30 | 235 |
| 17.3 | | | | |
| 15.52 | Uucaacgc uguucaagggcuuauacgagcggauua ccc | 38 | ND | 236 |
| 15.53 | Uucaacgc uguccuagggcuuauaugagcgcaggauaccc | 40 | 70 | 237 |
| 15.55 | Uucuacgc uguuuaagggcuuauaugagcgaauua ccc | 38 | ND | 238 |
| 15.57 | Uucuacgu uguccaagggcuuauaugagcggauua ccc | 38 | ND | 239 |
| 15.58 | Uucgacgu uguugaagggcuuauaugagcggauua ccc | 38 | ND | 240 |
| 17.1 | Uucaacgc ugucaaagggcuuauauaagcggauua ccc | 38 | 380 | 241 |
| 17.2(2) | Uucuacgc ugug aagggcuuauaugagcggauua ccc | 37 | 2 | 242 |
| 17.5 | Uucgacgc ugug aagggcuuauaugagcggau acaccc | 38 | 5 | 243 |
| 17.7 (2) | Uucuacgc ugug aagggcuuauacgagcggauua ccc | 37 | 6 | 244 |
| 17.8 | Uucaacgu ugucuaagggcuuauaugagcggauua ccc | 38 | 18 | 245 |
| 17.10 | Uucuacgu uguugaagggcuuauaugagcggauua ccc | 38 | ND | 246 |
| 17.11 | Uucuacgc ugug aagggcuuauaugagcgaauua ccc | 37 | 4 | 247 |
| 17.13 | Uucaacgc uguccaagggcuuauaugggcggauua ccc | 38 | 10 | 248 |
| 17.16 | Uucaacgc ugug aagggcuuauacgagcggauua ccc | 37 | 8 | 249 |
| | | | | |

**Table 12. αᵥβ₃ Family 2 aptamer sequences**

| Clone name (# of isolates) | Sequence of variable region 5'-gggagacaagaauaaacgcucaa [40N] uucgacaggaggcucacaacag gc-3' | Seq. length | K_{D} (nM) | SEQ ID NO: 310 |
|---|---|---|---|---|
| | | | | |
| 7.4 | GUACCGGAUCGCCCUGCCACGGUAUUUGAGACAUUGAAA | 39 | ND | 250 |
| 7.5(3) | GGUAGUAAAUGGACUCCUGCCAUCCAAUACUAUCUCUGAG | 40 | >1000 | 251 |
| 7.13 | UGUAGUCGCAUGUCGAGCAGCAAUUCCUGCCAUUGUAGG | 39 | >1000 | 252 |
| 7.14 (2) | UGAAGAACUAGACCUGCCCAAGUCCUUCAUCGUGCUUGCU | 40 | ND | 253 |
| 7.27 (2) | CGAUUAUACUAUCCCUGCCAGUAGUAAUCAGUGCUAUA | 38 | ND | 254 |
| 7.29 | CGGUGAAGACCUCUAUUAACAACAUGACCUGCCUGCGUUG | 40 | ND | 255 |
| 7.32 | CGCAAAUAUGUUCCUGCCAAAUACGGGCGUUGACGCUAGA | 40 | ND | 256 |
| 7.43 | GGACCCUGCCGAGCACAUUUAUUCUGGUAACUGAGCCCCC | 40 | ND | 257 |
| 7.51 | CGCUGAGAGAAAGCCCUGCCCUUUCAGCUCGAGAGUUAUA | 40 | ND | 258 |
| 7.58 | UGAGAUGCAGUUCCUGCCUGCUGCAUUUCUUAGAGUGUGU | 40 | ND | 259 |
| 7.83 | GAUUAACGGUUAUCCUGCCAACCGAUUAUAAGAGCAUGGA | 40 | ND | 260 |
| 7.89 | UGAGAGACUACAAUAGAACUUAUGUAACCUGCCACAUAGG | 40 | ND | 261 |
| 7.97 | UAGGAAGUGUAACCUGCCUCACGGUCCUAUCGAGUAGUUU | 40 | ND | 262 |
| 7.100 | UGAAAACGCAACCUGCCGGCGUCGUCCUUUGGGUAAUUUA | 40 | ND | 263 |
| 7.104 | AUAGGGGGUUACCUGCCGACCCCAGAAAUAAGCGUGAUU | 39 | ND | 264 |
| 7.105 | UCCUGCCAUAGCGUCUUCAUGUCUGACGUUUGAGUUUCCG | 40 | ND | 265 |
| 7.107 | UCCUAGGUUGGUCCUGCCACAGCUCAAAGGUUUAGCUUCA | 40 | ND | 266 |
| 7.109 | ACAUGCAGACAACCCUGCCUUCUGCGUGGUUUAGGAGUA | 39 | ND | 267 |
| 7.120 | AACCUCAGGCGACCUGCCGCUGUCUGAAGUUCGAGCAUAA | 40 | ND | 268 |
| 7.122 | ACUCAAGACCCUGCCACUAUGUGUUACUGAGUAGGAGCGU | 40 | ND | 269 |
| 7.125 | AUUCGAAAUACGGGUUAAAUCCCUGCCUUUAACACGACA | 39 | ND | 270 |
| 15.19 | UGUAGCCGCAUGUCGAGCAGCAAUUCCUGCCAUUGUAGG | 39 | 770 | 271 |
| 15.21 | CGGUGAAGACCUCUAUUAACAACAUGACCUGCCUGCGUUG | 40 | 200 | 272 |
| 15.34 | UCCCACCCUGCCUUGUCUGUUUGAUAGAGACACUGUCCUU | 40 | 190 | 273 |

**Table 13. αᵥβ₃ orphan aptamer sequences**

| Clone name (# of isolates) | Sequence of variable region 5'-gggagacaagaauaaacgcucaa [40N] uucgacaggaggcucacaacaggc-3' | Seq. length | K_{D} (nM) | SEQ ID NO: 310 |
|---|---|---|---|---|
| 7.1 | gguuugaaagauugccuguagcuccaaaucuuggugagcu | 40 | ND | 274 |
| 7.2 | ucccgccgauagcuuccacgaagaguuaucuguaaaacaa | 40 | ND | 275 |
| 7.11 | ugagcuccugauuccaaaccuauuccguuucugggu | 36 | ND | 276 |
| 7.30 | acuggacaagucaaucucuccggcuugagacuugguuuac | 40 | ND | 277 |
| 7.33 (2) | cgagcucuugcuuccaaaccuauuccagacguuu cuggg | 40 | ND | 278 |
| 7.41 (2) | gcgagccuauugucuaagaugcaccaggccuguuaagcau | 40 | >1000 | 279 |
| 7.42 | gccuguacggcgauuaugucuuuaccuuaacuguucc | 37 | ND | 280 |
| 7.46 | uaccaauggcacgaauaacugacuaccccccaaaauggaa | 40 | ND | 281 |
| 7.47 | gcggggcuuugcucaaguguuugcaaacgguaaauuccac | 40 | ND | 282 |
| 7.61 | ccuaccgacguccgccgcuggguuaaccuguaaagucacu | 40 | ND | 283 |
| 7.66 (2) | gugaaccgauaagcgaaaguaguaccccugcuugacuacu | 40 | >1000 | 284 |
| 7.67 | ggagcuccuaguuccaaaccuauuccagaaguuuucugggu | 41 | ND | 285 |
| 7.75 | uaguacgcagucauagcggggcagggacuuucuccgugca | 40 | ND | 286 |
| 7.76 | uuauacugguaugccgccgaccagaauuaauccaaugcgu | 40 | ND | 287 |
| 7.82 | ugagcuccugguuccaaaccuauuccagacguuucagggu | 40 | ND | 288 |
| 7.85 | ucuggccugugacuguagucguuucuucgaguugugacgc | 40 | ND | 289 |
| 7.92 | cucaacgauguccaagggcuuauaugagcgcguuacccc | 39 | ND | 290 |
| 7.93 | gcgagccuauugucuaagaugcgccaagccuguaaagcau | 40 | ND | 291 |
| 7.94 | gacuagccggccugagauccuuguucgccacacaugcugg | 40 | ND | 292 |
| 7.96 | cuucccccgcaaacacauguuuaguacugggagacuuggg | 40 | ND | 293 |
| 7.101 | ugagcuccugauuccgaaccuauuccagacguuucugggu | 40 | ND | 294 |
| 7.102 | cugauccucuugucauuguacaucucgcag | 30 | ND | 295 |
| 7.106 | uacuaagccuaacaaaagagcggauauuggcgcggcacg | 39 | ND | 296 |
| 7.108 | agucuuaguaguaccgccugcuucuaaccuugggcgcuuu | 40 | ND | 297 |
| 7.112 | ugauuucaugacuuaugccgccggcaugacuucaaugacg | 40 | ND | 298 |
| 7.114 | ucaaaggacggaagugccugugcccgacuaaagaguugag | 40 | ND | 299 |
| 7.118 | Cuaucgaucguuuuuucauuucccccugaccaucgccug | 39 | ND | 300 |
| 7.123 | Uugucccgcgcagaaacgugacaaauuuaacacgcaccgu | 40 | ND | 301 |
| 7.128 | Uucaacguuguucaagggcuuauaugagcgcguuauaccc | 40 | ND | 302 |
| 15.4(4) | Ugauuucaugacuuaugccgccggcaugacuucaaugacg | 40 | 2000 | 303 |
| 15.5 | Gcauucaaaauuugcgagaacgaauagaaguccgagagcc | 40 | 4000 | 304 |
| 15.13 | Gcgggauuuuccugaucaucccacugauucggggccuuac | 40 | 790 | 305 |
| (2) | | | | |
| 15.39 | Ucaaucucggacuagacuaacgaccuugguugacgcuca | 39 | 410 | 306 |
| 15.43 | Cgccguuaucacgacgugcguucugggcgguacucgcgca | 40 | 45 | 307 |

## Claims

1. A purified and isolated nucleic acid ligand which binds specifically and with high affinity to HGF and inhibits the interaction of HGF with c-met, wherein the nucleic acid ligand comprises the sequence CGUGAGCCUAUUUAUGUCAUCGU-C-UG or is chosen from one of SEQ ID No.s 12, 13, 18, 49, 57, 68, 74, 101.

2. A nucleic acid ligand according to claim 1 wherein the nucleic acid ligand is single stranded.

3. A nucleic acid ligand according to claim 1 or 2 wherein the nucleic acid ligand is a deoxyribonucleic acid ligand.

4. A nucleic acid ligand according to claim 1 or 2 wherein the nucleic acid ligand is a ribonucleic acid ligand.

5. A nucleic acid ligand according to claim 4 which comprises a modified ribonucleic acid and comprises the primary sequence CGUGAGCCUAUUUAUGUCAUCGU-C-UG or the primary sequence of one of SEQ ID No.s 12, 13, 18, 49, 57, 68, 74, 101.

6. A nucleic acid ligand according to claim 5 wherein said modified ribonucleic acid is chosen from the group consisting of a 2'-amino (2'NH₂) modified ribonucleic acid, a 2'-fluoro (2'-F) modified ribonucleic acid, and a 2'-O-methyl modified ribonucleic acid.

7. A nucleic acid ligand according to claims 1 or 2 wherein said nucleic acid ligand is RNA and is comprised of 2'-fluoro (2'-F) modified nucleotides.

8. A nucleic acid ligand according to claim 7 wherein said 2'-fluoro (2'-F) modified nucleotide is on the sugar moiety of pyrimidine residues.

9. A nucleic acid ligand according to any one of claims 1 to 4 wherein the nucleic acid ligand is modified, the modification chosen from:
- 2'-position sugar modifications;
- 5-position pyrimidine modifications;
- 8-position purine modifications;
- modifications at exocyclic amines;
- substitution of 4-thiouridine;
- substitution of 5-bromo-uracil;
- substitution of 5-iodo-uracil;
- backbone modifications;
- phosphorothioate modifications;
- alkyl phosphate modifications;
- methylations;
- isocytidine;
- isoguanidine;
- 5' and 3' capping.

10. A pharmaceutical composition comprising a nucleic acid ligand as defined in any one of claims 1 to 9 and a pharmaceutically acceptable excipient.

11. A nucleic acid ligand as defined in any one of claims 1 to 9 for use in treating or preventing disease.

12. Use of a nucleic acid ligand as defined in any one or claims 1 to 9 in the formulation of a pharmaceutical composition for treating or preventing a disease.

13. A nucleic acid ligand or use according to claim 11 or claim 12 wherein the disease is selected from: tumorigenic conditions, hypertension, arteriosclerosis, myocardial infarction and rheumatoid arthritis.

14. A nucleic acid ligand or use according to claim 11 or 12 wherein the disease is a tumorigenic condition.

15. Use of a nucleic acid ligand as defined in any one of claims 1 to 9 as an in vitro diagnostic reagent.

16. A nucleic acid ligand as defined in any one of claims 1 to 9 for use as an in vivo diagnostic reagent.

17. A nucleic acid ligand or use according to any one of the preceding claims wherein the HGF is human HGF.

## Patentansprüche

1. Gereinigter und isolierter Nucleinsäureligand, der spezifisch und mit hoher Affinität an HGF bindet und die Wechselwirkung von HGF mit c-met hemmt, worin der Nucleinsäureligand die Sequenz CGUGAGCCUAUUUAUGUCAUCGU-C-UG umfasst oder aus einer von Seq.-ID Nr. 12, 13, 18, 49, 57, 68, 74 und 101 ausgewählt ist.

2. Nucleinsäureligand nach Anspruch 1, worin der Nucleinsäureligand einzelsträngig ist.

3. Nucleinsäureligand nach Anspruch 1 oder 2, worin der Nucleinsäureligand ein Desoxyribonucleinsäureligand ist.

4. Nucleinsäureligand nach Anspruch 1 oder 2, worin der Nucleinsäureligand ein Ribonucleinsäureligand ist.

5. Nucleinsäureligand nach Anspruch 4, der eine modifizierte Ribonucleinsäure umfasst und die Primärsequenz CGUGAGCCUAUUUAUGUCAUCGU-C-UG oder die Primärsequenz einer von Seq.-ID Nr. 12, 13, 18, 49, 57, 68, 74 und 101 umfasst.

6. Nucleinsäureligand nach Anspruch 5, worin die modifizierte Ribonucleinsäure aus der aus einer 2'-Amino- (2'-NH₂-) modifizierten Ribonucleinsäure, einer 2'-Fluor-(2'-F-) modifizierten Ribonucleinsäure und einer 2'-O-Methyl-modifizierten Ribonucleinsäure bestehenden Gruppe ausgewählt ist.

7. Nucleinsäureligand nach Anspruch 1 oder 2, worin der Nucleinsäureligand RNA ist und aus 2'-Fluor- (2'-F-) modifizierten Nucleotiden besteht.

8. Nucleinsäureligand nach Anspruch 7, worin das 2'-Fluor- (2'-F-) modifizierte Nucleotid auf der Zuckergruppierung von Pyrimidinresten vorliegt.

9. Nucleinsäureligand nach einem der Ansprüche 1 bis 4, worin der Nucleinsäureligand modifiziert ist, wobei die Modifikation ausgewählt ist aus:
- Zucker-Modifikationen an Position 2';
- Pyrimidin-Modifikationen an Position 5;
- Purin-Modifikationen an Position 8;
- Modifikationen an exozyklischen Aminen;
- einer Substitution von 4-Thiouridin;
- einer Substitution von 5-Ioduracil ;
- Rückgratmodifikationen;
- Thiophosphat-Modifikationen;
- Methylierungen;
- Isocytidin;
- Isoguanidin;
- 5'- und 3'-Verkappung.

10. Pharmazeutische Zusammensetzung, umfassend einen Nucleinsäureliganden nach einem der Ansprüche 1 bis 9 sowie einen pharmazeutisch annehmbaren Arzneimittelträger.

11. Nucleinsäureligand nach einem der Ansprüche 1 bis 9 zur Verwendung bei der Behandlung oder Verhinderung einer Erkrankung.

12. Verwendung eines Nucleinsäureliganden nach einem der Ansprüche 1 bis 9 zur Formulierung einer pharmazeutischen Zusammensetzung zur Behandlung oder Verhinderung einer Erkrankung.

13. Nucleinsäureligand oder Verwendung nach Anspruch 11 oder 12, worin die Erkrankung aus folgenden ausgewählt ist: tumorigenen Leiden, Hypertonie, Arteriosklerose, Myokardinfarkt und chronischer Polyarthritis.

14. Nucleinsäureligand oder Verwendung nach Anspruch 11 oder 12, worin die Erkrankung ein tumorigenes Leiden ist.

15. Verwendung eines Nucleinsäureliganden nach einem der Ansprüche 1 bis 9 als In-vitro-Diagnosereagens.

16. Nucleinsäureligand nach einem der Ansprüche 1 bis 9 zur Verwendung als Invivo-Diagnosereagens.

17. Nucleinsäureligand oder Verwendung nach einem der vorangegangenen Ansprüche, worin der HGF menschlicher HGF ist.

## Revendications

1. Ligand d'acide nucléique purifié et isolé qui se lie spécifiquement et avec une affinité élevée au HGF et empêche l'interaction de HGF avec c-met, où le ligand d'acide nucléique comprend la séquence CGUGAGCCUAUUUAUGUCAUCGU-C-UG ou bien est sélectionné dans une des SEQ ID No.s 12, 13, 18, 49, 57, 68, 74, 101.

2. Ligand d'acide nucléique selon la revendication 1, où le ligand d'acide nucléique est à brin simple.

3. Ligand d'acide nucléique selon la revendication 1 ou 2, où le ligand d'acide nucléique est un ligand d'acide déoxyribonucléique.

4. Ligand d'acide nucléique selon la revendication 1 ou 2, où le ligand d'acide nucléique est un ligand d'acide ribonucléique.

5. Ligand d'acide nucléique selon la revendication 4, qui comprend un acide ribonucléique modifié et comprend la séquence primaire CGUGAGCCUAUUUAUGUCAUCGU-C-UG ou bien la séquence primaire d'une des SEQ ID No.s 12, 13, 18, 49, 57, 68, 74, 101.

6. Ligand d'acide nucléique selon la revendication 5, où ledit acide ribonucléique modifié est sélectionné dans le groupe consistant en acide 2'-amino(2'NH₂) modifié ribonucléique, un acide 2'-fluoro(2'-F) modifié ribonucléique et d'un acide 2'-O-méthyl modifié ribonucléique.

7. Ligand d'acide nucléique selon les revendications 1 ou 2, où ledit ligand d'acide nucléique est ARN et est constitué de nucléotides 2'-fluoro(2'-F) modifiés.

8. Ligand d'acide nucléique selon la revendication 7, où ledit nucléotide 2'-fluoro (2'-F) modifié est sur un fragment sucre de résidus de pyrimidine.

9. Ligand d'acide nucléique selon l'une quelconque des revendications 1 à 4, où le ligand d'acide nucléique est modifié, la modification est sélectionnée parmi:
- des modifications en position-2' du sucre;
- des modifications en position-5 de la pyrimidine;
- des modifications en position-8 de la purine;
- des modifications aux amines exocycliques;
- la substitution de 4-thiouridine;
- la substitution de 5-bromo-uracil;
- la substitution de 5-iodo-uracil;
- des modifications de squelette;
- des modifications de phosphorothioate;
- des modifications d'alkyl phosphate;
- des méthylations;
- la isocytidine;
- la isoguanidine;
- le coiffage de 5' et 3'.

10. Composition pharmaceutique comprenant un ligand d'acide nucléique tel que défini dans l'une quelconque des revendications 1 à 9 et un excipient pharmaceutiquement acceptable.

11. Ligand d'acide nucléique tel que défini dans l'une quelconque des revendications 1 à 9 destiné à être utilisé pour le traitement ou la prévention de maladie.

12. Utilisation d'un ligand d'acide nucléique tel que défini dans l'une quelconque des revendications 1 à 9 dans la formulation d'une composition pharmaceutique pour traiter ou prévenir une maladie.

13. Ligand d'acide nucléique ou utilisation selon la revendication 11 ou la revendication 12, où la maladie est sélectionnée parmi: pathologies oncogènes, hypertension, artériosclérose, infarctus du myocarde et arthrite rhumatoïde.

14. Ligand d'acide nucléique ou utilisation selon la revendication 11 ou 12, où la maladie est une pathologie oncogène.

15. Utilisation d'un ligand d'acide nucléique tel que défini dans l'une quelconque des revendications 1 à 9 comme un réactif de diagnostic in vitro.

16. Ligand d'acide nucléique tel que défini dans l'une quelconque des revendications 1 à 9 pour utilisation comme réactif de diagnostic in vivo.

17. Ligand d'acide nucléique ou utilisation selon l'une quelconque des revendications précédentes, où le HGF est un HGF humain.


### SEQUENCE LISTING

<110> NEXSTAR PHARMACEUTICALS, INC.
<120> Nucleic Acid Ligands Which Bind to Hepatocyte Growth Factor/Scatter Factor (HGF/SF) or its Receptor C-Met and to Integrins
<130> NEX 82/PCT
<140>
   <141>
<150> 09/364,543
   <151> 1999-07-29
<150> 09/364,539
   <151> 1999-07-29
<160> 310
<170> PatentIn Ver. 2.0
<210> 1
   <211> 78
   <212> DNA
   <213> Artificial Sequence
<220>
   <223> Description of Artificial Sequence: Synthetic Sequence
<220>
   <221> modified_base
   <222> (1)..(78)
   <223> N at positions 33-62 is any base.
<400> 1
   taatacgact cactataggg augacgaugc ggnnnnnnnn nnnnnnnnnn nnnnnnnnnn 60
   nncagacgac ucgcccga                                                78
<210> 2
   <211> 61
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<220>
   <221> modified_base
   <222> (1)..(61)
   <223> N at positions 16-45 is any base.
<400> 2
   gggaggacga ugcggnnnnn nnnnnnnnnn nnnnnnnnnn nnnnncagac gacucgcccg 60
   a 61
<210> 3
   <211> 32
   <212> DNA
   <213> Artificial Sequence
<220>
   <223> Description of Artificial Sequence: Synthetic Sequence
<400> 3
   taatacgact cactataggg augacgaugc gg 32
<210> 4
   <211> 16
   <212> DNA
   <213> Artificial Sequence
<220>
   <223> Description of Artificial Sequence: Synthetic Sequence
<400> 4
   tcgggcgagt cgtctg 16
<210> 5
   <211> 94
   <212> DNA
   <213> Artificial Sequence
<220>
   <223> Description of Artificial Sequence: Synthetic Sequence
<220>
   <221> modified_base
   <222> (1)..(99)
   <223> N at positions 41-70 is any base.
<400> 5
   taatacgact cactataggg agacaagaat aaacgctcaa nnnnnnnnnn nnnnnnnnnn 60
   nnnnnnnnnn ttcgacagga ggctcacaac aggc 94
<210> 6
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<220>
   <221> modified_base
   <222> (1)..(77)
   <223> N at positions 24-53 is any base.
<400> 6
   gggagauaag aauaaacgcu caannnnnnn nnnnnnnnnn nnnnnnnnnn nnnuucgaca 60
   ggaggcucac aacaggc 77
<210> 7
   <211> 26
   <212> DNA
   <213> Artificial Sequence
<220>
   <223> Description of Artificial Sequence: Synthetic Sequence
<400> 7
   taatacgact cactataggg agacaa 26
<210> 8
   <211> 24
   <212> DNA
   <213> Artificial Sequence
<220>
   <223> Description of Artificial Sequence: Synthetic Sequence
<400> 8
   gcctgttgtg agcctcctgt cgaa 24
<210> 9
   <211> 15
   <212> DNA
   <213> Artificial Sequence
<220>
   <223> Description of Artificial Sequence: Synthetic Sequence
<220>
   <221> modified_base
   <222> (1)..(15)
   <223> Purines and pyrimidines are 2'OMe; purines and pyrimidines at postions 1-11 are RNA; purines and pyrimidines at positions 12-15 are DNA.
<400> 9
   cccuccugcu acgcc 15
<210> 10
   <211> 16
   <212> DNA
   <213> Artificial Sequence
<220>
   <223> Description of Artificial Sequence: Synthetic Sequence
<220>
   <221> modified_base
   <222> (1)..(16)
   <223> Purines and pyrimidines are 2'OMe; purines and pyrimidines at positons 1-4 are DNA; purines and pyrimidines at positions 5-16 are RNA.
<400> 10
   gtctgcugag cgggcu 16
<210> 11
   <211> 34
   <212> RNA
   <213> Artificial Sequence
<220>
   <223> Description of Artificial Sequence: Synthetic Sequence
<220>
   <221> modified_base
   <222> (1)..(34)
   <223> All pyrimidines are 2'F.
<220>
   <221> modified_base
   <222> (1)..(34)
   <223> N at positions 3-32 is any base.
<400> 11
   ggnnnnnnnn nnnnnnnnnn nnnnnnnnnn nnca 34
<210> 12
   <211> 61
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<400> 12
   gggaggacga ugcggcgagu gccuguuuau gucaucgucc gucgucagac gacucgcccg 60
   a 61
<210> 13
   <211> 36
   <212> RNA
   <213> Artificial Sequence
<220>
   <223> Description of Artificial Sequence: Synthetic Sequence
<220>
   <221> modified_base
   <222> (1)..(36)
   <223> All pyrimidines are 2'F.
<400> 13
   ggacgaugcg gcgagugccu guuuauguca ucgucc 36
<210> 14
   <211> 28
   <212> RNA
   <213> Artificial Sequence
<220>
   <223> Description of Artificial Sequence: Synthetic Sequence
<220>
   <221> modified_base
   <222> (1)..(28)
   <223> All pyrimidines are 2'F.
<400> 14
   gacgaugcgg cgagugccug uuuauguc 28
<210> 15
   <211> 88
   <212> DNA
   <213> Artificial Sequence
<220>
   <223> Description of Artificial Sequence: Synthetic Sequence
<220>
   <221> modified_base
   <222> (1)..(88)
   <223> N at positions 33-72 is any base.
<400> 15
   taatacgact cactataggg augacgaugc ggnnnnnnnn nnnnnnnnnn nnnnnnnnnn 60
   nnnnnnnnnn nncagacgac ucgcccga 88
<210> 16
   <211> 28
   <212> RNA
   <213> Artificial Sequence
<220>
   <223> Description of Artificial Sequence: Synthetic Sequence
<220>
   <221> modified_base
   <222> (1)..(28)
   <223> All pyrimidines are 2'F.
<400> 16
   ggacgaugcg ggcggcucga ugucgucu 28
<210> 17
   <211> 38
   <212> RNA
   <213> Artificial Sequence
<220>
   <223> Description of Artificial Sequence: Synthetic Sequence
<220>
   <221> modified_base
   <222> (1)..(38)
   <223> All pyrimidines are 2'F.
<400> 17
   gggaggacga ugcgggcggc ucgaugucgu cuuauccc 38
<210> 18
   <211> 61
   <212> RNA
   <213> Artificial Sequence
<220>
   <223> Description of Artificial Sequence: Synthetic Sequence
<220>
   <221> modified_base
   <222> (1) .. (60)
   <223> All pyrimidines are 2'F.
<400> 18
   gggaggacga ugcggcggug ugaaccuguu uauguccgcg uaccccagac gacucgcccg 60
   a 61
<210> 19
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 19
   gggaggacga ugcggcggug uggaccuguu uauguccgcg uaccccagac gacucgcccg 60
   a                                                                 61
<210> 20
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 20
   gggaggacga ugcggaguga uccuauuuau gacaucgcgg gcugccagac gacucgcccg 60
   a                                                                 61
<210> 21
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 21
   gggaggacga ugcgguguga accuguuuau gucaucuuuu gucgucagac gacucgcccg 60
   a                                                                 61
<210> 22
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 22
   gggaggacga ugcgguguga accuauuuau gccaucucga gugcccagac gacucgcccg 60
   a 61
<210> 23
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 23
   gggaggacga ugcggcguga gccuauuuau gucaucaugu cuguccagac gacucgcccg 60
   a 61
<210> 24
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 24
   gggaggacga ugcggcgaga gccuauuuau gucaucaugc cugugcagac gacucgcccg 60
   a 61
<210> 25
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 25
   gggaggacga ugcggcggga gccuuuuuau gucaucaugu cugugcagac gacucgcccg 60
   a 61
<210> 26
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 26
   gggaggacga ugcggcguga gccuauuuau gucaucaugu cugugcagac gacucgcccg 60
   a 61
<210> 27
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 27
   gggaggacga ugcggcgcga gccuauuuau gucaucaugu cugugcagac gacucgcccg 60
   a 61
<210> 28
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 28
   gggaggacga ugcggcguga gccuauuuau gucaucaugu cuggucagac gacucgcccg 60
   a                                                                 61
<210> 29
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 29
   gggaggacga ugcggcguga gccuauuuac gucaucaugu cugugcagac gacucgcccg 60
   a                                                                 61
<210> 30
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 30
   gggaggacga ugcgguguga accuauuuau gccauuaugu cugugcagac gacucgcccg 60
   a                                                                 61
<210> 31
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 31
   gggaggacga ugcggcguga gccuauuuau gucaucaagu cugugcagac gacucgcccg 60
   a                                                                 61
<210> 32
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 32
   gggaggacga ugcggcguga gccuguuuau gaccucgucc auggccagac gacucgcccg 60
   a                                                                 61
<210> 33
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 33
   gggaggacga ugcggcguga gccuauuuau gacaugaccc ucgagcagac gacucgcccg 60
   a                                                                 61
<210> 34
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 34
   gggaggacga ugcggcguga gccuguauau gucauuguuc uccggcagac gacucgcccg 60
   a 61
<210> 35
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 35
   gggaggacga ugcggugagu accuguuuau gucaccacuu ucccccagac gacucgcccg 60
   a 61
<210> 36
   <211> 58
   <212> RNA
   <213> Artificial Sequence
<220>
   <223> Description of Artificial Sequence: Synthetic Sequence
<220>
   <221> modified_base
   <222> (1)..(57)
   <223> All pyrimidines are 2'F.
<400> 36
   gggaggacga ugcggugauu accuauuaug ucucgcccuc uccagacgac ucgcccga 58
<210> 37
   <211> 61
   <212> RNA
   <213> Artificial Sequence
<220>
   <223> Description of Artificial Sequence: Synthetic Sequence
<220>
   <221> modified_base
   <222> (1) .. (60)
   <223> All pyrimidines are 2'F.
<400> 37
   gggaggacga ugcggugauu accuauuuau gucaugcucc ucccccagac gacucgcccg 60
   a 61
<210> 38
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 38
   gggaggacga ugcggugaua accuguuuau gccaucgugc ugggccagac gacucgcccg 60
   a 61
<210> 39
   <211> 61
   <212> RNA
   <213> Artificial Sequence
<220>
   <223> Description of Artificial Sequence: Synthetic Sequence
<220>
   <221> modified base
   <222> (1)..(60)
   <223> All pyrimidines are 2'F.
<400> 39
   gggaggacga ugcggugaua accuguuuau gucaucgugc ugggccagac gacucgcccg 60
   a 61
<210> 40
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 40
   gggaggacga ugcggugaga accuauuuau gucaucgugu cuggccagac gacucgcccg 60
   a 61
<210> 41
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 41
   gggaggacga ugcggugaua accuauuuau gacgucgugg cuccccagac gacucgcccg 60
   a 61
<210> 42
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 42
   gggaggacga ugcgguggga accuauuuau gucaucuccg uccccagacg acucgcccga 60
<210> 43
   <211> 59
   <212> RNA
   <213> Artificial Sequence
<220>
   <223> Description of Artificial Sequence: Synthetic Sequence
<220>
   <221> modified_base
   <222> (1)..(58)
   <223> All pyrimidines are 2'F.
<400> 43
   gggaggacga ugcggcgaug augccuguuu augucgaugu cccccgggag acgaugcgg 59
<210> 44
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 44
   gggaggacga ugcggcgaua gccuauuuau gaccucgucc ccgugcagac gacucgcccg 60
   a 61
<210> 45
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 45
   gggaggacga ugcggcguga gccuauuuau gacaucguuc uuggccagac gacucgcccg 60
   a 61
<210> 46
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 46
   gggaggacga ugcggcguga gccuaucuau gucaucgugu gugcccagac gacucgcccg 60
   a 61
<210> 47
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 47
   gggaggacga ugcggugagu acuauuuaug ucgucguucg ugcccagacg acucgcccga 60
<210> 48
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 48
   gggaggacga ugcggcguga gccuuccaau gacgucgucc uuggccagac gacucgcccg 60
   a 61
<210> 49
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 49
   gggaggacga ugcgggcgac ucaaucugaa ucgucuuguc ccgugcagac gacucgcccg 60
   a 61
<210> 50
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 50
   gggaggacga ugcggucagc ggcgcgagcc uguuuauguc ugcugcagac gacucgcccg 60
   a 61
<210> 51
   <211> 57
   <212> RNA
   <213> Artificial Sequence
<220>
   <223> Description of Artificial Sequence: Synthetic Sequence
<220>
   <221> modified_base
   <222> (1)..(56)
   <223> All pyrimidines are 2'F.
<400> 51
   gggaggacga ugcggcguga gccuauuuau gucaucgucu gcagacgacu cgcccga 57
<210> 52
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 52
   gggaggacga ugcggucagu augacuuuua uagcacguuc gccccagacg acucgcccga 60
<210> 53
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.

<400> 53
   gggaggacga ugcggacagg uagucuucua uagcacuucc ucccccagac gacucgcccg 60
   a 61
<210> 54
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 54
   gggaggacga ugcggucaga augacuuuca uagcacgcuu uccccagacg acucgcccga 60
<210> 55
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 55
   gggaggacga ugcggacaua agucuucuau agcucguccu uugugcagac gacucgcccg 60
   a 61
<210> 56
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 56
   gggaggacga ugcggucagu auggcuucua uagcucguuc cucggcagac gacucgcccg 60
   a 61
<210> 57
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 57
   gggaggacga ugcgggugac ucaaaauggu gauccucguu uccgccagac gacucgcccg 60
   a 61
<210> 58
   <211> 62
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<400> 58
   gggaggacga ugcgggugac ucaaaauggu gauccucgau uuccgccaga cgacucgccc 60
   ga 62
<210> 59
   <211> 62
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<400> 59
   gggaggacga ugcgggugac ucaaaauggu gauccucgau ugccgccaga cgacucgccc 60
   ga 62
<210> 60
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 60
   gggaggacga ugcgggccga aaauucgucg acaucucccu gucugcagac gacucgcccg 60
   a 61
<210> 61
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 61
   gggaggacga ugcggggcga cuuuccucca auucucaccu cugcacagac gacucgcccg 60
   a 61
<210> 62
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 62
   gggaggacga ugcgggccau ucgaucgauu cuccgccgga ucgugcagac gacucgcccg 60
   a 61
<210> 63
   <211> 62
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<400> 63
   gggaggacga ugcggauccc gcgaccaggg cguuucuucc ucgucccaga cgacucgccc 60
   ga 62
<210> 64
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 64
   gggaggacga ugcggucccg aauuuaagug cguuuccucc gcguccagac gacucgcccg 60
   a 61
<210> 65
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 65
   gggaggacga ugcgguccca agauucaggg cguuucuucc ucguccagac gacucgcccg 60
   a 61
<210> 66
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 66
   gggaggacga ugcggucccg aguuugaggg cguuucuucu ucguccagac gacucgcccg 60
   a 61
<210> 67
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 67
   gggaggacga ugcgguccca guuucagggg cgauuccucu ucguccagac gacucgcccg 60
   a 61
<210> 68
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 68
   gggaggacga ugcgggcggc ucgaugucgu cuuaucccuu ugccccagac gacucgcccg 60
   a                                                                 61
<210> 69
   <211> 64
   <212> RNA
   <213> Artificial Sequence
<220>
   <223> Description of Artificial Sequence: Synthetic Sequence
<220>
   <221> modified_base
   <222> (1)..(63)
   <223> All pyrimindines are 2'F.
<400> 69
   gggaggacga ugcgggcggg cucgaugucg ucuuaucccc uuugccccca gacgacucgc 60
   ccga                                                              64
<210> 70
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 70
   gggaggacga ugcggccggc ucgaugucgu cuuaccccuu ugccccagac gacucgcccg 60
   a                                                                 61
<210> 71
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimindines are 2'F.
<400> 71
   gggaggacga ugcggguuug agugaugucg ucuugucccg ccugccagac gacucgcccg 60
   a 61
<210> 72
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 72
   gggaggacga ugcggguuag aguuuugucg ucuuguccca ugugcagacg acucgcccga 60
<210> 73
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 73
   gggaggacga ugcgggcuug agucuuugau cgucuuaucc cucgucagac gacucgcccg 60
   a 61
<210> 74
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 74
   gggaggacga ugcggguuug agugacgauc gucuuguccc augugcagac gacucgcccg 60
   a 61
<210> 75
   <211> 59
   <212> RNA
   <213> Artificial Sequence
<220>
   <223> Description of Artificial Sequence: Synthetic Sequence
<220>
   <221> modified_base
   <222> (1)..(58)
   <223> All pyrimidines are 2'F.
<400> 75
   gggaggacga ugcggguuug aguuaaacau cgguuuucuc cugcagacga cucgcccga 59
<210> 76
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 76
   gggaggacga ugcgggacgc guugauucau cgucuuaucc ugcugcagac gacucgcccg 60
   a 61
<210> 77
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 77
   gggaggacga ugcggguuug ggucuugauc ucgucuuguc ccgugcagac gacucgcccg 60
   a 61
<210> 78
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 78
   gggaggacga ugcggguuga uaggagucau caucgucuug uccgccagac gacucgcccg 60
   a                                                                 61
<210> 79
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 79
   gggaggacga ugcggguagu gaguuuucau ugucuugucc ccgugcagac gacucgcccg 60
   a                                                                 61
<210> 80
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 80
   gggaggacga ugcggugagu cauaguguug aucgucguau cccgucagac gacucgcccg 60
   a                                                                 61
<210> 81
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 81
   gggaggacga ugcgggugga gucaaaucgu cuugucccuu guccucagac gacucgcccg 60
   a                                                                 61
<210> 82
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 82
   gggaggacga ugcggguuug aguucugaca cgucuugucc caugccagac gacucgcccg 60
   a                                                                 61
<210> 83
   <211> 64
   <212> RNA
   <213> Artificial Sequence
<220>
   <223> Description of Artificial Sequence: Synthetic Sequence
<220>
   <221> modified_base
   <222> (1)..(63)
   <223> All pyrimidines are 2'F.
<400> 83
   gggaggacga ugcggguuag agcgugacag ucgucuuauc ccgggucaca gacgacucgc 60
   ccga                                                              64
<210> 84
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 84
   gggaggacga ugcggugaau uccucuggcu gaaaaugacu ugugccagac gacucgcccg 60
   a                                                                 61
<210> 85
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 85
   gggaggacga ugcggugaau uccuuuggcu gaaaaugacu ugugccagac gacucgcccg 60
   a                                                                 61
<210> 86
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 86
   gggaggacga ugcgggcaga gcgaaaaucg ucuugucccc gacgccagac gacucgcccg 60
   a                                                                  61
<210> 87
   <211> 60
   <212> RNA
   <213> Artificial Sequence
<220>
   <223> Description of Artificial Sequence: Synthetic Sequence
<220>
   <221> modified_base
   <222> (1)..(59)
   <223> All pyrimidines are 2'F.
<400> 87
   gggaggacga ugcgggugac ucaaaauggu gauccucguu ucgccagacg acucgcccga 60
<210> 88
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 88
   gggaggacga ugcggaggac uaaucccuaa ggaauagcuu gcccgcagac gacucgcccg 60
   a 61
<210> 89
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 89
   gggaggacga ugcggucgag cuucugaguu aaacuggggc cuccucagac gacucgcccg 60
   a 61
<210> 90
   <211> 63
   <212> RNA
   <213> Artificial Sequence
<220>
   <223> Description of Artificial Sequence: Synthetic Sequence
<220>
   <221> modified_base
   <222> (1)..(62)
   <223> All pyrimidines are 2'F.
<400> 90
   gggaggacga ugcggguccc cgaauuuaaa gugcguuuuc cuccgggcag acgacucgcc 60
   cga                                                               63
<210> 91
   <211> 62
   <212> RNA
   <213> Artificial Sequence
<220>
   <223> Description of Artificial Sequence: Synthetic Sequence
<220>
   <221> modified_base
   <222> (1)..(61)
   <223> All pyrimidines are 2'F.
<400> 91
   gggaggacga ugcgggguuu uucuuuucuu guucucuucu uucccccaga cgacucgccc 60
   ga                                                                62
<210> 92
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 92
   gggaggacga ugcggacagc ggcgacuagc cuguucaugc cugcccagac gacucgcccg 60
   a                                                                 61
<210> 93
   <211> 61
   <212> RNA
   <213> Artificial Sequence
<220>
   <223> Description of Artificial Sequence: Synthetic Sequence
<220>
   <221> modified_base
   <222> (1)..(60)
   <223> All pyrimidines are 2'F.
<400> 93
   gggaggacga ugcggguucu guguguccac guucuuaccc cugugcagac gacucgcccg 60
   a                                                                 61
<210> 94
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 94
   gggagauaag aauaaacgcu caaccuguuc ugaacgcaaa auggcguggu ggcuucgaca 60
   ggaggcucac aacaggc                                                77
<210> 95
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 95
   gggagauaag aauaaacgcu caaugucguu aguuuauuga caaggcccga aguucgacag 60
   gaggcucaca acaggc                                                 76
<210> 96
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 96
   gggagauaag aauaaacgcu caaucuuauu guguccagcu ucucccugca ggcuucgaca 60
   ggaggcucac aacaggc 77
<210> 97
   <211> 75
   <212> RNA
   <213> Artificial Sequence
<220>
   <223> Description of Artificial Sequence: Synthetic Sequence
<220>
   <221> modified_base
   <222> (1)..(75)
   <223> All pyrimidines are 2'F.
<400> 97
   gggagauaag aauaaacgcu caauguggca cuguugucca caagggccuc auucgacagg 60
   aggcucacaa caggc 75
<210> 98
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 98
   gggagauaag aauaaacgcu caauugacaa gguaccuguu gccuggcguu ucuuucgaca 60
   ggaggcucac aacaggc 77
<210> 99
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 99
   gggagauaag aauaaacgcu caaaguuagg cuuuaaagca cgauaaucag cauucgacag 60
   gaggcucaca acaggc                                                 76
<210> 100
   <211> 78
   <212> RNA
   <213> Artificial Sequence
<220>
   <223> Description of Artificial Sequence: Synthetic Sequence
<220>
   <221> modified_base
   <222> (1)..(78)
   <223> All pyrimidines are 2'F.
<400> 100
   gggagauaag aauaaacgcu caagucaaga ggaaaugaca cggcuccacu uuuauucgac 60
   aggaggcuca caacaggc                                               78
<210> 101
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 101
   gggagauaag aauaaacgcu caagccugag uuaaacauga cgguuuguga cccuucgaca 60
   ggaggcucac aacaggc                                                77
<210> 102
   <211> 81
   <212> RNA
   <213> Artificial Sequence
<220>
   <223> Description of Artificial Sequence: Synthetic Sequence
<220>
   <221> modified_base
   <222> (1)..(81)
   <223> All pyrimidines are 2'E.
<400> 102
   gggagauaag aauaaacgcu caagccugag uuaaacauga cggguuuugu gaccccuuuc 60
   gacaggaggc ucacaacagg c                                           81
<210> 103
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 103
   gggagauaag aauaaacgcu caagucugag uuggacacaa cgcauugaga cccuucgaca 60
   ggaggcucac aacaggc                                                77
<210> 104
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 104
   gggagauaag aauaaacgcu caagucugag uuggucacaa cgcauugaga cccuucgaca 60
   ggaggcucac aacaggc                                                77
<210> 105
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 105
   gggagauaag aauaaacgcu caagucugag uccguagggc gauuuguguc ccuucgacag 60
   gaggcucaca acaggc 76
<210> 106
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 106
   gggagauaag aauaaacgcu caaugccuua agagcggaac ucccugaccc accuucgaca 60
   ggaggcucac aacaggc 77
<210> 107
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.

<400> 107
   gggagauaag aauaaacgcu caagaucugu uggcgugucu acccgacccu ccuuucgaca 60
   ggaggcucac aacaggc 77
<210> 108
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 108
   gggagauaag aauaaacgcu caagaucugu uggcgugucu acccgacccu ccuuucgaca 60
   ggaggcucac aacaggc                                                77
<210> 109
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 109
   gggagauaag aauaaacgcu caacguuagc aucugaacga ugcccagccu caauucgaca 60
   ggaggcucac aacaggc                                                77
<210> 110
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 110
   gggagauaag aauaaacgcu caaguuagac ucaacaugag ucccagccuc aauucgacag 60
   gaggcucaca acaggc                                                 76
<210> 111
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 111
   gggagauaag aauaaacgcu caaucuguug gcgucguucu ccugacccuc cucuucgaca 60
   ggaggcucac aacaggc                                                77
<210> 112
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 112
   gggagauaag aauaaacgcu caagaguucc cuguugacuc gcucuccuga cccuucgaca 60
   ggaggcucac aacaggc                                                77
<210> 113
   <211> 78
   <212> RNA
   <213> Artificial Sequence
<220>
   <223> Description of Artificial Sequence: Synthetic Sequence
<220>
   <221> modified_base
   <222> (1)..(78)
   <223> All pyrimidines are 2'F.
<400> 113
   gggagauaag aauaaacgcu caauacagcg uguugguccc ggacggggac uuauuucgac 60
   aggaggcuca caacaggc                                               78
<210> 114
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 114
   gggagauaag aauaaacgcu caacgccugg accguuuguu uauccccgua gucuucgaca 60
   ggaggcucac aacaggc                                                77
<210> 115
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 115
   gggagauaag aauaaacgcu caacgugauu ccuaccauca gguaccuauc uuguucgaca 60
   ggaggcucac aacaggc                                                77
<210> 116
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 116
   gggagauaag aauaaacgcu caaagugaug ugagagcgug ccucuagucg guguucgaca 60
   ggaggcucac aacaggc                                                77
<210> 117
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 117
   gggagauaag aauaaacgcu caacgagccu ccuaccguuu agguaccauc uuguucgaca 60
   ggaggcucac aacaggc 77
<210> 118
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 118
   gggagauaag aauaaacgcu caauuagccu ccgaccguaa gguccuuuuc uuguucgaca 60
   ggaggcucac aacaggc 77
<210> 119
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 119
   gggagauaag aauaaacgcu caaggccucc aaccgcuaaa gguuccauuc uuguucgaca 60
   ggaggcucac aacaggc 77
<210> 120
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 120
   gggagauaag aauaaacgcu caacccgacc uccuguaacu gguugaggca cuauucgaca 60
   ggaggcucac aacaggc                                                 77
<210> 121
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 121
   gggagauaag aauaaacgcu caaggguucc ugauugaccc ugucucuaga cccuucgaca 60
   ggaggcucac aacaggc                                                 77
<210> 122
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 122
   gggagauaag aauaaacgcu caaggggagg cccuucagcc gucuccuuga cccuucgaca 60
   ggaggcucac aacaggc                                                 77
<210> 123
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 123
   gggagauaag aauaaacgcu caaugugaug ugagggcgug cuuccuaacg guguucgaca 60
   ggaggcucac aacaggc 77
<210> 124
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 124
   gggagauaag aauaaacgcu caauucauua ugcaucgaac aguauaccac agguguucau 60
   guguucgaca ggaggcucac aacaggc 87
<210> 125
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 125
   gggagauaag aauaaacgcu caaauccaaa uucuggucau gaggcgcugc agauacugcu 60
   gcguucgaca ggaggcucac aacaggc 87
<210> 126
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 126
   gggagauaag aauaaacgcu caaucugcgg acggugaggu uaaguugcaa cgacugcuug 60
   gcguucgaca ggaggcucac aacaggc 87
<210> 127
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 127
   gggagauaag aauaaacgcu caacagaccg ugcaaacccc ccuuagaggg uuuugucauu 60
   uacuucgaca ggaggcucac aacaggc 87
<210> 128
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 128
   gggagauaag aauaaacgcu caaccuuagg gcucccaaaa aucgggcccg ucgggccgau 60
   cacuucgaca ggaggcucac aacaggc 87
<210> 129
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 129
   gggagauaag aauaaacgcu caacgcggga uucucugagg acgaggcacg uguggguaau 60
   ucguucgaca ggaggcucac aacaggc 87
<210> 130
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2'F.
<400> 130
   gggagauaag aauaaacgcu caaucgggcu uggaugugga cguguauuuc uagcugugua 60
   cgcuucgaca ggaggcucac aacaggc 87
<210> 131
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 131
   gggagauaag aauaaacgcu caauuggguc gggacucgaa aggauuugau aggauacaug 60
   aauuucgaca ggaggcucac aacaggc 87
<210> 132
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 132
   cggugugaac cuguuuaugu ccgcguaccc 30
<210> 133
   <211> 29
   <212> RNA
   <213> Artificial Sequence
<220>
   <223> Description of Artificial Sequence: Synthetic Sequence
<220>
   <221> modified_base
   <222> (1) .. (29)
   <223> All pyrimidines are 2'F.
<400> 133
   ugggaaccua uuuaugucau cuccguccc 29
<210> 134
   <211> 30
   <212> RNA
   <213> Artificial Sequence
<220>
   <223> Description of Artificial Sequence: Synthetic
<220>
   <221> modified_base
   <222> (1)..(30)
   <223> All pyrimidines are 2'F.
<400> 134
   ugggaaccua uuuaugucau cgucugugcc 30
<210> 135
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 135
   cgugagccua uuuaugucau caugucugug 30
<210> 136
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 136
   ugugaaccug uuuaugccau cucgaguccc 30
<210> 137
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.

<400> 137
   ugugaaccua uuuaugccau cucgagugcc 30
<210> 138
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 138
   ugauaaccua uuuaugacgu cguggcuccc 30
<210> 139
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 139
   agugauccua uuuaugccgu cgcuucucgc 30
<210> 140
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 140
   agagnuccua uuuaugacau cccaugcccc 30
<210> 141
   <211> 30
   <212> RNA
   <213> Artificial Sequence
<220>
   <223> Description of Artificial Sequence: Synthetic Sequence
<220>
   <221> modified_base
   <222> (1) .. (30)
   <223> All pyrimidines are 2'F.
<400> 141
   ugaucaccug uuuaugccau cguucugggc 30
<210> 142
   <211> 30
   <212> RNA
   <213> Artificial Sequence
<220>
   <223> Description of Artificial Sequence: Synthetic Sequence
<220>
   <221> modified_base
   <222> (1) .. (30)
   <223> All pyrimidines are 2'F.
<400> 142
   ggugacccuu uuuaugacau cgcgucuggc 30
<210> 143
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 143
   aaucacagga aucaacuucu auucccgccc 30
<210> 144
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimindines are 2'F.
<400> 144
   aaucacagga aucgacuuuu auuccugccc 30
<210> 145
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimindines are 2'F.
<400> 145
   gcggcucgau gucgucuuau cccuuugccc 30
<210> 146
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimindines are 2'F.
<400> 146
   ucggcucguu gucgucuuau cccuuugccc 30
<210> 147
   <211> 31
   <212> RNA
   <213> Artificial Sequence
<220>
   <223> Description of Artificial Sequence: Synthetic Sequence
<220>
   <221> modified_base
   <222> (1)..(31)
   <223> All pyrimidines are 2'F.
<400> 147
   gcuggcucga ugucagguua ucccuuugcc c 31
<210> 148
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 148
   gugacucaaa auggugaucc ucguuuccgc 30
<210> 149
   <211> 30
   <212> RNA
   <213> Artificial Sequence
<220>
   <223> Description of Artificial Sequence: Synthetic Sequence
<220>
   <221> modified_base
   <222> (1) .. (30)
   <223> All pyrimidines are 2'F.
<400> 149
   ugaauuccuc uggcugaaaa ugacuugugc 30
<210> 150
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 150
   guuugaguga cgaucgucuu gucccaugug 30
<210> 151
   <211> 30
   <212> RNA
   <213> Artificial Sequence
<220>
   <223> Description of Artificial Sequence: Synthetic Sequence
<220>
   <221> modified_base
   <222> (1) .. (30)
   <223> All pyrimidines are 2'F.
<400> 151
   auugauucac ugcauccuug acucuucccc 30
<210> 152
   <211> 28
   <212> RNA
   <213> Artificial Sequence
<220>
   <223> Description of Artificial Sequence: Synthetic Sequence
<220>
   <221> modified_base
   <222> (1)..(28)
   <223> All pyrimidines are 2'F.
<400> 152
   cagacgacuc gcccgaagga cgaugcgg 28
<210> 153
   <211> 31
   <212> RNA
   <213> Artificial Sequence
<220>
   <223> Description of Artificial Sequence: Synthetic Sequence
<220>
   <221> modified_base
   <222> (1)..(31)
   <223> All pyrimidines are 2'F.
<400> 153
   gaguuauauu ucgucacccg uuccuuugcc c 31
<210> 154
   <211> 29
   <212> RNA
   <213> Artificial Sequence
<220>
   <223> Description of Artificial Sequence: Synthetic Sequence
<220>
   <221> modified_base
   <222> (1)..(29)
   <223> All pyrimidines are 2'F.
<400> 154
   acaguuuguc uucuauagcu cgucgcccc 29
<210> 155
   <211> 30
   <212> RNA
   <213> Artificial Sequence
<220>
   <223> Description of Artificial Sequence: Synthetic Sequence
<220>
   <221> modified_base
   <222> (1)..(30)
   <223> All pyrimidines are 2'F.
<400> 155
   ucagaaugac uuucauagcu cgcuuucccc 30
<210> 156
   <211> 37
   <212> RNA
   <213> Artificial Sequence
<220>
   <223> Description of Artificial Sequence: Synthetic Sequence
<220>
   <221> modified_base
   <222> (1)..(37)
   <223> All pyrimidines are 2'F. Bases at positions 1, 6, 13, 21, 33 are 2'OMe.
<400> 156
   ggacgaugcg gcgagugccu guuuauguca ucguccg 37
<210> 157
   <211> 37
   <212> RNA
   <213> Artificial Sequence
<220>
   <223> Description of Artificial Sequence: Synthetic Sequence
<220>
   <221> modified_base
   <222> (1)..(37)
   <223> All pyrimidines are 2'F. Bases at positions 2, 8, 14, 25 are 2'OMe.
<400> 157
   ggacgaugcg gcgagugccu guuuauguca ucguccg 37
<210> 158
   <211> 37
   <212> RNA
   <213> Artificial Sequence
<220>
   <223> Description of Artificial Sequence: Synthetic Sequence
<220>
   <221> modified_base
   <222> (1)..(37)
   <223> All pyrimidines are 2'F. Bases at positions 3, 10, 15 and 27 are 2'OMe.
<400> 158
   ggacgaugcg gcgagugccu guuuauguca ucguccg 37
<210> 159
   <211> 37
   <212> RNA
   <213> Artificial Sequence
<220>
   <223> Description of Artificial Sequence: Synthetic Sequence
<220>
   <221> modified_base
   <222> (1)..(37)
   <223> All pyrimidines are 2'F. Bases at positions 5, 11, 17, 30 are 2'OMe.
<400> 159
   ggacgaugcg gcgagugccu guuuauguca ucguccg 37
<210> 160
   <211> 70
   <212> RNA
   <213> Artificial Sequence
<220>
   <223> Description of Artificial Sequence: Synthetic Sequence
<220>
   <221> modified_base
   <222> (1)..(70)
   <223> All pyrimidines are 2'F.
<400> 160
   gggaggacga ugcgguuuga cuaugucuga cgggucugug gucaauuccg cccccagacg 60
   acucgcccga 70
<210> 161
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 161
   gggaggacga ugcggauccg uguugauguc cauauaaccu uaucccgucg cuccccagac 60
   gacucgcccg a 71
<210> 162
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 162
   gggaggacga ugcggguguu gacuucuagc cagaauaaca uuuuguaccc cuccccagac 60
   gacucgcccg a 71
<210> 163
   <211> 70
   <212> RNA
   <213> Artificial Sequence
<220>
   <223> Description of Artificial Sequence: Synthetic Sequence
<220>
   <221> modified_base
   <222> (1)..(70)
   <223> All pyrimidines are 2'F.
<400> 163
   gggaggacga ugcggucguu gagcuuuuga uagggcuugu ucuucgagcg uccccagacg 60
   acucgcccga 70
<210> 164
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 164
   gggaggacga ugcggugauc uuggguuuga ucguaauuac uucacccucc guccccagac 60
   gacucgcccg a 71
<210> 165
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 165
   gggaggacga ugcggcuccu uuuccgcuaa acaagaccac uuugagcccu gcccccagac 60
   gacucgcccg a 71
<210> 166
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 166
   gggaggacga ugcggccacc ucguuacgua cugauuuugg caucgcaguu ugccccagac 60
   gacucgcccg a 71
<210> 167
   <211> 73
   <212> RNA
   <213> Artificial Sequence
<220>
   <223> Description of Artificial Sequence: Synthetic Sequence
<220>
   <221> modified_base
   <222> (1)..(73)
   <223> All pyrimidines are 2'F.
<400> 167
   gggaggacga ugcgggggca ccucgauacg uacugauuuu gaauaucagu uagcccccag 60
   acgacucgcc cga 73
<210> 168
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 168
   gggaggacga ugcggcgauu cgucguauag aaaugauuug aaugcaccuc cuccccagac 60
   gacucgcccg a 71
<210> 169
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 169
   gggaggacga ugcggugugu uuguguguug uguuuguuau uccuguuugu guccucagac 60
   gacucgcccg a 71
<210> 170
   <211> 72
   <212> RNA
   <213> Artificial Sequence
<220>
   <223> Description of Artificial Sequence: Synthetic Sequence
<220>
   <221> modified_base
   <222> (1)..(72)
   <223> All pyrimidines are 2'F.
<400> 170
   gggaggacga ugcggucggu cguaaaaaau cguugguguc uaucuauugu ucuccccaga 60
   cgacucgccc ga 72
<210> 171
   <211> 70
   <212> RNA
   <213> Artificial Sequence
<220>
   <223> Description of Artificial Sequence: Synthetic Sequence
<220>
   <221> modified_base
   <222> (1)..(70)
   <223> All pyrimidines are 2'F.
<400> 171
   gggaggacga ugcggugcuc cagaggaacc aucguuuacu ucauuuauuc gccccagacg 60
   acucgcccga 70
<210> 172
   <211> 72
   <212> RNA
   <213> Artificial Sequence
<220>
   <223> Description of Artificial Sequence: Synthetic Sequence
<220>
   <221> modified_base
   <222> (1)..(72)
   <223> All pyrimidines are 2'F.
<400> 172
   gggaggacga ugcggugcuc cuuaggaacc aucgucuaua ucccauucug acugcccaga 60
   cgacucgccc ga 72
<210> 173
   <211> 71
   <212> RNA
   <213> Artificial Sequence
<220>
   <223> Description of Artificial Sequence: Synthetic Sequence
<220>
   <221> modified_base
   <222> (1) .. (71)
   <223> All pyrimidines are 2'F.
<400> 173
   gggaggacga ugcggugcuc cucaggaacc aucguuuuuc ccauguccuu cugcccagac 60
   gacucgcccg a 71
<210> 174
   <211> 71
   <212> RNA
   <213> Artificial Sequence
<220>
   <223> Description of Artificial Sequence: Synthetic Sequence
<220>
   <221> modified_base
   <222> (1)..(71)
   <223> All pyrimidines are 2'F.
<400> 174
   gggaggacga ugcggugcuc cuuggauuac caaggaacca uuuuccucua ccccccagac 60
   gacucgcccg a 71
<210> 175
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 175
   gggagauaag aauaaacgcu caagugcuca uuacgaacuu gaccgaugcc uauucgacag 60
   gaggcucaca acaggc 76
<210> 176
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 176
   gggagauaag aauaaacgcu caaggugcuc auuacgaacu ugaccgaagc cuauucgaca 60
   ggaggcucac aacaggc 77
<210> 177
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 177
   gggagauaag aauaaacgcu caaggugcuc auuacgaacu ugaccgaugc cuauucgaca 60
   ggaggcucac aacaggc 77
<210> 178
   <211> 74
   <212> RNA
   <213> Artificial Sequence
<220>
   <223> Description of Artificial Sequence: Synthetic Sequence
<220>
   <221> modified_base
   <222> (1)..(74)
   <223> All pyrimidines are 2'F.
<400> 178
   gggagauaag aauaaacgcu caaagugcuc caaugaacuu ugcucgcuga uucgacagga 60
   ggcucacaac aggc 74
<210> 179
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 179
   gggagauaag aauaaacgcu caaggugcuc cguuuggaac uugaucggua ggauucgaca 60
   ggaggcucac aacaggc 77
<210> 180
   <211> 76
   <212> RNA
   <213> Artificial Sequence
<220>
   <223> Description of Artificial Sequence: Synthetic Sequence
<220>
   <221> modified_base
   <222> (1)..(76)
   <223> All pyrimidines are 2'F.
<400> 180
   gggagauaag aauaaacgcu caagugcuca uucagaacuu gacguauaac cauucgacag 60
   gaggcucaca acaggc 76
<210> 181
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 181
   gggagauaag aauaaacgcu caaggugcuc cuuaggaacu ugaccguccg ccauucgaca 60
   ggaggcucac aacaggc 77
<210> 182
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 182
   gggagauaag aauaaacgcu caaguggugc uccacuaacc aaguggaacc uuguucgaca 60
   ggaggcucac aacaggc 77
<210> 183
   <211> 66
   <212> RNA
   <213> Artificial Sequence
<220>
   <223> Description of Artificial Sequence: Synthetic Sequence
<220>
   <221> modified_base
   <222> (1)..(66)
   <223> All pyrimidines are 2'F.
<400> 183
   gggagauaag aauaaacgcu caagugcucu ugaacuugac cguucgacag gaggcucaca 60
   acaggc 66
<210> 184
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2'F.
<400> 184
   gggagauaag aauaaacgcu caaacgauaa gugggaguga guaaguuuga guauucgaca 60
   ggaggcucac aacaggc 77
<210> 185
   <211> 77
   <212> RNA
   <213> Artificial Sequence
<220>
   <223> Description of Artificial Sequence: Synthetic Sequence
<220>
   <221> modified_base
   <222> (1) .. (77)
   <223> All pyrimidines are 2'F.
<400> 185
   gggagauaag aauaaacgcu caaccuagac ccccagguuc cuccccacua gucuucgaca 60
   ggaggcucac aacaggc                                                77
<210> 186
   <211> 36
   <212> RNA
   <213> Artificial Sequence
<220>
   <223> Description of Artificial Sequence: Synthetic Sequence
<220>
   <221> modified_base
   <222> (1)..(36)
   <223> All pyrimidines are 2'F. Purines at positions 1, 2, 6, 8, 11, 13-15, 17, 21, 27, 30, 33 are 2'OMe.
<400> 186
   ggacgaugcg gcgagugccu guuuauguca ucgucc 36
<210> 187
   <211> 36
   <212> RNA
   <213> Artificial Sequence
<220>
   <223> Description of Artificial Sequence: Synthetic Sequence
<220>
   <221> modified_base
   <222> (1)..(36)
   <223> All pyrimidines are 2'F. Purines at 1-3, 6, 8, 10-11, 13-15, 17, 21, 27, 30, 33 are 2'OMe.
<400> 187
   ggacgaugcg gcgagugccu guuuauguca ucgucc 36
<210> 188
   <211> 39
   <212> DNA
   <213> Artificial Sequence
<220>
   <223> Description of Artificial Sequence: Synthetic Sequence
<400> 188
   acgagtttat cgaaaaagaa cgatggttcc aatggagca 39
<210> 189
   <211> 27
   <212> DNA
   <213> Artificial Sequence
<220>
   <223> Description of Artificial Sequence: Synthetic Sequence
<400> 189
   cgcaggatcc taatacgact cactata 27
<210> 190
   <211> 104
   <212> DNA
   <213> Artificial Sequence
<220>
   <223> Description of Artificial Sequence: Synthetic Sequence
<220>
   <221> modified_base
   <222> (1) .. (104)
   <223> N at positions 41-80 is any base
<400> 190
   ttatacgact cactataggg agacaagaat aaacgctcaa nnnnnnnnnn nnnnnnnnnn 60
   nnnnnnnnnn nnnnnnnnnn ttcgacagga ggctcacaac aggc 104
<210> 191
   <211> 24
   <212> DNA
   <213> Artificial Sequence
<220>
   <223> Description of Artificial Sequence: Synthetic Sequence
<400> 191
   gcctgttgtg agcctcctgt cgaa 24
<210> 192
   <211> 40
   <212> DNA
   <213> Artificial Sequence
<220>
   <223> Description of Artificial Sequence: Synthetic Sequence
<400> 192
   taatacgact cactataggg agacaagaat aaacgctcaa 40
<210> 193
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 193
   gggagacaag aauaaucgcu caacguugaa ugcugcauua uggaguauug accgcuacau 60
   cccuucgaca ggaggcucac aacaggc                                     87
<210> 194
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 194
   gggagacaag aauaaacgcu caauucuacg uuguuuaagg gcuuauauga gcgcauuaua 60
   cccuucgaca ggaggcucaa caggc                                       85
<210> 195
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 195
   gggagacaag aauaaacgcu caauucaacg cuguuuaagg gcuuauauga gcgcguuaua 60
   cccuucgaca ggaggcucaa caggc                                       85
<210> 196
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 196
   gggagacaag aauaaacgcu caauucaacg cuguuuaagg gcuuauauga gcgcguuaca 60
   cccuucgaca ggaggcucac aacaggc 87
<210> 197
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 197
   gggagacaag aauaaacgcu caauucaacg cuguucaagg gcuuauauga gcgcguuaua 60
   cccuucgaca ggaggcucaa caggc 85
<210> 198
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 198
   gggagacaag aauaaacgcu caauucaacg auguuuaagg gcuuauauga gcgcguuaua 60
   cccuucgaca ggaggcucac aacaggc 87
<210> 199
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 199
   gggagacaag aauaaacgcu caauucauga aguccaaggg cuuauaugag cgcguuauac 60
   ccuugacagg aggcucacaa caggc 85
<210> 200
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 200
   gggagacaag aauaaacgcu caauucaacg cugucaaagg gcuuauauga gcgcguuaua 60
   cccuugacag gaggcucaca acaggc 86
<210> 201
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 201
   gggagacaag aauaaacgcu caauuaacgu uguucaaggg uuuauaugag ugcguuauac 60
   ccuucgacag gaggcucaca acaggc 86
<210> 202
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 202
   gggagacaag aauaaacgcu caauucaacg cuguccaagg gcuuauauga gcgcguuaua 60
   cccuucgaca ggaggcucac aacaggc 87
<210> 203
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 203
   gggagacaag aauaaacgcu caauucaacg gauguccaag ggcuuuauga gcgcguuaua 60
   cccuucgaca ggaggcucac aacaggc 87
<210> 204
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 204
   gggagacaag aauaaacgcu caauucgacg cuguucaagg gcuuauauga gcgcauuaua 60
   uccuucgaca ggaggcucac aacaggc 87
<210> 205
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 205
   gggagacaag aauaaacgcu caauucgacg cuguucaagg gcuuauauga gcgcguuaua 60
   cccuucgaca ggaggcucac aacaggc 87
<210> 206
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 206
   gggagacaag aauaaacgcu caauucgacg auguccaagg gcuuauauga gcgcauuaua 60
   cccuucgaca ggaggcucac aacaggc 87
<210> 207
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 207
   gggagacaag aauaaacgcu caauucaacg cuguucaagg gcuuauauga gcgcguuaca 60
   cccuucgaca ggaggcucac aacaggc 87
<210> 208
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 208
   gggagacaag aauaaacgcu caauucauga uguucaaggg cuuauaugag cgcauuauac 60
   ccuucgacag gaggcucaca acaggc 86
<210> 209
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 209
   gggagacaag aauaaacgcu caauucaacg auguugaggg gcuuauauga gcgcauuaua 60
   cccuucgaca ggaggcucac aacaggc 87
<210> 210
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 210
   gggagacaag aauaaacgcu caauucaacg auguccaagg gcuuauauga gcgcauuaua 60
   cccuucgaca ggaggcucac aacaggc 87
<210> 211
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 211
   gggagacaag aauaaacgcu caauucaacg cuguucaagg gcuuauguga gcgcguuaua 60
   cccuucgaca ggaggcucac aacaggc 87
<210> 212
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 212
   gggagacaag aauaaacgcu caauucaacg uuguccaagg gcuuauauga gcgcauuaua 60
   cccuucgaca ggaggcucac aacaggc 87
<210> 213
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 213
   gggagacaag aauaaacgcu caauucaacg cuguucaagg gcuuauauga gcgcauuaua 60
   cccuucgaca ggaggcucac aacaggc 87
<210> 214
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 214
   gggagacaag aauaaacgcu caauucaacg auguccaagg gcuuauauga gcggauuacc 60
   cuucgacagg aggcucacaa caggc 85
<210> 215
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 215
   gggagacaag aauaaacgcu caauucaaca cugugaaggg cuuauaugag cgcgucauac 60
   ccuucgacag gaggcucaca acaggc 86
<210> 216
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 216
   gggagacaag aauaaacgcu caauucaacg uuguucaagg gcuuauauga gcgcguuaua 60
   cccuucgaca ggaggcucac aacaggc 87
<210> 217
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 217
   gggagacaag aauaaacgcu caauucaacg uugucaaagg gcuuauauga gcggauuacc 60
   cuucgacagg aggcucacaa caggc 85
<210> 218
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 218
   gggagacaag aauaaacgcu caauucaacg uuguccaagg gcuuauauga gcggauuacc 60
   cuucgacagg aggcucacaa caggc 85
<210> 219
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.

<400> 219
   gggagacaag aauaaacgcu caauucuacg augucaaagg gcuuauauga gcggauuacc 60
   cuucgacagg aggcucacaa caggc 85
<210> 220
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 220
   gggagacaag aauaaacgcu caauucgacg cuguugaagg gcuuauacga gcggauuacc 60
   cuucgacagg aggcucacaa caggc                                       85
<210> 221
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 221
   gggagacaag aauaaacgcu caauucaacg cuguucaagg gcuuauauga gcggauuacc 60
   cuucgacagg aggcucacaa caggc                                       85
<210> 222
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 222
   gggagacaag aauaaacgcu caauucaaca uuguccaagg gcuuauauga gcggauuacc 60
   cuucgacagg aggcucacaa caggc                                       85
<210> 223
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 223
   gggagacaag aauaaacgcu caauucaacg uugucaaagg gcuuauacgg gcggauuacc 60
   cuucgacagg aggcucacaa caggc 85
<210> 224
   <211> 84
   <212> RNA
   <213> Artificial Sequence
<220>
   <223> Description of Artificial Sequence: Synthetic Sequence
<220>
   <221> modified_base
   <222> (1) .. (84)
   <223> All pyrimidines are 2' F.
<400> 224
   gggagacaag aauaaacgcu caauucaacg cugugaaggg cuuauaugag cggauuaccc 60
   uucgacagga ggcucacaac aggc 84
<210> 225
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 225
   gggagacaag aauaaacgcu caauucaacg cuguccaagg gcuuauauga gcgcauuaua 60
   cccuucgaca ggaggcucac aacaggc 87
<210> 226
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 226
   gggagacaag aauaaacgcu caauucgacu auguccaagg gcuuauauga gcggauuacc 60
   cuucgacagg aggcucacaa caggc 85
<210> 227
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 227
   gggagacaag aauaaacgcu caauucgacg augucuaagg gcuuauauga gcggauuacc 60
   cuucgacagg aggcucacaa caggc 85
<210> 228
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 228
   gggagacaag aauaaacgcu caauucaacg cuguugaagg gcuuauacga gcggauuacc 60
   cuucgacagg aggcucacaa caggc 85
<210> 229
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 229
   gggagacaag aauaaacgcu caauucaacg uuguccaagg gcuuauacga gcggauuacc 60
   cuucgacagg aggcucacaa caggc                                       85
<210> 230
   <211> 85
   <212> RNA
   <213> Artificial Sequence
<220>
   <223> Description of Artificial Sequence: Synthetic Sequence
<220>
   <221> modified_base
   <222> (1) .. (85)
   <223> All pyrimidines are 2' F.
<400> 230
   gggagacaag aauaaacgcu caauucaacg cuguccaagg gcuuauacga gcggauuacc 60
   cuucgacagg aggcucacaa caggc                                       85
<210> 231
   <211> 84
   <212> RNA
   <213> Artificial Sequence
<220>
   <223> Description of Artificial Sequence: Synthetic Sequence
<220>
   <221> modified_base
   <222> (1)..(84)
   <223> All pyrimidines are 2' F.
<400> 231
   gggagacaag aauaaacgcu caauucgacg cugugaaggg cuuauaugag cggauuaccc 60
   uucgacagga ggcucacaac aggc                                        84
<210> 232
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 232
   gggagacaag aauaaacgcu caauucaacg uugucaaagg gcuuauacga gcggauuacc 60
   cuucgacagg aggcucacaa caggc                                       85
<210> 233
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 233
   gggagacaag aauaaacgcu caauucaacg cuguugaagg gcuuauauga gcggauuacc 60
   cuucgacagg aggcucacaa caggc                                       85
<210> 234
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 234
   gggagacaag aauaaacgcu caauucuacg uugucuaagg gcuuauauga gcggauuacc 60
   cuucgacagg aggcucacaa caggc                                       85
<210> 235
   <211> 84
   <212> RNA
   <213> Artificial Sequence
<220>
   <223> Description of Artificial Sequence: Synthetic Sequence
<220>
   <221> modified_base
   <222> (1)..(84)
   <223> All pyrimidines are 2' F.
<400> 235
   gggagacaag aauaaacgcu caauucgacg cugugaaggg cuuauacgag cggauuaccc 60
   uucgacagga ggcucacaac aggc 84
<210> 236
   <211> 85
   <212> RNA
   <213> Artificial Sequence
<220>
   <223> Description of Artificial Sequence: Synthetic Sequence
<220>
   <221> modified_base
   <222> (1) .. (85)
   <223> All pyrimidines are 2' F.
<400> 236
   gggagacaag aauaaacgcu caauucaacg cuguucaagg gcuuauacga gcggauuacc 60
   cuucgacagg aggcucacaa caggc 85
<210> 237
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 237
   gggagacaag aauaaacgcu caauucaacg cuguccuagg gcuuauauga gcgcaggaua 60
   cccuucgaca ggaggcucac aacaggc 87
<210> 238
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 238
   gggagacaag aauaaacgcu caauucuacg cuguuuaagg gcuuauauga gcgaauuacc 60
   cuucgacagg aggcucacaa caggc 85
<210> 239
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 239
   gggagacaag aauaaacgcu caauucuacg uuguccaagg gcuuauauga gcggauuacc 60
   cuucgacagg aggcucacaa caggc 85
<210> 240
   <211> 85
   <212> RNA
   <213> Artificial Sequence
<220>
   <223> Description of Artificial Sequence: Synthetic Sequence
<220>
   <221> modified_base
   <222> (1) .. (85)
   <223> All pyrimidines are 2' F.
<400> 240
   gggagacaag aauaaacgcu caauucgacg uuguugaagg gcuuauauga gcggauuacc 60
   cuucgacagg aggcucacaa caggc 85
<210> 241
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 241
   gggagacaag aauaaacgcu caauucaacg cugucaaagg gcuuauauaa gcggauuacc 60
   cuucgacagg aggcucacaa caggc 85
<210> 242
   <211> 84
   <212> RNA
   <213> Artificial Sequence
<220>
   <223> Description of Artificial Sequence: Synthetic Sequence
<220>
   <221> modified_base
   <222> (1)..(84)
   <223> All pyrimidines are 2' F.
<400> 242
   gggagacaag aauaaacgcu caauucuacg cugugaaggg cuuauaugag cggauuaccc 60
   uucgacagga ggcucacaac aggc 84
<210> 243
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 243
   gggagacaag aauaaacgcu caauucgacg cugugaaggg cuuauaugag cggauacacc 60
   cuucgacagg aggcucacaa caggc 85
<210> 244
   <211> 84
   <212> RNA
   <213> Artificial Sequence
<220>
   <223> Description of Artificial Sequence: Synthetic Sequence
<220>
   <221> modified_base
   <222> (1)..(84)
   <223> All pyrimidines are 2' F.
<400> 244
   gggagacaag aauaaacgcu caauucuacg cugugaaggg cuuauacgag cggauuaccc 60
   uucgacagga ggcucacaac aggc 84
<210> 245
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 245
   gggagacaag aauaaacgcu caauucaacg uugucuaagg gcuuauauga gcggauuacc 60
   cuucgacagg aggcucacaa caggc 85
<210> 246
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 246
   gggagacaag aauaaacgcu caauucuacg uuguugaagg gcuuauauga gcggauuacc 60
   cuucgacagg aggcucacaa caggc 85
<210> 247
   <211> 84
   <212> RNA
   <213> Artificial Sequence
<220>
   <223> Description of Artificial Sequence: Synthetic Sequence
<220>
   <221> modified_base
   <222> (1) .. (84)
   <223> All pyrimidines are 2' F.
<400> 247
   gggagacaag aauaaacgcu caauucuacg cugugaaggg cuuauaugag cgaauuaccc 60
   uucgacagga ggcucacaac aggc                                        84
<210> 248
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 248
   gggagacaag aauaaacgcu caauucaacg cuguccaagg gcuuauaugg gcggauuacc 60
   cuucgacagg aggcucacaa caggc                                       85
<210> 249
   <211> 84
   <212> RNA
   <213> Artificial Sequence
<220>
   <223> Description of Artificial Sequence: Synthetic Sequence
<220>
   <221> modified_base
   <222> (1)..(84)
   <223> All pyrimidines are 2' F.
<400> 249
   gggagacaag aauaaacgcu caauucaacg cugugaaggg cuuauacgag cggauuaccc 60
   uucgacagga ggcucacaac aggc                                        84
<210> 250
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 250
   gggagacaag aauaaacgcu caaguaccgg aucgcccugc cacgguauuu gagacauuga 60
   aauucgacag gaggcucaca acaggc                                      86
<210> 251
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 251
   gggagacaag aauaaacgcu caagguagua aauggacucc ugccauccaa uacuaucucu 60
   gaguucgaca ggaggcucac aacaggc                                     87
<210> 252
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 252
   gggagacaag aauaaacgcu caauguaguc gcaugucgag cagcaauucc ugccauugua 60
   gguucgacag gaggcucaca acaggc                                      86
<210> 253
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 253
   gggagacaag aauaaacgcu caaugaagaa cuagaccugc ccaaguccuu caucgugcuu 60
   gcuuucgaca ggaggcucac aacaggc                                     87
<210> 254
   <211> 85
   <212> RNA
   <213> Artificial Sequence
<220>
   <223> Description of Artificial Sequence: Synthetic Sequence
<220>
   <221> modified_base
   <222> (1)..(85)
   <223> All pyrimidines are 2' F.
<400> 254
   gggagacaag aauaaacgcu caacgauuau acuaucccug ccaguaguaa ucagugcuau 60
   auucgacagg aggcucacaa caggc                                       85
<210> 255
   <211> 88
   <212> RNA
   <213> Artificial Sequence
<220>
   <223> Description of Artificial Sequence: Synthetic Sequence
<220>
   <221> modified_base
   <222> (1)..(88)
   <223> All pyrimidines are 2' F.
<400> 255
   gggagacaag aauaaacgcu caacggugaa gaccucuauu aacaacauga ccugccugcg 60
   uugauucgac aggaggcuca caacaggc                                    88
<210> 256
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 256
   gggagacaag aauaaacgcu caacgcaaau auguuccugc caaauacggg cguugacgcu 60
   agauucgaca ggaggcucac aacaggc                                     87
<210> 257
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 257
   gggagacaag aauaaacgcu caaggacccu gccgagcaca uuuauucugg uaacugagcc 60
   cccuucgaca ggaggcucac aacaggc                                     87
<210> 258
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 258
   gggagacaag aauaaacgcu caacgcugag agaaagcccu gcccuuucag cucgagaguu 60
   auauucgaca ggaggcucac aacaggc                                     87
<210> 259
   <211> 88
   <212> RNA
   <213> Artificial Sequence
<220>
   <223> Description of Artificial Sequence: Synthetic Sequence
<220>
   <221> modified_base
   <222> (1)..(88)
   <223> All pyrimidines are 2' F.
<900> 259
<210> 260
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 260
   gggagacaag aauaaacgcu caagauuaac gguuauccug ccaaccgauu auaagagcau 60
   ggauucgaca ggaggcucac aacaggc 87
<210> 261
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 261
   gggagacaag aauaaacgcu caaugagaga cuacaauaga acuuauguaa ccugccacau 60
   agguucgaca ggaggcucac aacaggc 87
<210> 262
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 262
   gggagacaag aauaaacgcu caauaggaag uguaaccugc cucacggucc uaucgaguag 60
   uuuuucgaca ggaggcucac aacaggc                                     87
<210> 263
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 263
   gggagacaag aauaaacgcu caaugaaaac gcaaccugcc ggcgucgucc uuuggguaau 60
   uuauucgaca ggaggcucac aacaggc                                     87
<210> 264
   <211> 86
   <212> RNA
   <213> Artificial Sequence
<220>
   <223> Description of Artificial Sequence: Synthetic Sequence
<220>
   <221> modified_base
   <222> (1) .. (86)
   <223> All pyrimidines are 2' F.
<400> 264
   gggagacaag aauaaacgcu caaauagggg guuaccugcc gaccccagaa auaagcguga 60
   uuuucgacag gaggcucaca acaggc                                      86
<210> 265
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 265
   gggagacaag aauaaacgcu caauccugcc auagcgucuu caugucugac guuugaguuu 60
   ccguucgaca ggaggcucac aacaggc                                     87
<210> 266
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 266
   gggagacaag aauaaacgcu caauccuagg uugguccugc cacagcucaa agguuuagcu 60
   ucauucgaca ggaggcucac aacaggc                                     87
<210> 267
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 267
   gggagacaag aauaaacgcu caaacaugca gacaacccug ccuucugcgu gguuuaggag 60
   uauucgacag gaggcucaca acaggc                                      86
<210> 268
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 268
   gggagacaag aauaaacgcu caaaaccuca ggcgaccugc cgcugucuga aguucgagca 60
   uaauucgaca ggaggcucac aacaggc                                     87
<210> 269
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 269
   gggagacaag aauaaacgcu caaacucaag acccugccac uauguguuac ugaguaggag 60
   cguuucgaca ggaggcucac aacaggc                                     87
<210> 270
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 270
   gggagacaag aauaaacgcu caaauucgaa auacggguua aaucccugcc uuuaacacga 60
   cauucgacag gaggcucaca acaggc                                      86
<210> 271
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 271
   gggagacaag aauaaacgcu caauguagcc gcaugucgag cagcaauucc ugccauugua 60
   gguucgacag gaggcucaca acaggc                                      86
<210> 272
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.

<400> 272
   gggagacaag aauaaacgcu caacggugaa gaccucuauu aacaacauga ccugccugcg 60
   uuguucgaca ggaggcucac aacaggc                                     87
<210> 273
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 273
   gggagacaag aauaaacgcu caaucccacc cugccuuguc uguuugauag agacacuguc 60
   cuuuucgaca ggaggcucac aacaggc                                     87
<210> 274
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 274
   gggagacaag aauaaacgcu caagguuuga aagauugccu guagcuccaa aucuuggugu 60
   gcuuucgaca ggaggcucac aacaggc 87
<210> 275
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 275
   gggagacaag aauaaacgcu caaucccgcc gauagcuucc acgaagaguu aucuguaaaa 60
   caauucgaca ggaggcucac aacaggc 87
<210> 276
   <211> 83
   <212> RNA
   <213> Artificial Sequence
<220>
   <223> Description of Artificial Sequence: Synthetic Sequence
<220>
   <221> modified_base
   <222> (1)..(83)
   <223> All pyrimidines are 2' F.
<400> 276
   gggagacaag aauaaacgcu caaugagcuc cugauuccaa accuauuccg uuucuggguu 60
   ucgacaggag gcucacaaca ggc 83
<210> 277
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 277
   gggagacaag aauaaacgcu caaacuggac aagucaaucu cuccggcuug agacuugguu 60
   uacuucgaca ggaggcucac aacaggc 87
<210> 278
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 278
   gggagacaag aauaaacgcu caacgagcuc uugcuuccaa accuauucca gacguuucug 60
   gguucgacag gaggcucaca acaggc 86
<210> 279
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 279
   gggagacaag aauaaacgcu caagcgagcc uauugucuaa gaugcaccag gccuguuaag 60
   cauuucgaca ggaggcucac aacaggc 87
<210> 280
   <211> 84
   <212> RNA
   <213> Artificial Sequence
<220>
   <223> Description of Artificial Sequence: Synthetic Sequence
<220>
   <221> modified_base
   <222> (1)..(89)
   <223> All pyrimidines are 2' F.
<400> 280
   gggagacaag aauaaacgcu caagccugua cggcgauuau gucuuuaccu uaacuguucc 60
   uucgacagga ggcucacaac aggc 84
<210> 281
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 281
   gggagacaag aauaaacgcu caauaccaau ggcacgaaua acugacuacc ccccaaaaug 60
   gaauucgaca ggaggcucac aacaggc 87
<210> 282
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 282
   gggagacaag aauaaacgcu caagcggggc uuugcucaag uguuugcaaa cgguaaauuc 60
   cacuucgaca ggaggcucac aacaggc 87
<210> 283
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 283
   gggagacaag aauaaacgcu caaccuaccg acguccgccg cuggguuaac cuguaaaguc 60
   acuuucgaca ggaggcucac aacaggc 87
<210> 284
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 284
   gggagacaag aauaaacgcu caagugaacc gauaagcgaa aguaguaccc cugcuugacu 60
   acuuucgaca ggaggcucac aacaggc 87
<210> 285
   <211> 88
   <212> RNA
   <213> Artificial Sequence
<220>
   <223> Description of Artificial Sequence: Synthetic Sequence
<220>
   <221> modified_base
   <222> (1)..(88)
   <223> All pyrimidines are 2' F.
<400> 285
   gggagacaag aauaaacgcu caaggagcuc cuaguuccaa accuauucca gaaguuuucu 60
   ggguuucgac aggaggcuca caacaggc 88
<210> 286
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 286
   gggagacaag aauaaacgcu caauaguacg cagucauagc ggggcaggga cuuucuccgu 60
   gcauucgaca ggaggcucac aacaggc 87
<210> 287
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 287
   gggagacaag aauaaacgcu caauuauacu gguaugccgc cgaccagaau uaauccaaug 60
   cguuucgaca ggaggcucac aacaggc 87
<210> 288
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 288
   gggagacaag aauaaacgcu caaugagcuc cugguuccaa accuauucca gacguuucag 60
   gguuucgaca ggaggcucac aacaggc 87
<210> 289
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 289
   gggagacaag aauaaacgcu caaucuggcc ugugacugua gucguuucuu cgaguuguga 60
   cgcuucgaca ggaggcucac aacaggc 87
<210> 290
   <211> 86
   <212> RNA
   <213> Artificial Sequence
<220>
   <223> Description of Artificial Sequence: Synthetic Sequence
<220>
   <221> modified_base
   <222> (1) .. (86)
   <223> All pyrimidines are 2' F.
<400> 290
   gggagacaag aauaaacgcu caacucaacg auguccaagg gcuuauauga gcgcguuacc 60
   ccuucgacag gaggcucaca acaggc 86
<210> 291
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 291
   gggagacaag aauaaacgcu caagcgagcc uauugucuaa gaugcgccaa gccuguaaag 60
   cauuucgaca ggaggcucac aacaggc 87
<210> 292
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 292
   gggagacaag aauaaacgcu caagacuagc cggccugaga uccuuguucg ccacacaugc 60
   ugguucgaca ggaggcucac aacaggc 87
<210> 293
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 293
   gggagacaag aauaaacgcu caacuucccc cgcaaacaca uguuuaguac ugggagacuu 60
   ggguucgaca ggaggcucac aacaggc 87
<210> 294
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 294
   gggagacaag aauaaacgcu caaugagcuc cugauuccga accuauucca gacguuucug 60
   gguuucgaca ggaggcucac aacaggc 87
<210> 295
   <211> 77
   <212> RNA
   <213> Artificial Sequence
<220>
   <223> Description of Artificial Sequence: Synthetic Sequence
<220>
   <221> modified_base
   <222> (1)..(77)
   <223> All pyrimidines are 2' F.
<400> 295
   gggagacaag aauaaacgcu caacugaucc ucuugucauu guacaucucg caguucgaca 60
   ggaggcucac aacaggc 77
<210> 296
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 296
   gggagacaag aauaaacgcu caauacuaag ccuaacaaaa gagcggauau uggcgcggca 60
   cguucgacag gaggcucaca acaggc 86
<210> 297
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 297
   gggagacaag aauaaacgcu caaagucuua guaguaccgc cugcuucuaa ccuugggcgc 60
   uuuuucgaca ggaggcucac aacaggc 87
<210> 298
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 298
   gggagacaag aauaaacgcu caaugauuuc augacuuaug ccgccggcau gacuucaaug 60
   acguucgaca ggaggcucac aacaggc 87
<210> 299
   <211> 87
   <212> RNA
   <213> Artificial Sequence
<220>
   <223> Description of Artificial Sequence: Synthetic Sequence
<220>
   <221> modified_base
   <222> (1) .. (87)
   <223> All pyrimidines are 2' F.
<400> 299
   gggagacaag aauaaacgcu caaucaaagg acggaagugc cugugcccga cuaaagaguu 60
   gaguucgaca ggaggcucac aacaggc 87
<210> 300
   <211> 86
   <212> RNA
   <213> Artificial Sequence
<220>
   <223> Description of Artificial Sequence: Synthetic Sequence
<220>
   <221> modified_base
   <222> (1) .. (86)
   <223> All pyrimidines are 2' F.
<400> 300
   gggagacaag aauaaacgcu caacuaucga ucguuuuuuc auuucccccu gaccaucgcc 60
   uguucgacag gaggcucaca acaggc 86
<210> 301
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 301
   gggagacaag aauaaacgcu caauuguccc gcgcagaaac gugacaaauu uaacacgcac 60
   cguuucgaca ggaggcucac aacaggc 87
<210> 302
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 302
   gggagacaag aauaaacgcu caauucaacg uuguucaagg gcuuauauga gcgcguuaua 60
   cccuucgaca ggaggcucac aacaggc 87
<210> 303
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 303
   gggagacaag aauaaacgcu caaugauuuc augacuuaug ccgccggcau gacuucaaug 60
   acguucgaca ggaggcucac aacaggc 87
<210> 304
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 304
   gggagacaag aauaaacgcu caagcauuca aaauuugcga gaacgaauag aaguccgaga 60
   gccuucgaca ggaggcucac aacaggc 87
<210> 305
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 305
   gggagacaag aauaaacgcu caagcgggau uuuccugauc aucccacuga uucggggccu 60
   uacuucgaca ggaggcucac aacaggc 87
<210> 306
   <211> 86
   <212> RNA
   <213> Artificial Sequence
<220>
   <223> Description of Artificial Sequence: Synthetic Sequence
<220>
   <221> modified_base
   <222> (1)..(86)
   <223> All pyrimidines are 2' F.
<400> 306
   gggagacaag aauaaacgcu caaucaaucu cggacuagac uaacgaccuu gguugacgcu 60
   cauucgacag gaggcucaca acaggc 86
<210> 307
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2' F.
<400> 307
   gggagacaag aauaaacgcu caacgccguu aucacgacgu gcguucuggg cgguacucgc 60
   gcauucgaca ggaggcucac aacaggc 87
<210> 308
   <211> 71
   <212> RNA
   <213> Artificial Sequence
<220>
   <223> Description of Artificial Sequence: Synthetic Sequence
<220>
   <221> modified_base
   <222> (1) .. (71)
   <223> All pyrimidines are 2'F;
<400> 308
   gggaggacga ugcggnnnnn nnnnnnnnnn nnnnnnnnnn nnnnnnnnnn nnnnncagac 60
   gacucgcccg a 71
<210> 309
   <211> 87
   <212> RNA
   <213> Artificial Sequence
<220>
   <223> Description of Artificial Sequence: Synthetic Sequence
<220>
   <221> modified_base
   <222> (1)..(87)
   <223> All pyrimidines are 2'F.
<400> 309
   gggagauaag aauaaacgcu caannnnnnn nnnnnnnnnn nnnnnnnnnn nnnnnnnnnn 60
   nnnuucgaca ggaggcucac aacaggc 87
<210> 310
   <211> 87
   <212> RNA
   <213> Artificial Sequence
<220>
   <223> Description of Artificial Sequence: Synthetic Sequence
<400> 310
   gggagacaag aauaaacgcu caannnnnnn nnnnnnnnnn nnnnnnnnnn nnnnnnnnnn 60
   nnnuucgaca ggaggcucac aacaggc                                      87